# EUROPEAN PATENT APPLICATION

(11) **EP 3 904 368 A2**
(43) Date of publication of application: **03.11.2021**
(21) Application number: 19906010.4
(22) Date of filing: 26.12.2019
(51) Int. Cl.: C07K 7/06, A23L 33/18, A61K 8/64, A61P 25/14, A61Q 19/00, A61K 38/00

(54) **ACETYLCHOLINE RECEPTOR INHIBITORY PEPTIDES AND USES THEREOF**

(30) Priority: 26.12.2018 KR 20180169425; 24.12.2019 KR 20190173644
(71) Applicant: Skinmed Co., Ltd., Daejeon 34050 (KR)
(72) Inventor: KIM, Sung Hyun, Sejong-si 30098 (KR); CHOI, Won Il, Seoul 04500 (KR); SHIN, Yong Chul, Jinju-si, Gyeongsangnam-do 52817 (KR); YUN, Young Sung, Jinju-si, Gyeongsangnam-do 52731 (KR); KIM, Jin Hwa, Daejeon 34049 (KR); LEE, Jeung Hoon, Daejeon 34124 (KR)
(74) Representative: Ladendorf, Oliver
(86) International application number: PCT/KR2019/018460
(87) International publication number: WO 2020/138948

(57) **Abstract**

The present invention relates to acetylcholine receptor inhibitory peptides and uses thereof, wherein phages with high binding affinity for acetylcholine receptors were screened using a random peptide recombinant phage, and acetylcholine receptor-binding peptides were selected through DNA of the phages. By having confirmed, using the selected peptides, the binding affinity for acetylcholine receptors and the effect of inhibiting the action of acetylcholine receptors, and having confirmed, via the modifications of the peptides, peptide sites and sequences that are vital in binding to acetylcholine receptors, the acetylcholine receptor inhibitory peptides of the present invention are expected to be used in the development of a cosmetic composition for alleviating wrinkles, a medicine for the prevention or treatment of acetylcholine receptor-associated diseases, and a health functional food for the amelioration thereof.

## Description

### Technical Field

The present invention relates to acetylene receptor inhibitory peptides and uses thereof.

### Background Art

Acetylcholine, which is a chemical substance that is present in animal nervous tissues, is secreted at the nerve ending and serves to transmit the nerve stimulation to muscles. Transmitters secreted from nerve endings are known to be acetylcholine at the motor nerves and parasympathetic nerves and epinephrine (adrenaline) at the sympathetic nerves. The secretion of acetylcholine results in physiological actions, such as blood pressure lowering, heart rate suppression, intestinal contraction, and skeletal muscle contraction. For muscle contraction, the nerve sends a command to the muscle to contract, and thus the muscle contracts. In response to the command, the nerve secrets acetylcholine at the site where the nerve and the muscle meet each other (nerve-muscle junction), and this substance binds to the muscle's acetylcholine receptor, allowing the muscle to contract (Vincent, A., 1985; and Lindstrom, J.M., et al., 1976). The blockage of acetylcholine receptors at the peripheral site controlling femoral skeletal muscles causes muscular paralysis, and the blockage of acetylcholine receptors in smooth and cardiac muscles responsible for respiration or heat motion causes respiratory and cardiac paralysis.

Acetylcholine receptors are classified into muscarinic acetylcholine receptors (mAchR) and nicotinic acetylcholine receptors (nAchR). Muscarinic acetylcholine receptors are G protein-coupled receptors that can be activated by muscarine, and activate different signaling mechanisms depending on the subtype. Muscarinic acetylcholine receptors are distributed throughout the body, including the central nervous system and peripheral organs, and mainly serve to mediate physiological actions of acetylcholine secreted from postganglionic fibers of the parasympathetic nervous system.

Nicotinic acetylcholine receptors are receptors that mimic the pharmacological actions of nicotine and are ion channels operated by neurotransmitters. Nicotinic acetylcholine receptors are non-selective cation channels through which sodium, potassium, calcium ions, and the like pass non-selectively by opening and closing of ion channels, and regulate electronic signaling between nerve cells and muscle cells. Nicotinic acetylcholine receptors may be divided into a muscle type and a neuronal type according to the expression site. The muscle-type nicotinic acetylcholine receptors are expressed in the neuromuscular junction where motor neurons and skeletal muscles meet each other, and acetylcholine secreted from motor neurons contributes to induce an end plate potential (EPP) of Skeletal muscle cell membranes. Meanwhile, the neuronal nicotinic acetylcholine receptors are expressed in peripheral ganglia of the autonomic nervous system (ANS), and thus acetylcholine secreted from preganglionic fibers contributes to excite postganglionic fibers.

Drugs that hinder or inhibit the activity of acetylcholine or mimic the behavior of acetylcholine are very advantageously used. Acetylcholine receptor agonists are used to treat myasthenia gravis and Alzheimer's disease. Myasthenia gravis is an autoimmune disease in which antibodies to nicotinic acetylcholine receptors are produced in the body to inhibit normal acetylcholine signaling, and myasthenia gravis can be treated by increasing the time while acetylcholine can interact with each receptor, before inactivation, in the synaptic cleft between a nerve and a muscle, through acetylcholine esterase (AChE).

In addition, the obstruction of acetylcholine secretion suppresses muscle contraction to cause muscular paralysis and straighten wrinkles, for which Botox is used. Botox blocks the secretion of acetylcholine, which is a substance essential for muscle contraction at the motor nerve endings. As a result, the muscles are immobile, and the wrinkles induced by the muscles disappear. The muscle-relaxation effect by Botox gradually disappears after 3 to 6 weeks, and thus repeated administration is required.

Accordingly, the present inventors, while studying acetylcholine receptor-binding peptides, screened and secured peptides with high binding affinity to acetylcholine receptors, and verified that these peptides bind to acetylcholine reactors to prevent acetylcholine binding, thereby inhibiting the action of acetylcholine receptors, and thus, the present inventors could complete the present invention.

Furthermore, the present inventors identified predetermined arrangement orders of specific amino acids, which are important for inhibiting actions of acetylcholine receptors through the binding of the screened peptides and the acetylcholine receptors, and verified that libraries of the peptides set forth in predetermined formulas bind to acetylcholine receptors to inhibit the actions of the acetylcholine receptors, and thus the present inventors completed the present invention.

Korea Patent No. 1216008, which corresponds to a prior art, discloses a peptide that binds to an acetylcholine receptor and is selected using biopanning, but fails to disclose peptides and libraries containing the amino acid sequences of the present invention. In addition, Korean Patent Publication No. 2018-0028748 discloses a neurotransmitter release-controlling peptide containing acetylcholine and a wrinkle relief effect thereof, but fails to disclose the acetylcholine receptor binding affinity of the peptides containing amino acid sequences of the present invention and the resultant acetylcholine receptor inhibitory effect. Korean Patent Publication No. 2014-0139010 discloses a peptide for promoting percutaneous penetration, but has a different constitution from the inhibitory peptides through acetylcholine receptor binding of the present invention.

### Detailed Description of the Invention

### Technical Problem

An aspect of the present invention is to provide acetylcholine receptor inhibitory peptides and compositions containing the same.

### Technical Solution

The present invention is directed to an acetylcholine receptor-binding peptide containing an amino acid sequence set forth in Formula 1 below:

[Formula 1] X_{L}-(K or R-X-(K or R)-X_{M}-(K or R)-X_{N}

wherein X_{L}, X_{M}, and X_{N} each may independently represent a sequence composed of one to eight arbitrary amino acids and X may represent a sequence composed of one arbitrary amino acid and, preferably, X_{L}, X_{M}, and X_{N} each may independently represent one to four arbitrary amino acids and X may represent one arbitrary amino acid.

The present invention is directed to an acetylcholine receptor-binding peptide containing an amino acid sequence set forth in Formula 2 below:

[Formula 2] (K or R)-X-(K or R)-(K or R)-X_{M-1-}(K or R)

wherein X_{M―1} may represent a sequence composed of one to three arbitrary amino acids and X may represent a sequence composed of one arbitrary amino acid and, preferably, X_{M-1} may represent three arbitrary amino acids and X may represent one arbitrary amino acid.

The peptide may be a peptide of any one sequence selected from the group consisting of SEQ ID NOs: 1 to 200 (see Table 10).

The present invention is directed to an acetylcholine receptor-binding peptide containing an amino acid sequence set forth in Formula 3 or 3-1 below:

[Formula 3] X_{L}-(K or R)-X-(K or R)-(K or R)-X_{M-1}-(K or R)

wherein X_{L} and X_{M-1} each may independently represent a sequence composed of one to three arbitrary amino acids and X may represent a sequence composed of one arbitrary amino acid and, preferably, X_{L} and X_{M-1} each may independently represent three arbitrary amino acids and X may represent one arbitrary amino acid; and

[Formula 3-1] (K or R)-X-(K or R)-(K or R)-X_{M-1}-(K or R)-X_{N}

wherein X_{M-1} and X_{N} each may independently represent a sequence composed of one to three arbitrary amino acids and X may represent a sequence composed of one arbitrary amino acid and, preferably, X_{M-1} and X_{N} each may independently represent three amino acids and X may represent one arbitrary amino acid.

The peptide may be a peptide of any one sequence selected from the group consisting of SEQ ID NOS: 201 to 400 (see Table 11).

The present invention is directed to an acetylcholine receptor-binding peptide containing an amino acid sequence set forth in Formula 4 below:

[Formula 4] X_{L}-(K or R)-X-(K or R)-(K or R)-X_{M-1}-(K or R)-X_{N}

wherein X_{L}, X_{M-1} and X_{N} each may independently represent a sequence composed of one to three arbitrary amino acids and X may represent a sequence composed of one arbitrary amino acid and, preferably, X_{L}, X_{M-1}, and X_{N} each may independently represent three arbitrary amino acids and X may represent one arbitrary amino acid.

The peptide may be a peptide of any one sequence selected from the group consisting of SEQ ID NOS: 401 to 600 (see Table 12).

The peptides may be composed of an 8- to 28-amino acid sequence. The number of amino acids is preferably 8 to 16, more preferably 8 to 14, still more preferably 11 to 16, and most preferably 14.

In the acetylcholine receptor-binding peptide, based on the amino acid sequence set forth in Formula 1, the 1st, 3rd, and 8th amino acids, which are K or R, may be important sites in acetylcholine receptor binding.

In the acetylcholine receptor-binding peptide, based on the amino acid sequence set forth in Formula 2, the 1st, 3rd, 4th, and 8th amino acids, which are K or R, may be important sites in acetylcholine receptor binding.

The amino acid sequences of the acetylcholine receptor-binding peptides exclude the amino acid sequences disclosed in Korean Patent No. 10-1971092, which corresponds to a prior patent of the present invention. The amino acid sequences disclosed in Korean Patent No. 10-1971092 include WTWKGRKSLLR.

The amino acid sequences of the acetylcholine receptor-binding peptides exclude the amino acid sequences disclosed in Korean Patent Publication No. 102014-0139010. The amino acid sequences disclosed in Korean Patent Publication No. 10-2014-0139010 are peptides of (gly)ₙ₁-(arg)ₙ₂, (gly)ₚ-RGRDDRRQRRR-(gly)_{q}, (gly)ₚ-YGRKKRRQRRR-(gly)_{q}, and (gly)ₚ-RKKRRQRRR-(gly)_{q}, wherein the subscripts p and q each are independently an integer of 0 to 20; the subscript n1 is independently an integer of 1 to 8; and the subscript n2 is independently an odd number from 7 to 17.

The present invention is directed to acetylcholine receptor-binding peptides obtained by modifying the N-terminus or C-terminus of the above-described peptides.

The N-terminus or C-terminus may be modified by palmitoylation, acetylation, amidation, formylation or PEGylation, or by a linkage of at least one selected from the group consisting of 2-mercaptoacetic acid, 3-mercaptopropionic acid, 6-mercaptohexanoic acid, pyroglutamic acid, succinimide acid, cystramine, cysteamine, methyl ester, ethyl ester, benzyl ester, myristic acid, stearic acid, palmitic acid, cholesterol, 6-amino hexanoic acid, and 8-amino octanoic acid.

The present invention is directed to an acetylcholine receptor-binding peptide.

As used herein, the term "peptide" refers to a polymer consisting of two or more amino acids joined together by amide linkage or peptide linkage.

As used herein, the term "amino acid" includes all natural amino acids and other amino acids, for example, L- and D-isomers of natural amino acids, unnatural amino acids, amino acids not encoded by nucleotide sequences, and the like, which are used to prepare synthetic peptides in the field of peptides.

The natural amino acids may be alanine (Ala, A), cysteine (Cys, C), aspartic acid (Asp, D), glutamic acid (Glu, E), phenylalanine (Phe, F), glycine (Gly, G), histidine (His, H), isoleucine (Ile, I), lysine (Lys, K), leucine (Leu, L), methionine (Met, M), asparagine (Asn, N), proline (Pro, P), glutamine (Gln, Q), arginine (Arg, R), serine (Ser, S), threonine (Thr, T), valine (Val, V), tryptophan (Trp, W), and tyrosine (Tyr, Y).

The other amino acids may be 2-aminoadipic acid (2-aminohexanedioic acid), α-asparagine, 2-aminobutanoic acid, 2-aminocapric acid (2-aminodecanoic acid), α-glutamine, α-aminoisobutyric acid (α-methyl alanine), 2-aminopimelic acid (2-aminohepanedioic acid), γ-amino-β-hydroxybenzenepentanoic acid, 2-aminosuberic acid (2-aminooctanedioic acid), 2-carboxyazetidine, β-alanine, β-aspartic acid, 3,6-diaminohexanoic acid (β-lysine), butanoic acid, 4-amino-3-hydroxybutanoic acid, γ-amino-β-hydroxycyclohexanepentanoic acid, 3-cyclohexylalanine, N5-aminocarbonylornithine, 3-sulfoalanine, 2,3-diaminopropanoic acid, 2,7-diaminosuberic acid (2,7-diaminooctanedioic acid), S-ethylthiocysteine, γ-glutamic acid, γ-carboxylglutamic acid, hydroxyacetic acid (glycolic acid), pyroglutamic acid, homogrginine, homocysteine, homohistidine, 2-hydroxyisovaleric acid, homoserine, 2-hydroxypentanoic acid, 5-hhydroxylysine, 4-hydroxyproline, isovaline, 2-hydroxypropanoic acid (lactic acid), mercaptoacetic acid, mercaptobutanoic acid, 3-hydroxy-4-methylproline, mercaptopropanoic acid, 3-naphthylalanine, norleucine, nortyrosine, norvaline, 2-carboxyoctahydroindole, ornithine, penicillamine (3-mercaptovaline), 2-phenylglycine, 2-carboxypiperidine, sarcosine (N-methylglycine), 1-amino-1-carboxycyclopentane, statin (4-amino-3-hydroxy-6-methylheptanoic acid), 3-thienylalanine, 3-carboxyisoquinoline, 3-methylvaline, ε-N-trimethyllysine, 3-thiazolylalanine, α-amino-2,4-dioxopyrimidinepropanoi c acid, and the like.

The peptides of the present invention may be prepared by the methods widely known in the art. Specifically, the peptides of the present invention may be prepared by using genetic recombination or protein expression systems, or may be prepared by a method of synthesis in vitro through chemical synthesis, such as peptide synthesis, and a cell-free protein synthesis method. More specifically, the peptides may be produced by well-known methods in the art, for example, an automated peptide synthesizer, and may be produced by genetic manipulation technology, but is not limited thereto. For example, a desired peptide may be produced by preparing a gene encoding a fusion protein composed of a fusion partner and the peptide of the present invention through genetic manipulation; transforming the prepared gene into a host microorganism; expressing the gene in the form of a fusion protein in the host microorganism; and cleaving and isolating the peptide of the present invention from the fusion protein via protease or compounds.

The peptides of the present invention may be present in the form of a salt. A salt form usable in the present invention may be prepared during the final isolation and purification of compounds or by reaction of an amino group with an appropriate acid. Examples of an acid addition salt may be acetates, adipates, alginates, citrates, aspartates, benzoates, benzenesulfonates, bisulfates, butyrates, camphorates, camphorsulfonates, digluconates, glycerophosphates, hemisulfates, heptanoates, hexanoates, formates, fumarates, hydrochloride, hydrobromide, hydroiodide, 2-hydroxyethanesulfonate, lactates, maleates, mesitylene sulfonate, methane sulfonate, naphthylene sulfonate, nicotinates, 2-naphthalenesulfonate, oxalates, pamoates, pectinates, persulfates, 3-phenylpropionate, picrates, pivalates, propionates, succinates, tartrates, trichloroacetates, trifluoroacetates, phosphates, glutamates, bicarbonates, para-toluene sulfonates, and undecanoates, but are not limited thereto. Also, examples of acids usable to form the acid addition salts may include inorganic acids, such as hydrochloric acid, hydrobromic acid, sulfuric acid, and phosphoric acid; and organic acids, such as oxalic acid, maleic acid, succinic acid, and citric acid, but are not limited thereto.

With respect to the peptides, a targeting sequence, a tag, a labeled residue, or an amino acid sequence designed for a purpose of increasing stability of the peptides may be added; an antibody or an antibody fragment thereof, human serum albumin (HSA), and the like for increasing targeting, efficacy increase, or stability may be bound; and the N-termini or C-termini of the peptides may be modified.

The term "antibody" refers to a specific protein molecule that is directed to an antigenic site. Preferably, the antibody refers to an antibody that specifically binds to a specific protein or an immunogenic fragment thereof, and may include all of monoclonal antibodies (mAb), polyclonal antibodies (pAb), and recombinant antibodies. The antibody may be easily produced using a known technique widely known in the art.

The antibody may include a complete form having two full-length light chains and two full-length heavy chains as well as a functional fragment of an antibody molecule. The functional fragment of the antibody molecule refers to a fragment retaining at least an antigen-binding function, and includes Fab, F(ab'), F(ab')₂, F(ab)₂, Fv, and the like.

The peptides may be encapsulated or immobilized in nanoparticles, microparticles, metal particles, ceramic particles, hydrogels, and the like, for delivery to specific tissues or to ensure stability, but are not limited thereto.

The nanoparticles, microparticles, metal particles, ceramic particles, hydrogels, and the like may be biocompatible and non-toxic.

As used herein, the term "acetylcholine receptor (AchR)" refers to a receptor to which acetylcholine secreted from the nerve endings is bound, and serves as a route for transmitting nerve stimulation by acetylcholine. For example, when in need of muscle contraction, the nerve sends a command to the muscle to contract, the nerve allows the secretion of acetylcholine at the site where the nerve and the muscle meet each other, and the secreted acetylcholine binds to the muscle's acetylcholine receptor, allowing the muscle to contract.

The acetylcholine receptors in the present invention are classified into muscarinic acetylcholine receptors and nicotinic acetylcholine receptors, and the acetylcholine receptors in the present invention are preferably nicotinic acetylcholine receptors.

The acetylcholine receptor-binding peptides bind to acetylcholine receptors, thereby preventing the binding of acetylcholine to the receptors, and thus can inhibit the actions of the acetylcholine receptors. Preferably, the peptides can relieve wrinkles and suppress abnormal muscle contraction by inhibiting muscle contraction; and, during surgery, can secure the convenience of surgery by promoting muscle relaxation.

Furthermore, the present invention provides a polynucleotide encoding the acetylcholine receptor-binding peptide. A polynucleotide containing a nucleotide sequence that is homologous to the nucleotide sequence constituting the polynucleotide may also be included in the scope of the polynucleotide provided in the present invention as long as the polynucleotide can encode a peptide capable of exhibiting binding activity to the acetylcholine receptor. Such a polynucleotide is preferably a polynucleotide containing an amino acid sequence showing at least 80% homology, more preferably a polynucleotide containing an amino acid sequence showing at least 90% homology, and most preferably a polynucleotide containing an amino acid sequence showing at least 95% homology.

Furthermore, the present invention provides a cosmetic composition, for wrinkle relief, containing the acetylcholine receptor peptide.

The acetylcholine receptor-binding peptide can relieve wrinkles by inhibiting the action of the acetylcholine receptor to prevent muscle contraction.

The cosmetic composition may further contain the acetylcholine receptor-binding peptide, and an adjuvant commonly used in the field of cosmetics, for example, a hydrophilic or lipophilic gelling agent, a hydrophilic or lipophilic activator, a preservative, an antioxidant, a solvent, a flavoring agent, a filler, a blocker, a pigment, a deodorant, or a dye.

The amount of the adjuvant is an amount that is commonly used in the art and, in any case, the adjuvant and the proportion thereof may be for selected so as not to adversely affect desirable properties of the cosmetic composition according to the present invention.

The cosmetic composition for wrinkle relief may be prepared by further containing an additive.

The additive may be a moisturizer, a functional raw material, a thickener, a softener, an emulsifier, a surfactant, a pH adjuster, and the like.

The moisturizer may include glycerin, propylene glycol, butylene glycol, hyaluronic acid, a ceramide component, and the like, but is not limited thereto.

The thickener may include a polymer, xanthan gum, and guar gum, but is not limited thereto.

The softener may include mineral oil, shea butter, or paraffin, but is not limited thereto.

The emulsifier may include dimethicone, beeswax, and the like.

The cosmetic composition for wrinkle relief may be used by mixing with a raw material having a wrinkle relief effect.

The raw material having a wrinkle relief effect may include vitamin A, a vitamin A derivative (retinyl palmitate, retinyl acetate, etc.), adenosine, and polyethoxylated retinamide, but is not limited thereto.

The cosmetic composition may be in the formulation of at least one selected from the group consisting of a lotion, a skin softener, a skin toner, an astringent, a cream, a foundation, an essence, a pack, a mask pack, a soap, a body cleanser, a cleansing foam, a body oil, and a body lotion, but is not limited thereto.

The cosmetic composition may be used every day, and may also be used even for an undetermined period, and preferably, the amount of use, the number of times of use, and the period of the cosmetic composition may be adjusted according to user's age, skin condition, or skin type.

Furthermore, the present invention provides a pharmaceutical composition for prevention or treatment of an acetylcholine receptor hyperactivity-associated disease, the pharmaceutical composition containing the acetylcholine receptor-binding peptide.

The acetylcholine receptor-binding peptide can bind to an acetylcholine receptor to inhibit the activation of the acetylcholine receptor, thereby preventing or treating the acetylcholine receptor hyperactivity-associated disease.

The acetylcholine receptor hyperactivity-associated disease refers to a disease in which the muscle contracts abnormally excessively, and examples thereof may be cervical dystonia, limb dystonia, truncal dystonia, blepharospasm, spasticity, hemifacial spasm, strabismus, nystagmus, tics, chronic pain, chronic migraine, neurogenic bladder, detrusor-sphincter dyssynergia, achalasia cardia, hyperhidrosis, sialorrhea, pediatric cerebral palsy, post-stroke muscle stiffness, back pain, enlarged prostate, urinary incontinence, vocal cord nodules and correction, hemorrhoids, dentition, and the like.

In addition, the pharmaceutical composition can be used to secure the convenience of surgery by promoting muscle relaxation during surgery, and can be used as a therapeutic agent or adjuvant for diseases caused by nicotine addiction, used for wrinkle removal, and used for square jaw or calf correction, but is not limited thereto.

The pharmaceutical composition may contain the acetylcholine receptor-binding peptide and a pharmaceutically acceptable excipient.

The pharmaceutical composition may be formulated in the forms of: an oral formulation, such as a powder, granules, a tablet, a capsule, a suspension, an emulsion, a syrup, or an aerosol; an externally applied preparation; a suppository; and a sterile injectable solution, according to usual methods, respectively. Examples of a carrier, an excipient, and a diluent that may be contained in the pharmaceutical composition may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil. The pharmaceutical composition may be prepared by using a diluent or an excipient that is usually used, such as a filler, an extender, a binder, a humectant, a disintegrant, or a surfactant. Solid preparations for oral administration include a tablet, a pill, a powder, granules, a capsule, and the like. These solid preparations may be prepared by mixing the acetylcholine receptor-binding peptide with at least one excipient, for example, starch, calcium carbonate, sucrose or lactose, gelatin, or the like. Also, a lubricant, such as magnesium stearate or talc, may be used in addition to simple excipients. Liquid preparations for oral administration correspond to a suspension, a liquid for internal use, an emulsion, a syrup, and the like, and may contain simple diluents that are frequently used, such as water and liquid paraffin, as well as several excipients, such as a humectant, a sweetener, a flavoring agent, and a preservative. Preparations for parenteral administration include a sterile aqueous solution, a non-aqueous solvent, a suspension, an emulsion, a freeze-drying agent, and a suppository. Examples of the non-aqueous solvent and suspension may include propylene glycol, polyethylene glycol, vegetable oils such as olive oil, injectable esters such as ethyl oleate, and the like. A base material for the suppository may include Witepsol, Macrogol, Tween 61, cocoa butter, laurin butter, glycerogelatin, and the like.

Although not particularly limited to the formulation, the pharmaceutical composition may be used as an externally-applied preparation for skin, having one formulation selected from an ointment agent, a lotion agent, a spray agent, a patch agent, a cream agent, a gel agent, and a gel. The pharmaceutical composition may contain an agent for increasing transdermal absorption, such as, but not limited to, dimethyl sulfoxide, dimethylacetamide, dimethylformamide, a surfactant, an alcohol, acetone, propylene glycol, or polyethylene glycol. The frequency of application may vary significantly depending on the age, sex, and weight of a subject to be treated, a specific disease or pathological condition to be treated, the severity of a disease or pathological condition, the route of administration, and the judgment of a prescriber. The frequency of application may range from once a month up to 10 times a day, preferably from once a week up to 4 times a day, more preferably from three times a week up to three times a day, still more preferably one or two times a day.

The pharmaceutical composition of the present invention may be administered to mammals, such as a rat, livestock, and a human, through various routes. All modes of administration may be expected, and for example, administration may be conducted orally, rectally, or by intravenous, intramuscular, subcutaneous, transdermal, endometrial, or intracerebrovascular injection. Preferably, administration may be conducted by transdermal injection.

Furthermore, the present invention is directed to a health functional food composition, containing the acetylcholine receptor-binding peptide, for alleviating an acetylcholine receptor hyperactivity-associated disease.

The health functional food composition may contain the acetylcholine receptor-binding peptide and a food acceptable food supplement additive.

The health functional food composition of the present invention includes forms of a tablet, a capsule, a pill, a liquid preparation, and the like, and examples of foods to which the acetylcholine receptor-binding peptide of the present invention can be added include various kinds of foods, beverages, gums, teas, vitamin complexes, and health functional foods.

In accordance with still another aspect of the present invention, there is provided a composition for a medicinal device, the composition containing the acetylcholine receptor-binding peptide.

The composition for a medicinal device may be a filler, but is not limited thereto.

As used herein, the term "filler" refers to a substance that can supplement skin tissues, and has the purpose of filling through injection for restoration of the resilient face, improvement of facial contour, and relief of wrinkles.

The composition for a medicinal device can relieve wrinkles by suppressing muscle contraction and can exhibit a contour improving effect, through the acetylcholine receptor-binding peptide, and microparticles, nanoparticles, and hydrogels, on which the acetylcholine receptor-binding peptide is immobilized, can be injected to fill tissue.

### Advantageous Effects

The present invention relates to acetylcholine receptor inhibitory peptides and uses thereof, wherein phages having high binding affinity to acetylcholine receptors were screened using random peptide recombinant phages and acetylcholine receptor-binding peptides were selected through phage DNA. By using the selected peptides, the acetylcholine receptor binding affinity and acetylcholine receptor inhibitory effects were verified, and through the modification of peptides, the sites and sequences of the peptides, which are crucial to acetylcholine receptor binding, were identified.

Furthermore, the identified amino acid sequences of peptides were expressed by general formulas, on the basis of the crucial peptide sites and sequences, and the libraries of the peptides were constructed, and it was verified that the constructed libraries were generally bound to acetylcholine receptors to inhibit the action of the acetylcholine receptors.

It is therefore expected that the acetylcholine receptor inhibitory peptides of the present invention can be used to develop a cosmetic composition for wrinkle relief, a medicinal product for preventing or treating an acetylcholine receptor-associated disease, and a health functional food for alleviating an acetylcholine receptor-associated disease.

### Brief Description of the Drawings

FIG. 1 shows the results of analyzing the acetylcholine receptor binding affinity of screened and selected peptides (Spep-1 to Spep-11).
FIG. 2 shows the results of investigating the acetylcholine receptor inhibitory effect of the selected peptides in TE671 cells. (A) to (C) show the acetylcholine receptor inhibitory effect by acetylcholine according to the concentrations of Synake, Spep-1, Spep-2, Spep-4, and Spep-10 peptides; and (D) shows the acetylcholine receptor inhibitory effect by nicotine.
FIG. 3 shows the cytotoxicity results of Synake, Spep-1, and Spep-2 peptides on TE671 cells. All had no cytotoxicity even at a high concentration of 100 µM.
FIG. 4 shows the results of investigating acetylcholine receptor binding affinity according to concentration of Spep-2.
FIG. 5 shows the results of investigating sites of the sequence, which are crucial to acetylcholine receptor binding according to the peptide size, by using peptides with both the truncated termini in the amino acid sequence of Spep-2. (A) shows the investigation results using peptides with the truncated N-terminus (Spep-2-ND1 to -ND5) and (B) shows the investigation results using peptides with the truncated C-terminus (Spep-2-CD1 to -CD5).
FIG. 6 shows the results of comparing acetylcholine receptor binding affinity of (A) Spep-2-ND3 and (B) Spep-2-ND5. Compared with the binding affinity of Spep-2 (11-mer), the binding affinity of down to Spep-ND3 (8-mer) showed no significant difference, but the binding affinity of Spep-2-ND5 (6-mer) was reduced by 48 times.
FIG. 7 shows the results of investigating crucial sites in acetylcholine receptor binding by using alanine-scanning based on the amino acid sequences of Spep-1-ND3 (A) and Spep-2-ND3 (B).
FIG. 8 shows the results of comparing the acetylcholine receptor binding affinity between palmitoyl-conjugated Spep-2 and Spep-2. It can be verified that the conjugation of palmitoyl enhanced the binding affinity.
FIG. 9 verifies that palmitoyl-Spep-2 formed micelles. Pal-Spep-2 formed micelles and enhanced the binding affinity by the avidity effect.
FIG. 10 shows the results of comparing the AchR inhibitory effect of Palmitoyl-Spep-2 at different concentrations with those of Spep-2, Synake, and Synake, and bungarotoxin. As the binding affinity of Pal-Spep-2 was enhanced, the inhibitory ability of Pal-Spep-2 was also enhanced by about 10 times compared with Spep-2.
FIG. 11 shows the cytotoxicity results of palmitoyl-conjugated Spep-2 on TE671 cells. Palmitoyl-conjugated Spep-2 showed no cytotoxicity even at a high concentration of 10 µM.
FIG. 12 shows the results of investigating the AchR binding affinity of peptides in which K was substituted with R and R was substituted with K or N in the amino acid sequence of Spep-2-ND3 as a wild type, by the same method as the surface plasmon resonance analysis. The activity was maintained in the substitution with K or R, but the activity was significantly reduced in the substitution with N.

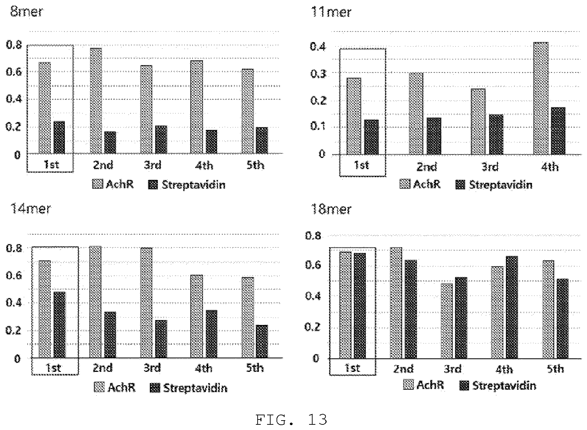

FIG. 13 shows the results of investigating binding affinity and specificity of phages to acetylcholine receptors, wherein the phages were screened in the biopanning of 8-mer L1 [((K or R)-X-(K or R)(K or R)-XXX-(K or R), X is a sequence composed of one arbitrary amino acid], 11-mer L2 [XXX(K or R)-X-(K or R)(K or R)-XXX-(K or R), X is a sequence of one arbitrary amino acid], 14-mer L3 [XXX (K or R)-X-(K or R) (K or R)-XXX- (K or R)XXX, X is a sequence of one arbitrary amino acid], and 18-mer L4[XXXXX(K or R)-X-(K or R)(K or R)-XXX-(K or R)XXXXX, X is a sequence of one arbitrary amino acid], which are peptide libraries optimized for acetylcholine receptors. The boxed parts are the first libraries, and it was verified that the first libraries among all of the L1 (8-mer), L2 (11-mer), and L3 (14-mer) libraries already showed high binding affinity and specificity to acetylcholine receptors, but the L4 (18-mer) library with a largest peptide size had no binding affinity and specificity to acetylcholine receptors.
FIG. 14 shows the result of analyzing the ratio of acetylcholine receptor absorbance to streptavidin absorbance when peptide phages specific to acetylcholine receptors were screened in the 4th and 5th biopanning of the optimized peptide L1 (8-mer) libraries. Spep-2 was also used as a control, and it was verified that all the peptides had higher binding affinity and specificity than Spep-2.
FIG. 15 shows the result of analyzing the ratio of acetylcholine receptor absorbance to streptavidin absorbance when peptide phages specific to acetylcholine receptors were screened in the 4th and 5th biopanning of the optimized peptide L2 (11-mer) libraries. Spep-2 was also used as a control, and it was verified that all the peptides had higher binding affinity and specificity than Spep-2.
FIG. 16 shows the result of analyzing the ratio of acetylcholine receptor absorbance to streptavidin absorbance when peptide phages specific to acetylcholine receptors were screened in the 4th and 5th biopanning of the optimized peptide L3 (14-mer) libraries. Spep-2 was also used as a control, and it was verified that all the peptides had higher binding affinity and specificity than Spep-2.
FIG. 17 shows the results of comparing the binding affinity to acetylcholine receptors among 40 peptides with excellent specificity selected from the optimized peptide L1 (8-mer) library. All the optimized peptides showed higher binding affinity than Spep-2.
FIG. 18 shows the results of comparing the binding affinity to acetylcholine receptors among 40 peptides with excellent specificity selected from the optimized peptide L2 (11-mer) library. All the optimized peptides showed higher binding affinity than Spep-2.
FIG. 19 shows the results of comparing the binding affinity to acetylcholine receptors among 40 peptides with excellent specificity selected from the optimized peptide L3 (14-mer) library. All the optimized peptides showed higher binding affinity than Spep-2.
FIG. 20 shows the results of comparing the acetylcholine receptor inhibitory effect of 20 peptides in the highest order among 40 optimized L1 (8-mer) peptides. All the optimized peptides showed higher inhibitory ability than Spep-2.
FIG. 21 shows the results of comparing the acetylcholine receptor inhibitory effect of 20 peptides in the highest order among 40 optimized L2 (11-mer) peptides. All the optimized peptides showed higher inhibitory ability than Spep-2.
FIG. 22 shows the results of comparing the acetylcholine receptor inhibitory effect of 20 peptides in the highest order among 40 optimized L3 (14-mer) peptides. All the optimized peptides showed higher inhibitory ability than Spep-2.
FIG. 23 shows the results of comparing the acetylcholine receptor binding affinity among representative peptides with the highest affinity among the optimized peptides L1 (8-mer), L2 (11-mer), and L3 (14-mer).
FIG. 24 shows the results of comparing the acetylcholine receptor inhibitory between Spep-2 and the selected representative peptides according to the concentration.
FIG. 25 shows the results of comparing the multiples of acetylcholine receptor inhibitory effects between Spep-2 and the selected representative peptides. L3-37 peptide had an inhibitory ability enhanced by 32 times compared with Spep-2.
FIG. 26 shows the result of comparing the binding affinity to acetylcholine receptors through various modifications of a terminus of the representative L3-37 peptide.
FIG. 27 shows the result of comparing the binding affinity to acetylcholine receptors through various modifications of a terminus of the representative L3-28 peptide.
FIG. 28 shows the result of comparing the binding affinity to acetylcholine receptors through various modifications of a terminus of the representative L3-27 peptide.
FIG. 29 shows the result of comparing the binding affinity to acetylcholine receptors through various modifications of a terminus of the representative L2-110 peptide.
FIG. 30 shows the result of forming micelles by Myristic-L3-37 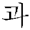 Stearic-L3-37.
FIG. 31 shows the results of comparing the AchR inhibitory effect of Palmitoyl-L3-37, Palmitoyl-L3-28, and Palmitoyl-L3-27, Palmitoyl-L2-110 at different concentrations compared with bungarotoxin.
FIG. 32 shows the cytotoxicity results of Palmitoyl-L3-37 on TE671 cells.
FIG. 33 shows the cytotoxicity results of Palmitoyl-L3-28 on TE671 cells.
FIG. 34 shows the cytotoxicity results of Palmitoyl-L3-27 on TE671 cells.
FIG. 35 shows the cytotoxicity results of Palmitoyl-L2-110 on TE671 cells.
FIG. 36 shows the results of animal efficacy assay for Palmitoyl-L3-37 peptide by using Catwalk equipment.
FIG. 37 shows the results of using DAS assay to perform animal efficacy assay of Palmitoyl-L3-37 peptide at different concentrations.
FIG. 38 shows the results of restoration of animal efficacy of Palmitoyl-L3-37 peptide after DAS assay.
FIG. 39 shows the results of animal acute toxicity assay of Palmitoyl-L3-37 peptide.

### Mode for Carrying Out the Invention

Hereinafter, preferable exemplary embodiments of the present invention will be described in detail. However, the present invention is not limited to the exemplary embodiments described herein and can be embodied in many different forms. Rather, these exemplary embodiments are provided so that the present disclosure will be thorough and complete and will fully convey the scope of the disclosure to those skilled in the art.

### <Example 1: Acetylcholine receptor-binding peptide screening through biopanning>

The present inventors produced random recombinant phages through random peptide library DNA preparation and transformation and screened acetylcholine receptor (AchR)-binding peptides through biopanning, according to the methods described in a prior patent (Korean Patent No. 10-1971092). Table 1 shows the sequencing results of AchR-specific peptides selected from the input phages having high affinity and specificity to AchR in the 4th and 5th rounds of biopanning.

**TABLE 1**

| Peptide Name | Amino acid sequence |
|---|---|
| Spep-1 | WTWKGKGTLNR |
| Spep-2 | WTWKGRKSLLR |
| Spep-3 | WTWKGEDKGKN |
| Spep-4 | WTWKGRDKLQM |
| Spep-5 | WTWKGQLGQLS |
| Spep-6 | WTWKGGRLSAS |
| Spep-7 | WTWKGRQLNNQ |
| Spep-8 | WTWKGDNLQNN |
| Spep-9 | WTWKGLYQRLG |
| Spep-10 | WTWKGNKQVKF |
| Spep-11 | WTWKGETYDSK |

### <Example 2: Investigating acetylcholine receptor binding affinity of peptides>

Spep-1 to Spep-11 selected in Example 1 were synthesized, and the AchR binding affinity was compared thereamong using a surface plasmon resonance (SPR) analyzer (Biacore3000, Biacore A, Sweden).

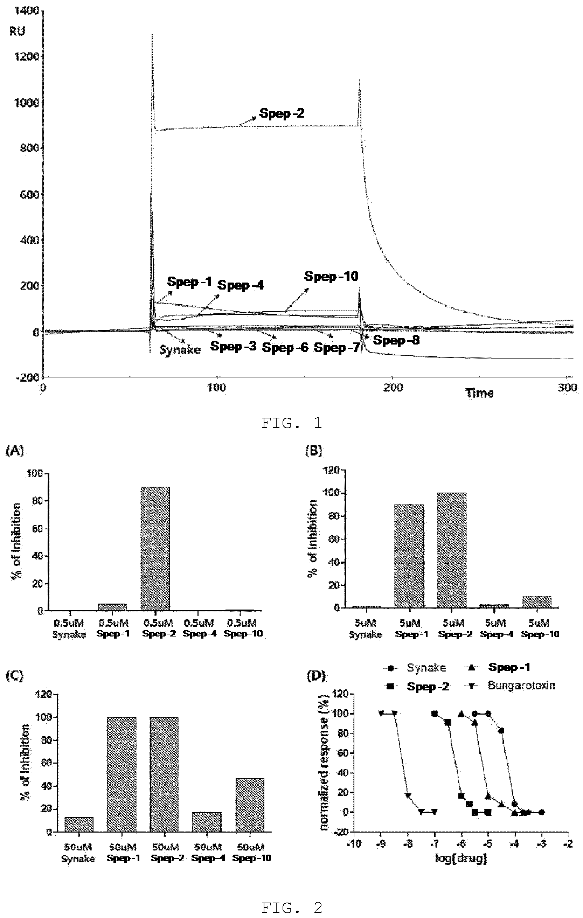

Acetylcholine receptor proteins were immobilized on CM5 chips, which are biosensor chips for surface plasmon resonance analysis, using EDC/NHS, and then analysis was performed under conditions of 20 mM Tris (pH 7.0) running buffer, rate of 30 µl/min, and 10 µM peptides (Spep-1 to Spep-11). The association and dissociation were observed for up to 500 seconds, and the results are shown in FIG. 1. Synake known to relieve wrinkles by binding to AchR was used as a positive control.

As shown in FIG. 1, Spep-1, Spep-2, Spep-4, and Spep-10 showed high AchR binding affinity compared with Synake known to bind to AchR.

### <Example 3: Investigating acetylcholine receptor inhibition of peptides>

To investigate AchR inhibitory effects of the peptides having high acetylcholine receptor binding affinity in Example 2, AchR-overexpressed TE671 cells were used.

TE671 cells were cultured for 4 days using DMEM containing 10% fetal bovine serum (FBS) and 1% penicillin/streptomycin. The cultured cells were detached by trypsinization, and then inoculated at 2 ×10⁴ cells/well in 12-well cell culture plates with 18-mm cover slides placed therein, and cultured for 4 days. The cover slides with the cultured cells were transferred in new 12-well plates, and 997 µl of Hanks' balanced salt solution and 3 µl of Fura-2-AM (intracellular calcium ion indicator) were added thereto, followed by culture in a 5% CO2 incubator at 37°C for 15 minutes. Thereafter, the cells were washed three or four times with HBSS buffer to remove the remaining Fura-2-AM, and then 1 ml of HBSS buffer was added thereto. The cover slides with cells grown thereon were inserted into a microscopic observation chamber, and then 500 µl of HBSS buffer was added thereto. Thereafter, the peptides selected in Example 2 were added at 0.5, 5, or 50 µM, and then left for about 3 minutes so as to bind to AchR. Thereafter, the fluorescence intensity of Fura-2 in the cells was measured by addition of 125 µM nicotine or 1 µM acetylcholine, to thereby investigate the action of AchR. Synake and bungarotoxin were used as positive controls. The results are shown in FIG. 2.

As shown in FIG. 2, as for acetylcholine treatment (A to C), Spep-1 showed an AchR inhibitory effect of 5% at 0.5 µM, 90% at 5 µM, and 100% at 100 µM; and Spep-2 showed an AchR inhibitory effect of 90% at 0.5 µM and 100% at 5 µM; and Spep-4 and Spep-10 showed AchR inhibitory effects of 17% and 47% at 50 µM, respectively. As for nicotine treatment (D), Spep-1 and Spep-2, compared with Synake, showed excellent nicotinic acetylcholine receptor inhibitory effects.

It can be therefore seen that Spep-1 and Spep-2 had excellent AchR inhibitory effects, and especially, Spep-2 had the highest binding affinity to AchR, leading to the highest AchR inhibitory effect.

### <Example 4: Measuring cytotoxicity of Synake, Spep-1, and Spep-2>

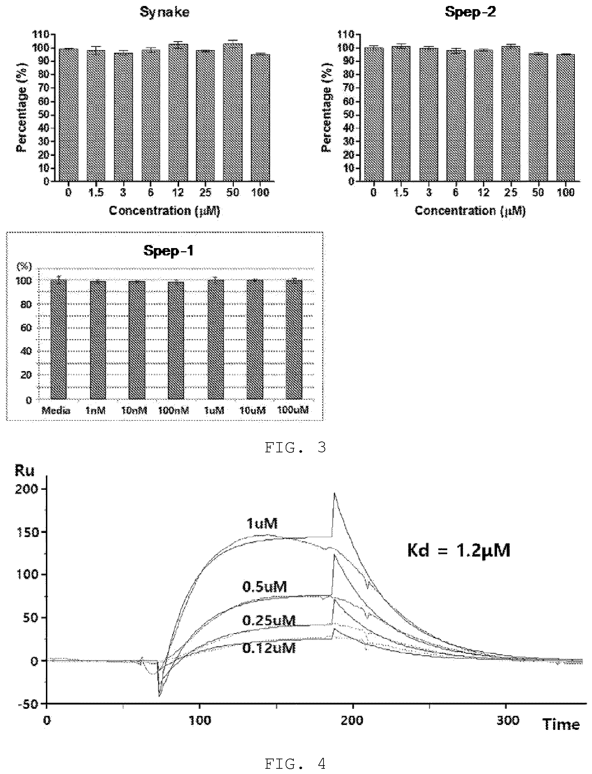

To evaluate the toxicity of Spep-1 and Spep-2, WST Assay was performed on TE671 cells. The toxicity of Synake was also evaluated, and the results are shown in FIG. 3.

As shown in FIG. 3, Spep-1, Spep-2, and Synake showed no toxicity even at a treatment concentration of 100 µM.

### <Example 5: Investigating acetylcholine receptor binding affinity of Spep-2 peptide with highest binding affinity>

To investigate the AchR binding affinity of Spep-2 with highest AchR binding affinity in Example 2, the dissociation constant (Kd) was analyzed using the surface plasmon analyzer in Example 2 by the same method, and the results are shown in FIG. 4. The treatment concentrations of Spep-2 were 0.12 to 1 µM, and the dissociation constant was compared while Synake was used as a positive control.

As shown in FIG. 4, the association curves rapidly increased as Spep-2 was allowed to flow, and the association curves increased in a dependent manner of the treatment concentration of Spep-2. After Spep-2 was allowed to completely flow, only a running buffer was allowed to flow for dissociation, during which the increased association curves were not reduced, indicating that Spep-2 was continuously bound to AchR.

The Kd value for AchR of Spep-2 was analyzed to be 1.2 µM, indicating that the AchR binding affinity was about 2,000 times superior to that of Synake, considering that the kd value of Synake was 2,300 µM.

### <Example 6: Investigating crucial site of peptide sequence in acetylcholine receptor binding>

### Example 6-1: Investigating acetylcholine receptor binding affinity through sequential deletion of amino acids from terminus

To identify the sites of a peptide, which play an important role in AchR binding, peptide modification was conducted based on the amino acid sequence of Spep-2 identified in Example 1 and the modified peptide was applied to the surface plasmon resonance analyzer in Example 2 to investigate the binding affinity to AchR.

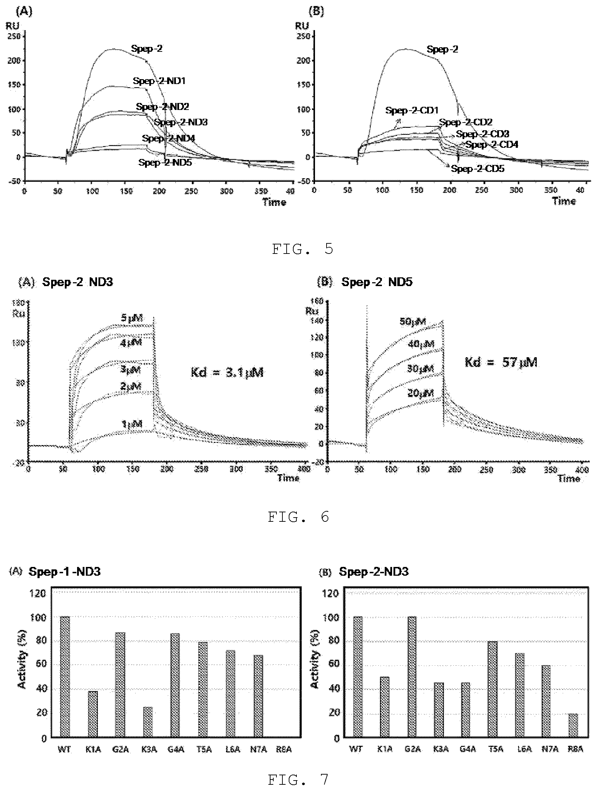

Based on the amino acid sequence of Spep-2, amino acids from the N-terminus or C-terminus of the peptide were stepwise deleted to synthesize the peptides shown in Table 2 below, and the AchR binding affinity of the synthesized peptides were compared, and the results are shown in FIG. 5.

**TABLE 2**

| Peptide name | Amino acid sequence |
|---|---|
| Spep-2 | WTWKGRKSLLR |
| Spep-2-ND1 | TWKGRKSLLR |
| Spep-2-ND2 | WKGRKSLLR |
| Spep-2-ND3 | KGRKSLLR |
| Spep-2-ND4 | GRKSLLR |
| Spep-2-ND5 | RKSLLR |
| Spep-2-CD1 | WTWKGRKSLL |
| Spep-2-CD2 | WTWKGRKSL |
| Spep-2-CD3 | WTWKGRKS |
| Spep-2-CD4 | WTWKGRK |
| Spep-2-CD5 | WTWKGR |

As shown in FIG. 5, in cases where the amino acids from the N-terminus of Spep-2 were deleted stepwise (A), Spep-2-ND1 to -ND3 showed a reduction in AchR binding affinity by about 50% compared with Spep-2, but still maintained the binding affinity to AchR, whereas Spep-2-ND4 and -ND5 showed significantly reduced binding affinity. In cases where the amino acids from the C-terminus of Spep-2 were deleted stepwise (B), all of Spep-2-CD1 to -CD5 showed rapid reductions in AchR binding.

It can be therefore seen that the 4th amino acid K (Lys) and 11th amino acid R (Arg) in the amino acid sequence of Spep-2 are crucial sites in AchR binding.

### Example 6-2: Investigating acetylcholine receptor binding affinity of modified peptides

Among the modified peptides of Spep-2 identified in Example 6-1, Spep-2-ND3, which was identified to have the minimum sequence necessary for binding to AchR, and Spep-2-ND5, which had a smaller size than Spep-2-ND3, were applied to the surface plasmon resonance analyzer in Example 2 to investigate the binding affinity to AchR, and the results are shown in FIG. 6.

As shown in FIG. 6, Spep-2-ND3 (A) showed higher binding affinity to AchR even at low concentrations compared with Spep-2-ND5 (B), and as a result of investigating the dissociation constant, Spep-2-ND3 was 3.1 µM, whereas Spep-2-ND5 was 57 µM, indicating that Spep-2-ND3 had significantly excellent AchR binding affinity compared with Spep-2-ND5.

It can be therefore seen that the 11-mer Spep-2 and the 8-mer Spep-2-ND3 did not have a large difference in AchR binding affinity, but the 6-mer Spep-2-ND5 showed a remarkable reduction in AchR binding affinity. These results indicate that three amino acids from the N-terminus of Spep-2 were not significantly crucial to AchR binding affinity.

### <Example 7: Identifying crucial sites of peptides in acetylcholine receptor binding by using alanine-scanning>

By using Spep-2-ND3 among the peptides identified in Example 6, and Spep-1-ND3 (KGKGTLNR), which has three amino acid deletions from the N-terminus of Spep-1 having high amino acid sequence similarity to Spep-2, alanine-scanning was conducted to identify crucial sites of each of the peptides.

Spep-1-ND3 and Spep-2-ND3 were set as a wild type, and the peptides in Table 3, in which alanine (Ala) substitution was sequentially conducted in the amino acid sequence of each peptide, were synthesized and then compared for AchR binding affinity by the same method as the surface plasmon resonance analysis in Example 2. The treatment concentration of each peptide was 20 µM, and the results are shown in FIG. 7.

**TABLE 3**

| Peptide name | Amino acid sequence | Peptide name | Amino acid sequence |
|---|---|---|---|
| Spep-1-ND3(WT) | KGKGTLNR | Spep-2-ND3(WT) | KGRKSLLR |
| Spep-1-ND3(K1A) | AGKGTLNR | Spep-2-ND3(K1A) | AGRKSLLR |
| Spep-1-ND3(G2A) | KAKGTLNR | Spep-2-ND3(G2A) | KARKSLLR |
| Spep-1-ND3(K3A) | KGAGTLNR | Spep-2-ND3 (R3A) | KGAKSLLR |
| Spep-1-ND3(G4A) | KGKATLNR | Spep-2-ND3(K4A) | KGRASLLR |
| Spep-1-ND3(T5A) | KGKGALNR | Spep-2-ND3(S5A) | KGRKALLR |
| Spep-1-ND3(L6A) | KGKGTANR | Spep-2-ND3(L6A) | KGRKSALR |
| Spep-1-ND3(N7A) | KGKGTLAR | Spep-2-ND3(L7A) | KGRKSLAR |
| Spep-1-ND3(R8A) | KGKGTLNA | Spep-2-ND3(R8A) | KGRKSLLA |

As shown in FIG. 7, the peptides in which the K at the 1st site, K at the 3rd site, and R at the 8th site were substituted with A in Spep-1-ND3 showed a reduction in AchR binding affinity (A); and the peptides in which the K at the 1st site, R at the 3rd site, K at the 4th site, and R at the 8th site were substituted with A in Spep-2-ND3 showed a reduction in AchR binding affinity. It can be therefore seen that K and R sites in Spep-1-ND3 and Spep-2-ND3 are crucial sites in AchR binding.

### <Example 8: Identifying acetylcholine receptor binding affinity of fatty acid derivative-conjugated Spep-2 according to peptide modification>

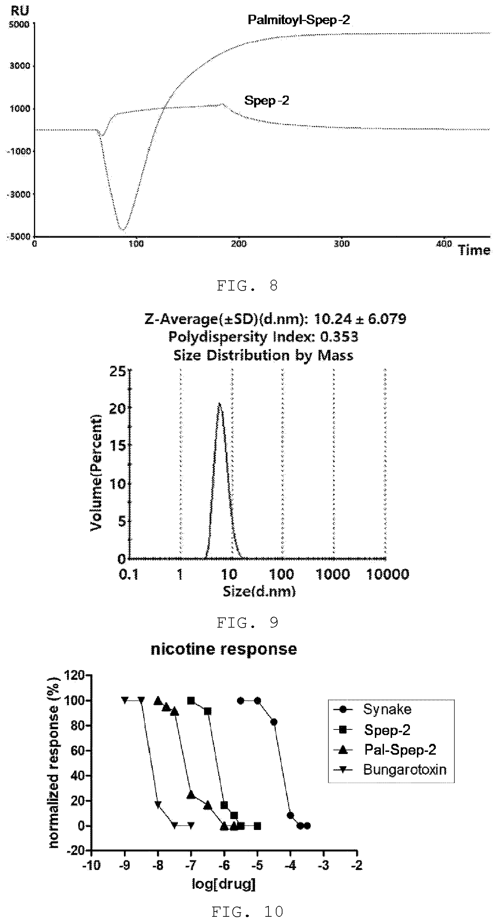

The change in AchR binding affinity according to the modification of peptides binding to AchR was investigated. Specifically, by using a peptide (Palmitoyl-Spep-2) obtained by conjugating palmitoyl as a fatty acid derivative to Spep-2 having the highest binding affinity to AchR among the peptides identified in Example 2, the AchR binding affinity was investigated by the same method as the surface plasmon resonance analysis in Example 2, and the results are shown in FIG. 8. The binding affinity between Spep-2 and AchR was used as a control.

As shown in FIG. 8, the AchR binding affinity of Palmitoyl-Spep-2 was stronger than that of Spep-2. The reason is that, as shown in FIG. 9, micelle structures in which the fat-soluble palmitoyl gather inward and the water-soluble Spep-2 is exposed outward bind to several sites of AchR to have an avidity effect.

### <Example 9: Investigating acetylcholine receptor inhibition of Palmitoyl-Spep-2>

The AchR inhibitory effect of Palmitoyl-Spep-2, which has been verified to have excellent AchR binding affinity in Example 8, was investigated. The AchR inhibitory effect was investigated by the same method as in Example 3, and the treatment with Palmitoyl-Spep-2 was conducted according to the concentration. For comparison of the AchR inhibitory effect, the treatment with Spep-2, Synake, and bungarotoxin as controls was conducted according to the concentration, and the results are shown in FIG. 10.

As shown in FIG. 10, Palmitoyl-Spep-2 showed a higher inhibitory effect on AchR than Spep-2. More specifically, as for IC50 value for AchR in each peptide, Synake was 75 µM; Spep-2 was 750 nM; Palmitoyl-Spep-2 was 75 nM; and bungarotoxin was 7.5 nM, indicating that the AchR inhibitory effect of Pal-Spep-2 was 10 times excellent compared with that of Spep-2.

### <Example 10: Measuring cytotoxicity of Palmitoyl-Spep-2>

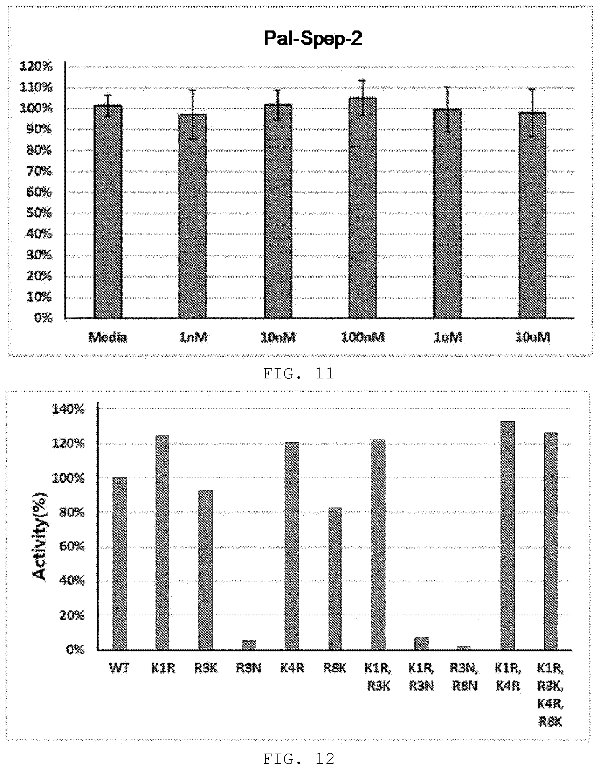

To evaluate cytotoxicity of Palmitoyl-Spep-2, WST assay was performed on TE671 cells, and the results are shown in FIG. 11.

As shown in FIG. 11, Palmitoyl-Spep-2 showed no cytotoxicity even at 10 µM.

### <Example 11: Investigating change in AchR binding affinity through amino acid substitutions of K and R in amino acid sequence of Spep-2-ND3>

It was investigated whether the AchR binding affinity was changed when in the amino acids K and R of Spep-2-ND3, which have been identified to be crucial sites in AchR binding in Example 7, K was substituted R and R was substituted with K or N.

Spep-2-ND3 was set to a wild type, and the peptides in Table 4, in which K was substituted with R and R was substituted with K or N in the amino acid sequence of Spep-2-ND3, were synthesized and then compared for AchR binding affinity by the same method as the surface plasmon resonance analysis in Example 2. The treatment concentration of each peptide was 20 µM, and the results are shown in FIG. 12.

**TABLE 4**

| Peptide name | Amino acid sequence |
|---|---|
| Spep-2-ND3(WT) | KGRKSLLR |
| Spep-2-K1R | RGRKSLLR |
| Spep-2-R3K | KGKKSLLR |
| Spep-2-R3N | KGNKSLLR |
| Spep-2-K4R | KGRRSLLR |
| Spep-2-R8K | KGRKSLLK |
| Spep-2-K1R-R3K | RGKKSLLR |
| Spep-2-K1R-R3N | RGNKSLLR |
| Spep-2-R3N-R8N | KGNKSLLN |
| Spep-2-K1R-K4R | RGRRSLLR |
| Spep-2-K1R-R3K-K4R-R8K | RGKRSLLK |

As shown in FIG. 12, the peptides in which K was substituted with R in Spep-2-ND3 showed an increase in AchR binding affinity, and the peptides in which R was substituted with K showed a slight reduction in AchR binding affinity, but no significant difference. However, the peptides in which R was substituted with N showed a significant reduction in AchR binding affinity. It can be therefore seen that the substitution of K and R with each other in Spep-2-ND3 made no difference in AchR binding, but the substitution of R with N resulted in no AchR binding affinity.

### <Example 12: Constructing peptide libraries for selection of optimized peptides with enhanced acetylcholine receptor binding affinity>

### Example 12-1: Random peptide library DNA construction and transformation

Through the results identified in Examples 6, 7, and 11, the Spep-2-ND3 peptide sequence was modified to construct random peptide libraries of 8-mer; (K/R)X(K/R)(K/R)XXX(K/R), 9-mer; X(K/R)X(K/R)(K/R)XXX(K/R), (K/R) X(K/R) (K/R) XXX (K/R) X, 10-mer; XX(K/R)X(K/R)(K/R)XXX(K/R), (K/R) X(K/R) (K/R) XXX (K/R) XX, X(K/R) X(K/R) (K/R) XXX (K/R) X, 11-mer; XXX(K/R)X(K/R)(K/R)XXX(K/R), (K/R)X(K/R)(K/R)XXX(K/R)XXX, X(K/R) X(K/R) (K/R) XXX (K/R) XX, XX(K/R)X(K/R)(K/R)XXX(K/R)X, 12-mer; X(K/R) X(K/R) (K/R) XXX (K/R) XXX, XX(K/R)X(K/R)(K/R)XXX(K/R)XX, XXX(K/R)X(K/R)(K/R)XXX(K/R)X, 13-mer; XX(K/R)X(K/R)(K/R)XXX(K/R)XXX, XXX(K/R)X(K/R)(K/R)XXX(K/R)XX, 14-mer; XXX(K/R)X(K/R)(K/R)XXX(K/R)XXX, and 18-mer; XXXXX(K/R)X(K/R)(K/R)XXX(K/R)XXXXX ((K/R) is one of either K or R and X is a random amino acid), and the random peptide libraries were investigated for the acetylcholine receptor inhibitory effect. The random libraries were verified to have acetylcholine receptor binding affinity similar or superior to that of Spep-2.

Hereinafter, an example will be described in which random peptide libraries of 8-mer; (K/R)X(K/R)(K/R)XXX(K/R), 11-mer; XXX(K/R)X(K/R)(K/R)XXX(K/R), 14-mer; XXX(K/R)X(K/R)(K/R)XXX(K/R)XXX, and 18-mer; XXXXX(K/R)X(K/R)(K/R)XXX(K/R)XXXXX ((K/R) is one of either K or R and X is a random amino acid) as representative peptides having excellent binding affinity to acetylcholine receptors was constructed and the acetylcholine receptor inhibitory effect was investigated.

To construct the peptide libraries of 8-mer; (K/R)X(K/R)(K/R)XXX(K/R), 11-mer; XXX(K/R)X(K/R)(K/R)XXX(K/R), 14-mer; XXX(K/R)X(K/R)(K/R)XXX(K/R)XXX, and 18-mer; XXXXX(K/R)X(K/R)(K/R)XXX(K/R)XXXXX ((K/R) is one of either K or R and X is a random amino acid), DNA libraries in Table 5 were synthesized (Bioneer, Korea). Thereafter, PCR was performed using two single-stranded primers (TTCTATGCGGCCCAG and AACAGTTTCTGCGGC) with the synthesized DNA libraries in Table 5 as templates, thereby amplifying insert DNA for insertion of double strands. The amplified insert DNA and the phagemid vector pIGT were digested with the restriction enzymes Sfi I and Not I, and each DNA was purified. The purified insert DNA and pIGT were ligated by T4 DNA ligase, and then precipitates obtained by ethanol precipitation were dissolved in Tris-EDAT (TE) buffer to prepare random peptide library DNA.

The prepared random peptide library DNA was added to and mixed with competent cells, and transformed using electroporation. The transformed cells were placed in a Luria Bertani (LB) liquid medium containing 20 mM glucose, transferred into a test tube, and then cultured at 37°C and 200 rpm for 1 hour. The cultured cells were placed in an LB liquid medium containing 20 mM glucose and 50 *µ*g/ml ampicillin, and cultured at 30°C for one day. After the one-day culture, the medium was centrifuged at 4°C and 4,000×g for 20 minutes to remove supernatant, thereby securing precipitated cells. The secured cells were suspended in an LB liquid medium, followed by the addition of glycerol to a final concentration of 20% or more, and then stored at -80°C, thereby securing random peptide libraries.

**TABLE 5**

| Library | Amino acid sequence | Nucleotide sequence |
|---|---|---|
| 8-mer (L1) | (K or R)-X-(K or R)(K or R)-XXX-(K or R), X=random amino acids | TTCTATGCGGCCCAGCTGGCCARANNKA RAARANNKNNKNNKARAGCGGCCGCAG AAACTGTT |
| 11-mer (L2) | XXX(K or R)-X-(K or R)(K or R)-XXX- (K or R), X=random amino acids | TTCTATGCGGCCCAGCTGGCCNNKNNKN NKARANNKARAARANNKNNKNNKARA GCGGCCGCAGAAACTGTT |
| 14-mer (L3) | XXX(K or R)-X-(K or R)(K or R)-XXX-(K or R)XXX, X=random amino acids | TTCTATGCGGCCCAGCTGGCCNNKNNKN NKARANNKARAARANNKNNKNNKARA NNKNNKNNKGCGGCCGCAGAAACTGTT |
| 18-mer | XXXXX(K or R)-X-(K or R)(K or R)-XXX-(K or R)XXXXX, X=random amino acids | TTCTATGCGGCCCAGCTGGCCNNKNNKN NKNNKNNKARANNKARAARANNKNNK NNKARANNKNNKNNKNNKNNKGCGGC CGCAGAAACTGTT |
| N : A or C or G or T / K : G or T / R : A or G / X : Random amino acids | | |

### Example 12-2: Optimized peptide recombinant phage production

The random peptide libraries secured in Example 12-1 were added to an SB liquid medium (3% tryptone, 2% yeast extract, 1% MOPS free acid, and 2% glucose), followed by culture at 37°C and 200 rpm for 20 minutes, and then 1×10¹⁰ pfu helper phages and ampicillin with a final concentration of 50 *µ*g/ml were added, followed by culture in the same conditions for 1 hour. The culture was transferred to an SB liquid medium containing 50 *µ*g/ml ampicillin and 10 *µ*g/ml kanamycin, and cultured in the same conditions for 16 hours, thereby producing random peptide recombinant phages.

The produced random peptide recombinant phages were centrifuged at 4°C and 5,000 rpm for 10 minutes to obtain supernatant, and the supernatant and polyethylene glycol (PEG)/NaCl (20% PEG, 15% NaCl) were mixed at 5:1 (v:v), left on ice for 1 hour, and centrifuged at 4°C and 13,000 rpm for 20 minutes to remove supernatant. The precipitates were suspended in 1 ml of phosphate buffered saline (PBS) to secure random peptide recombinant phages.

### <Example 13: Screening optimized peptides with enhanced binding affinity to acetylcholine receptors>

### Example 13-1: Biopanning

A procedure in which immobilized antigens were treated with a phage library surface-expressing antibodies to thereby antibody candidates binding to the antigens is called biopanning, and the biopanning is composed of three steps, binding/washing/elution. The phages having antibodies with weak binding affinity were removed during a washing step, and resultantly, only phages expressing antibodies with strong binding affinity remained. This procedure can be repeated three to four times to discover antibody candidates with excellent antigen binding affinity and specificity. Therefore, biopanning was used to screen acetylcholine receptor-binding peptides with excellent binding affinity and specificity to acetylcholine receptors.

In eight wells of a 96-well plate, 10 *µ*g/ml AchR α1 was placed at 50 µl per well, and then left at 4°C overnight. The next day, the wells were washed once with 200 µl of Tris (20 mM, pH 7), followed by the addition of 200 µl of 2% bovine serum albumin (BSA), and then blocked at room temperature for 2 hours. Then, the solution was all discarded, and the wells were washed three times with 200 µl of Tris (20 mM, pH 7). After 400 µl of the random peptide recombinant phages (input phages) suspended in PBS in Example 12-2 was mixed with 400 µl of 2% BSA, the mixture was placed at 100 µl per well and then left at 30°C for 1 hour. The solution in the wells was all removed, and the wells were washed three times with Tris (20 mM, pH 7). Thereafter, 0.2 M glycine (pH 2.2) was placed at 100 µl per well, and the phages were isolated for 20 minutes, and then, the phages were collected in one e-tube, and neutralized by the addition of 200µl of 1 M Tris (pH 9.0) (output phages).

To repeat biopanning, 500 µl of the isolated phages were mixed with 5 ml of *E. coli,* followed by culture at 37°C and 200 rpm for 30 minutes, and then 1×10¹⁰ pfu helper phages and ampicillin were added to a final concentration of 50 µg/ml, followed by further culture for 30 minutes. The culture was transferred to an SB liquid medium containing 50 *µ*g/ml ampicillin and 10 *µ*g/ml kanamycin, and cultured in the same conditions overnight, thereby producing random peptide recombinant phages. The reproduced random peptide recombinant phages were centrifuged at 4°C and 5,000 rpm for 10 minutes to obtain supernatant, and then, the supernatant and PEG/NaCl were mixed at 5:1 (v:v), left on ice for 1 hour, and centrifuged at 4°C and 13,000 rpm for 20 minutes to remove supernatant. The precipitates were suspended in 1 ml of phosphate buffered saline (PBS), and used for a second round of biopanning.

Random recombinant phages were reproduced by the same method as above for each round of biopanning, which was performed by the same method as above. In the biopanning steps, the number of times of washing with Tris (20 mM, pH 7) was 3 or 6 times, and biopanning was performed 5 times on AchR.

To measure the number of input phages and output phages for each biopanning, the phages were mixed with *E. coli* having an absorbance at 600 nm of 0.7 (OD600 = 0.7), and plated on agar plates containing ampicillin. The results are shown in Tables 6 to 9 below.

**TABLE 6**

| 8-mer Biopanning | | | | |
|---|---|---|---|---|
| Round | Number of times of washing | Input phages | Output phages | Input/Output |
| 1st | 3 times | 10.4 × 10¹¹ | 264 ×10⁶ | 25.4×10⁻⁵ |
| 2nd | 3 times | 2.752 ×10¹¹ | 82 ×10⁶ | 29.8×10⁻⁵ |
| 3rd | 6 times | 1.024 ×10¹¹ | 42.8 ×10⁶ | 41.8×10⁻⁵ |
| 4th | 6 times | 15.8 ×10¹¹ | 144 ×10⁶ | 9.11×10⁻⁵ |
| 5th | 6 times | 12.96 ×10¹¹ | 44.8 ×10⁶ | 3.46×10⁻⁵ |

**TABLE 7**

| 11-mer Biopanning | | | | |
|---|---|---|---|---|
| Round | Number of times of washing | Input phages | Output phages | Input/Output |
| 1st | 3 times | 1.14×10¹² | 1.048×10⁸ | 8.77×10⁻⁵ |
| 2nd | 6 times | 9.28×10¹¹ | 3.4×10⁶ | 0.366×10⁻⁵ |
| 3rd | 6 times | 9.4×10¹¹ | 6.96×10⁷ | 7.4×10⁻⁵ |
| 4th | 6 times | 1.02×10¹¹ | 2.32×10⁷ | 22.74×10⁻⁵ |

**TABLE 8**

| 14-mer Biopanning | | | | |
|---|---|---|---|---|
| Round | Number of times of washing | Input phages | Output phages | Input/Output |
| 1st | 3 times | 1.52 × 10¹¹ | 112 ×10⁶ | 73.68 ×10⁻⁵ |
| 2nd | 3 times | 2.112 ×10¹¹ | 86 ×10⁶ | 40.72 ×10⁻⁵ |
| 3rd | 6 times | 1.0 ×10¹¹ | 32 ×10⁶ | 32 ×10⁻⁵ |
| 4th | 6 times | 12.7 ×10¹¹ | 192 ×10⁶ | 15.12 ×10⁻⁵ |
| 5th | 6 times | 17.54 ×10¹¹ | 29×10⁶ | 1.65×10⁻⁵ |

**TABLE 9**

| 18-mer Biopanning | | | | |
|---|---|---|---|---|
| Round | Number of times of washing | Input phages | Output phages | Input/Output |
| 1st | 3 times | 1.28 × 10¹² | 5.36 ×10⁸ | 41.87×10⁻⁵ |
| 2nd | 3 times | 1.6 × 10¹² | 6.4 ×10⁸ | 40×10⁻⁵ |
| 3rd | 6 times | 1.58 x 10¹² | 3.92 ×10⁷ | 2.48×10⁻⁵ |
| 4th | 6 times | 1.49 × 10¹¹ | 9.9 ×10⁶ | 6.6×10⁻⁵ |
| 5th | 6 times | 1.18 × 10¹² | 1.16 ×10⁸ | 9.83×10⁻⁵ |

### Example 13-2: Enzyme-linked immunosorbent assay (ELISA) using random peptide library input recombinant phages

ELISA was performed on streptavidin and AchR using the input phages for each round of biopanning in Example 13-1.

In a 96-well ELISA plate, 10 µg/ml AchR or streptavidin was added at 50 µl per well, and left at 4°C overnight. The next day, the wells were washed three times with Tris (20 mM, pH7), followed by the addition of 2% BSA diluted with PBS, and then blocked at room temperature for 2 hours. After the solution was discarded, the wells were washed three times with Tris (20mM, pH7). After 800 µl of the input pages for each of 1st, 2nd, 3rd, 4th, and 5^{th} rounds in Tables 6 to 9 in Example 13-1 were mixed with 200 µl of 10% BSA, the mixture was placed at 100 µl per well and then left at 30°C for 1 hour. The solution in the wells was all removed, and the wells were washed three times with Tris (20 mM, pH 7). Then, horseradish peroxidase (HRP)-conjugated anti-M13 Ab (GE Healthcare) diluted 1:1,000 was added at 100 µl per well, followed by culture at 30°C for 1 hour. After the wells were washed three times with Tris (20 mM, pH 7), 100 µl of a tetramethylbenzidine (TMB) solution, which is a substrate of HRP, was added to each well to induce a color development reaction, and then the reaction was stopped by the addition of 100 µl of 1M HCl, and the absorbance (OD450) was measured at 450 nm. The results are shown in FIG. 13.

As shown in FIG. 13, the 8-mer, 11-mer, and 14-mer random peptide libraries obtained from the input phages of biopanning generally showed very high binding specificity to AchR compared to streptavidin. That is, the library of polypeptides, set forth in the general formula: (K or R)-X-(K or R)-(K or R)-XXX- (K or R), in which the 1st, 3rd, 4th, and 8th amino acids are fixed to be lysine (K) or arginine (R) and the 2nd, 5th, 6th, and 7th amino acids each are an arbitrary amino acid, generally bound to AchR with high specificity, regardless of the types of the 2nd, 5th, 6th, and 7th amino acids (X).

Also, as for 11-Mer, the library of polypeptides, set forth in the general formula: XXX-(K or R)-X-(K or R)-(K or R)-XXX-(K or R) in which the 4th, 6th, 7th, and 11th amino acids are lysine (K) or arginine (R), generally bound to AchR with high specificity, regardless of the type of arbitrary amino acid (1st, 2nd, 3rd, 5th, 8th, 9th and 10th amino acids) expressed by X.

Also, as for 14-Mer, the library of polypeptides, set forth in the general formula: XXX-(K or R)-X-(K or R)-(K or R)-XXX-(K or R)-XXX in which the 4th, 6th, 7th, and 11th amino acids are lysine (K) or arginine (R), generally bound to AchR with high specificity, regardless of the type of arbitrary amino acids (1st, 2nd, 3rd, 5th, 8th, 9th, 10th, 12th, 13th and 14th amino acids) expressed by X.

It was verified through the above results that the acetylcholine receptor-binding peptides according to the present invention containing amino acid sequences set forth in [Formula 1], [Formula 2], [Formula 3], or [Formula 3] bound to AchR with high specificity:

[Formula 1] X_{L}-(K or R)-X-(K or R)-X_{M}-(K or R)-X_{N}

(X_{L}, X_{M}, and X_{N} each independently represent a sequence composed of one to eight arbitrary amino acids and X represents a sequence composed of one arbitrary amino acid);

[Formula 2] (K or R)-X-(K or R)-(K or R)-X_{M-1}-(K or R)

(X_{M-1} represents a sequence composed of one to three arbitrary amino acids and X represents a sequence composed of one arbitrary amino acid);

[Formula 3] X_{L}-(K or R)-X-(K or R)-(K or R)-X_{M-1}-(K or R)

(X_{L} and X_{M-1} each independently represent a sequence composed of one to three arbitrary amino acids and X represents a sequence composed of one arbitrary amino acid); and

[Formula 4] X_{L}-(K or R)-X-(K or R)-(K or R)-X_{M-1}-(K or R)-X_{N}

(X_{L}, X_{M-1} and X_{N} each independently represent a sequence composed of one to three arbitrary amino acids and X represents a sequence composed of one arbitrary amino acid).

The amino acid sequences were set forth in predetermined formulas on the basis of the crucial sites and sequences of the peptides, and library of the peptides was constructed. The constructed library was verified to bind to acetylcholine receptors to inhibit the actions of the acetylcholine receptors.

As shown in FIG. 13, the 8-mer, 11-mer, and 14-mer random peptide libraries obtained from the input phages of the first round of biopanning showed higher binding specificity to AchR compared with streptavidin, whereas the 18-mer random peptide library obtained from the input phages of the first round of biopanning had low binding affinity to AchR.

It was verified through the above results that the 8-mer to 14-mer random peptides maintained the binding affinity to AchR, but the 18-mer random peptides had reduced binding affinity to AchR. This difference in binding affinity to AchR is considered to be made by the number of random amino acids (X).

### Example 13-3: Screening optimized peptides with enhanced binding affinity to acetylcholine receptors

Screening was conducted to select optimized peptides with enhanced AchR binding affinity from the input phages for the 4th and 5th rounds of biopanning, which were identified to have high binding affinity and specificity to AchR in Examples 13-1 and 13-2.

The output phages for the 4th and 5th rounds of biopanning in Example 13-1 were inoculated to *E. coli,* and plated on the agar plates so as to form 100-200 plaques per plate. The plaques were inoculated on 1 ml of an SB liquid medium containing 50 µg/ml of ampicillin by using a sterile tip, and cultured with shaking at 37°C for 5 hours, followed by the addition of 30 µl of helper phages, and then cultured at 37°C and 200 rpm for one day. The culture was centrifuged at 12,000 rpm for 2 minutes to collect supernatant, and 2% BSA was added to the supernatant, and used for phage screening.

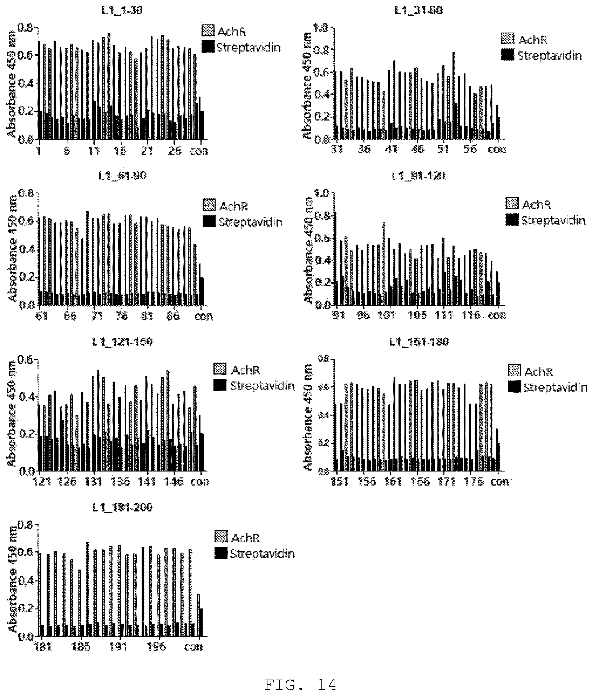

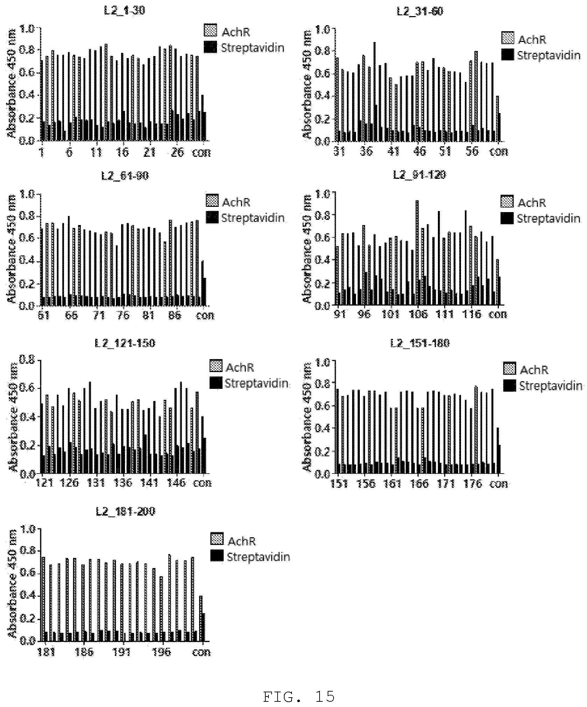

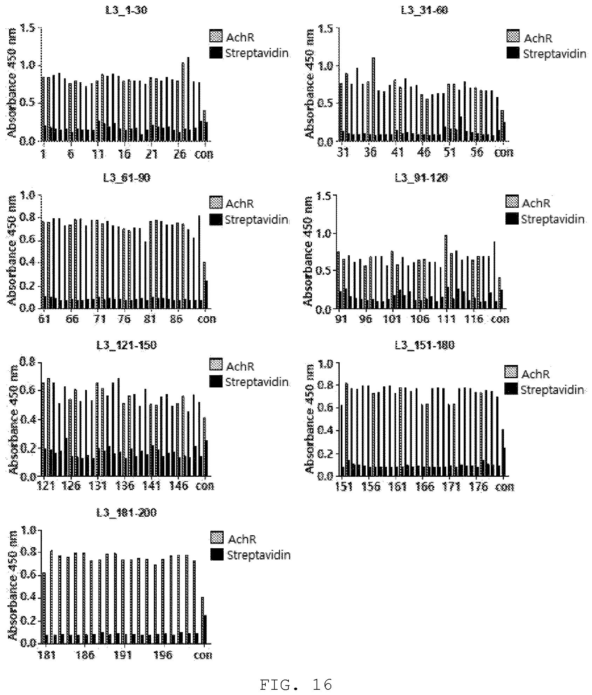

In a 96-well ELISA plate, 5 µg/ml AchR or streptavidin was added at 50 µl per well, and left at 4°C overnight. The next day, the solution in each well was discarded, followed by the addition of 2% BSA, and then blocked at room temperature for 2 hours. After the solution was all discarded, the wells were washed three times with Tris (20mM, pH7). In addition, 100 µl of each of the phage solutions obtained from the above-prepared plaques was dispensed into wells and left at 30°C for 1 hour. After the solution in the wells was all discarded, the wells were washed three times with Tris (20 mM, pH 7), and then the HRP-conjugated anti-M13 antibody diluted 1:2,000 was added at 100 µl per well, followed by culture at 30°C for 1 hour. After the wells were washed three times with Tris (20 mM, pH7), 100 µl of tetramethylbenzidine was added to each well to induce a color development reaction, and then the reaction was stopped by the addition of 100 µl of 1M H₂SO₄, and the absorbance at 450 nm (OD450) was measured. The measured absorbance was converted into a ratio of AchR absorbance to streptavidin absorbance (AchR signal/streptavidin signal), and the results are shown in FIGS. 14 to 16.

As shown in FIGS. 14 to 16, the binding affinity to AchR was observed to be different according to the phage, and among these, phages having the highest AchR signal/streptavidin signal were selected. The results are shown in Tables 10 to 12. The 8-mer (L1), 11-mer (L2), and 14-mer (L3) optimized peptides all showed higher binding affinity and specificity to AchR than Spep-2, a positive control.

Plasmid DNA was purified from the phages selected as above, and peptide sequencing was requested using the purified plasmid DNA and a primer for nucleotide sequencing, composed of GATTACGCCAAGCTTTGGAGC, and the optimized peptides having identified sequences and enhanced binding affinity to AchR were selected.

To compare with the peptides of the amino acid sequences disclosed in Korean Patent Publication No. 10-2014-0139010, the peptides of the amino acid sequences shown in Table 13 were synthesized, and investigated for the binding specificity to AchR by the same method as above. As a result, the synthesized peptides were verified to have the binding specificity similar to that of Spep-2. However, the sequences with R repeated, or the sequences with G repeated at both termini of the 8-mer showed a low level of binding specificity compared with all of the 11-mer or 14-mer library. This is thought to result from the charges or conformational characteristics of the peptides.

**TABLE 10**

| SEQ ID NO | Peptide (8-mer) | OD | Seq. |
|---|---|---|---|
| 1 | L1-1 | 0.693 | **K**A**KK**IRQ**R** |
| 2 | L1-2 | 0.68 | **K**K**RK**GSA**K** |
| 3 | L1-3 | 0.649 | **R**G**KR**QLG**R** |
| 4 | L1-4 | 0.696 | **K**L**KK**FPV**R** |
| 5 | L1-5 | 0.657 | **R**H**KK**SPW**K** |
| 6 | L1-6 | 0.652 | **R**P**RR**THI**K** |
| 7 | L1-7 | 0.68 | **K**H**KK**LNQ**R** |
| 8 | L1-8 | 0.653 | **R**A**KR**MCC**K** |
| 9 | L1-9 | 0.639 | **R**Q**RR**NDM**K** |
| 10 | L1-10 | 0.621 | **K**R**KK**DWW**R** |
| 11 | L1-11 | 0.703 | **R**A**RK**DAF**K** |
| 12 | L1-12 | 0.691 | **K**W**RR**EGF**K** |
| 13 | L1-13 | 0.73 | **R**F**RR**GVR**R** |
| 14 | L1-14 | 0.755 | **K**A**RR**QED**K** |
| 15 | L1-15 | 0.67 | **R**F**KR**LCM**K** |
| 16 | L1-16 | 0.62 | **K**L**KR**RSR**R** |
| 17 | L1-17 | 0.653 | **R**P**RR**SLD**R** |
| 18 | L1-18 | 0.627 | **K**H**KK**QNV**R** |
| 19 | L1-19 | 0.575 | **K**F**KR**TEG**R** |
| 20 | L1-20 | 0.619 | **R**G**KK**AVR**K** |
| 21 | L1-21 | 0.65 | **K**F**KK**HGV**K** |
| 22 | L1-22 | 0.732 | **K**T**KK**IHS**K** |
| 23 | L1-23 | 0.712 | **R**D**RK**IHD**K** |
| 24 | L1-24 | 0.742 | **R**P**RR**GHQ**K** |
| 25 | L1-25 | 0.709 | **K**T**KK**LYE**K** |
| 26 | L1-26 | 0.647 | **R**Q**KK**DNQ**K** |
| 27 | L1-27 | 0.662 | **R**Q**RK**SMQ**R** |
| 28 | L1-28 | 0.656 | **R**G**RR**WFV**K** |
| 29 | L1-29 | 0.646 | **R**N**KR**DEN**K** |
| 30 | L1-30 | 0.605 | **R**L**RR**WCV**K** |
| 31 | L1-31 | 0.604 | **R**Q**KK**PWV**K** |
| 32 | L1-32 | 0.607 | **R**W**KR**SEQ**R** |
| 33 | L1-33 | 0.529 | **R**L**KR**APG**R** |
| 34 | L1-34 | 0.634 | **K**M**RR**AQG**R** |
| 35 | L1-35 | 0.556 | **K**I**KK**AAR**R** |
| 36 | L1-36 | 0.553 | **K**N**RK**NLY**R** |
| 37 | L1-37 | 0.524 | **K**C**KK**MTE**R** |
| 38 | L1-38 | 0.514 | **K**T**RR**VDL**R** |
| 39 | L1-39 | 0.509 | **K**S**RK**WGW**R** |
| 40 | L1-40 | 0.424 | **K**R**RR**FRW**K** |
| 41 | L1-41 | 0.613 | **K**Y**KK**QHT**K** |
| 42 | L1-42 | 0.695 | **R**G**RK**KCG**K** |
| 43 | L1-43 | 0.601 | **K**L**RK**QKL**R** |
| 44 | L1-44 | 0.59 | **R**N**RK**IPL**K** |
| 45 | L1-45 | 0.597 | **R**L**RR**RYR**R** |
| 46 | L1-46 | 0.637 | **K**W**RK**RRI**K** |
| 47 | L1-47 | 0.54 | **K**T**KR**MLA**K** |
| 48 | L1-48 | 0.519 | **K**E**KR**AVH**K** |
| 49 | L1-49 | 0.504 | **R**V**RR**DGH**R** |
| 50 | L1-50 | 0.583 | **R**A**KK**CYH**K** |
| 51 | L1-51 | 0.66 | **K**G**KR**DSN**K** |
| 52 | L1-52 | 0.56 | **K**V**RK**SLW**K** |
| 53 | L1-53 | 0.78 | **R**S**KK**SLY**R** |
| 54 | L1-54 | 0.569 | **R**G**KR**GCE**R** |
| 55 | L1-55 | 0.585 | **K**Q**KK**TGF**R** |
| 56 | L1-56 | 0.468 | **K**Y**RR**NLV**R** |
| 57 | L1-57 | 0.408 | **R**I**RR**GSV**R** |
| 58 | L1-58 | 0.472 | **R**S**KR**MNG**K** |
| 59 | L1-59 | 0.479 | **R**H**KK**FIE**K** |
| 60 | L1-60 | 0.484 | **R**I**RR**VSQ**R** |
| 61 | L1-61 | 0.625 | **R**P**KK**WMQ**K** |
| 62 | L1-62 | 0.633 | **K**E**RK**HEA**K** |
| 63 | L1-63 | 0.618 | **K**E**RK**ASA**K** |
| 64 | L1-64 | 0.589 | **K**M**RR**IMY**K** |
| 65 | L1-65 | 0.585 | **K**W**KR**NCT**K** |
| 66 | L1-66 | 0.605 | **R**C**KK**NPE**K** |
| 67 | L1-67 | 0.593 | **K**Q**RK**PHG**R** |
| 68 | L1-68 | 0.55 | **K**A**KR**GHF**K** |
| 69 | L1-69 | 0.474 | **K**S**KR**HHE**K** |
| 70 | L1-70 | 0.668 | **K**S**RK**HCN**R** |
| 71 | L1-71 | 0.618 | **R**S**KK**INN**K** |
| 72 | L1-72 | 0.616 | **R**V**RR**SWI**R** |
| 73 | L1-73 | 0.644 | **K**S**RR**SAG**K** |
| 74 | L1-74 | 0.649 | **K**M**RK**HDV**R** |
| 75 | L1-75 | 0.581 | **K**G**KK**LSQ**R** |
| 76 | L1-76 | 0.588 | **K**W**RR**EQF**R** |
| 77 | L1-77 | 0.636 | **K**D**KR**WAN**R** |
| 78 | L1-78 | 0.642 | **K**H**KR**TAQ**R** |
| 79 | L1-79 | 0.583 | **K**P**KR**EMH**K** |
| 80 | L1-80 | 0.631 | **R**W**KK**NIN**K** |
| 81 | L1-81 | 0.63 | **K**W**KK**MME**R** |
| 82 | L1-82 | 0.596 | **R**I**RR**WWV**R** |
| 83 | L1-83 | 0.621 | **R**I**RR**HMY**R** |
| 84 | L1-84 | 0.576 | **K**P**KK**PPA**R** |
| 85 | L1-85 | 0.57 | **R**L**KR**GQF**K** |
| 86 | L1-86 | 0.551 | **K**N**KR**TPV**K** |
| 87 | L1-87 | 0.538 | **K**W**RR**AFG**R** |
| 88 | L1-88 | 0.562 | **K**N**KK**CVG**R** |
| 89 | L1-89 | 0.551 | **R**T**RK**CVL**K** |
| 90 | L1-90 | 0.439 | **K**I**KR**DLY**K** |
| 91 | L1-91 | 0.826 | **R**E**KR**YMA**R** |
| 92 | L1-92 | 0.581 | **R**V**KK**AET**R** |
| 93 | L1-93 | 0.616 | **R**G**RR**GTM**R** |
| 94 | L1-94 | 0.496 | **R**T**KR**WQL**K** |
| 95 | L1-95 | 0.535 | **R**L**RK**SLG**K** |
| 96 | L1-96 | 0.494 | **R**P**KR**NWA**K** |
| 97 | L1-97 | 0.546 | **K**D**RK**VNS**R** |
| 98 | L1-98 | 0.54 | **K**W**RR**NGQ**R** |
| 99 | L1-99 | 0.543 | **R**F**KR**MVW**R** |
| 100 | L1-100 | 0.737 | **K**E**RK**GAV**R** |
| 101 | L1-101 | 0.599 | **K**T**KR**GSL**K** |
| 102 | L1-102 | 0.507 | **K**T**KR**VDP**K** |
| 103 | L1-103 | 0.55 | **R**A**RK**LHN**R** |
| 104 | L1-104 | 0.46 | **R**G**KK**PWW**R** |
| 105 | L1-105 | 0.507 | **K**G**KK**YYQ**K** |
| 106 | L1-106 | 0.417 | **R**W**RR**SVI**K** |
| 107 | L1-107 | 0.534 | **R**Q**KK**DFL**K** |
| 108 | L1-108 | 0.535 | **R**G**RK**PIW**K** |
| 109 | L1-109 | 0.542 | **K**P**RR**PDT**R** |
| 110 | L1-110 | 0.422 | **K**V**KR**LWN**R** |
| 111 | L1-111 | 0.608 | **K**D**RK**GVY**K** |
| 112 | L1-112 | 0.431 | **R**V**KR**FVP**K** |
| 113 | L1-113 | 0.526 | **K**P**KR**DQS**R** |
| 114 | L1-114 | 0.42 | **K**D**KK**QWG**R** |
| 115 | L1-115 | 0.451 | **R**F**RK**MIP**K** |
| 116 | L1-116 | 0.492 | **K**F**RK**HVC**K** |
| 117 | L1-117 | 0.508 | **R**P**KR**SPS**K** |
| 118 | L1-118 | 0.471 | **R**Y**KK**VPA**K** |
| 119 | L1-119 | 0.463 | **R**S**RR**CPV**K** |
| 120 | L1-120 | 0.388 | **R**C**KR**CDN**K** |
| 121 | L1-121 | 0.354 | **R**G**KK**QLS**K** |
| 122 | L1-122 | 0.352 | **K**S**KR**PEG**R** |
| 123 | L1-123 | 0.409 | **K**C**RK**TMG**R** |
| 124 | L1-124 | 0.428 | **R**D**RK**NPV**R** |
| 125 | L1-125 | 0.345 | **K**P**KR**SAP**R** |
| 126 | L1-126 | 0.362 | **K**G**KR**TGC**K** |
| 127 | L1-127 | 0.412 | **R**F**RK**PTD**R** |
| 128 | L1-128 | 0.301 | **R**M**KK**FHT**R** |
| 129 | L1-129 | 0.421 | **K**I**KK**YYW**K** |
| 130 | L1-130 | 0.368 | **R**I**KR**NNC**K** |
| 131 | L1-131 | 0.505 | **R**H**KR**MLW**R** |
| 132 | L1-132 | 0.541 | **K**V**RR**VFL**K** |
| 133 | L1-133 | 0.504 | **K**D**KR**PEC**K** |
| 134 | L1-134 | 0.365 | **K**T**RR**SAC**R** |
| 135 | L1-135 | 0.479 | **K**F**RK**GPY**R** |
| 136 | L1-136 | 0.392 | **K**F**KK**SAP**R** |
| 137 | L1-137 | 0.458 | **K**P**RK**IQG**R** |
| 138 | L1-138 | 0.373 | **K**M**RK**GWD**K** |
| 139 | L1-139 | 0.456 | **K**F**RR**QST**R** |
| 140 | L1-140 | 0.381 | **K**N**RR**ADC**R** |
| 141 | L1-141 | 0.505 | **K**N**KR**WVF**K** |
| 142 | L1-142 | 0.469 | **R**C**RK**DTA**R** |
| 143 | L1-143 | 0.416 | **K**A**RR**VGT**K** |
| 144 | L1-144 | 0.503 | **K**V**KK**IYY**R** |
| 145 | L1-145 | 0.54 | **K**A**KR**DYL**R** |
| 146 | L1-146 | 0.362 | **K**V**KR**LPY**R** |
| 147 | L1-147 | 0.415 | **R**H**RR**ANF**R** |
| 148 | L1-148 | 0.426 | **K**P**KK**GGW**K** |
| 149 | L1-149 | 0.34 | **K**E**KR**LYA**R** |
| 150 | L1-150 | 0.457 | **R**E**KK**QEV**K** |
| 151 | L1-151 | 0.479 | **K**S**RR**AVF**R** |
| 152 | L1-152 | 0.484 | **R**H**RK**YVD**K** |
| 153 | L1-153 | 0.625 | **R**H**KR**WSG**R** |
| 154 | L1-154 | 0.633 | **R**D**RK**FVQ**K** |
| 155 | L1-155 | 0.618 | **R**Y**RR**WFY**R** |
| 156 | L1-156 | 0.589 | **R**Q**RR**MTP**R** |
| 157 | L1-157 | 0.585 | **R**N**KR**MDG**K** |
| 158 | L1-158 | 0.605 | **R**P**KK**HQE**R** |
| 159 | L1-159 | 0.593 | **K**K**RK**SLL**R** |
| 160 | L1-160 | 0.55 | **K**I**KR**NVF**K** |
| 161 | L1-161 | 0.474 | **K**Y**KR**EEY**R** |
| 162 | L1-162 | 0.668 | **K**I**RR**VTN**K** |
| 163 | L1-163 | 0.618 | **K**V**KR**VWG**R** |
| 164 | L1-164 | 0.616 | **R**I**KR**DYC**K** |
| 165 | L1-165 | 0.644 | **R**I**RR**AHD**R** |
| 166 | L1-166 | 0.649 | **R**A**RK**ESH**K** |
| 167 | L1-167 | 0.581 | **R**Y**KK**QQY**K** |
| 168 | L1-168 | 0.588 | **K**I**KK**LSQ**K** |
| 169 | L1-169 | 0.636 | **K**L**KR**AML**K** |
| 170 | L1-170 | 0.642 | **K**A**RK**NSI**R** |
| 171 | L1-171 | 0.583 | **K**E**RK**LWW**K** |
| 172 | L1-172 | 0.631 | **K**T**RK**QDH**R** |
| 173 | L1-173 | 0.63 | **R**C**KR**FVG**K** |
| 174 | L1-174 | 0.596 | **K**E**KK**LVW**K** |
| 175 | L1-175 | 0.621 | **K**A**RR**NSL**K** |
| 176 | Ll-17 6 | 0.479 | **K**M**RR**VAP**R** |
| 177 | L1-177 | 0.484 | **R**A**KK**IMF**R** |
| 178 | L1-178 | 0.625 | **K**T**KK**AAE**R** |
| 179 | L1-179 | 0.633 | **R**G**KR**HWH**R** |
| 180 | L1-180 | 0.618 | **R**T**KK**ENV**K** |
| 181 | L1-181 | 0.589 | **R**E**KK**YAY**K** |
| 182 | L1-182 | 0.585 | **K**W**RR**ELP**R** |
| 183 | L1-183 | 0.605 | **K**S**RR**MWG**R** |
| 184 | L1-184 | 0.593 | **K**C**KK**DND**K** |
| 185 | L1-185 | 0.55 | **K**D**KK**MPQ**R** |
| 186 | L1-186 | 0.474 | **R**H**KR**QQD**K** |
| 187 | L1-187 | 0.668 | **R**L**KK**HCG**K** |
| 188 | L1-188 | 0.618 | **K**A**RK**QEV**R** |
| 189 | L1-189 | 0.616 | **K**M**RK**FYS**K** |
| 190 | L1-190 | 0.644 | **K**M**RR**WWL**K** |
| 191 | L1-191 | 0.649 | **K**Q**KR**QWA**R** |
| 192 | L1-192 | 0.581 | **R**S**RR**GIG**K** |
| 193 | L1-193 | 0.588 | **K**D**KK**TPC**K** |
| 194 | L1-194 | 0.636 | **R**I**RK**ITW**R** |
| 195 | L1-195 | 0.642 | **R**H**KR**WEV**R** |
| 196 | L1-196 | 0.583 | **R**F**RR**NFH**K** |
| 197 | L1-197 | 0.631 | **K**W**KR**FSQ**R** |
| 198 | L1-198 | 0.63 | **K**Y**KK**SFT**K** |
| 199 | L1-199 | 0.596 | **K**T**RK**IVM**K** |
| 200 | L1-200 | 0.621 | **R**T**KK**AYV**K** |
| | **Spep-2 (con)** | **0.303** | **W**T**WKGRKSLLR** |

**Table 11**

| SEQ ID NO | Peptide (11-mer) | OD | Seq. |
|---|---|---|---|
| 201 | L2-1 | 0.705 | EDY**K**Y**RR**QNY**K** |
| 202 | L2-2 | 0.749 | GIW**K**F**RR**NQC**K** |
| 203 | L2-3 | 0.796 | SPV**R**W**KR**LCL**R** |
| 204 | L2-4 | 0.757 | ECY**R**N**RK**AYC**R** |
| 205 | L2-5 | 0.752 | TQQ**R**L**KR**VME**K** |
| 206 | L2-6 | 0.78 | PEL**K**C**KR**MIG**R** |
| 207 | L2-7 | 0.753 | WQM**K**T**KK**EIW**K** |
| 208 | L2-8 | 0.739 | FGH**R**G**KK**EWA**R** |
| 209 | L2-9 | 0.721 | ATC**K**P**KR**PWY**K** |
| 210 | L2-10 | 0.803 | TSQ**K**V**RK**MEA**K** |
| 211 | L2-11 | 0.791 | GIP**K**Y**RR**GCS**R** |
| 212 | L2-12 | 0.83 | AYA**K**A**RR**WGQ**K** |
| 213 | L2-13 | 0.855 | GGL**K**S**KR**LTT**R** |
| 214 | L2-14 | 0.746 | GMW**K**V**RK**TVF**R** |
| 215 | L2-15 | 0.705 | HTT**R**G**KR**CDP**R** |
| 216 | L2-16 | 0.77 | VDV**R**N**KK**SNN**R** |
| 217 | L2-17 | 0.72 | GLN**R**W**RR**CHH**K** |
| 218 | L2-18 | 0.753 | LQS**R**S**KK**YSV**K** |
| 219 | L2-19 | 0.727 | GWN**R**A**KR**EES**K** |
| 220 | L2-20 | 0.675 | SWQ**K**C**KK**AEV**R** |
| 221 | L2-21 | 0.719 | MVM**K**W**KR**WNQ**R** |
| 222 | L2-22 | 0.75 | MMM**R**H**KR**CQY**R** |
| 223 | L2-23 | 0.832 | QTY**R**L**KK**PLQ**K** |
| 224 | L2-24 | 0.812 | SFW**R**E**RK**NGF**R** |
| 225 | L2-25 | 0.842 | RLE**R**P**RR**ELT**K** |
| 226 | L2-26 | 0.809 | APW**R**L**KR**APQ**R** |
| 227 | L2-27 | 0.747 | QGE**K**Y**RR**TES**K** |
| 228 | L2-28 | 0.762 | CIW**K**S**KR**SPA**K** |
| 229 | L2-29 | 0.756 | AIL**K**G**KR**IPN**K** |
| 230 | L2-30 | 0.746 | PTL**K**W**RK**PVL**R** |
| 231 | L2-31 | 0.737 | NNS**R**S**KR**FMN**R** |
| 232 | L2-32 | 0.64 | LYM**R**C**KK**QAP**R** |
| 233 | L2-33 | 0.619 | INY**R**C**RK**WVD**K** |
| 234 | L2-34 | 0.604 | VMD**R**D**RK**WWW**R** |
| 235 | L2-35 | 0.683 | NFT**K**L**RK**PGP**R** |
| 236 | L2-36 | 0.76 | FIP**K**M**RK**CSP**R** |
| 237 | L2-37 | 0.66 | TLW**K**V**RK**AYS**R** |
| 238 | L2-38 | 0.88 | ESE**K**I**KR**LST**R** |
| 239 | L2-39 | 0.669 | LMT**K**N**RR**NSF**R** |
| 240 | L2-40 | 0.685 | PEV**K**V**RR**HSM**R** |
| 241 | L2-41 | 0.568 | AEV**K**D**KK**AYY**K** |
| 242 | L2-42 | 0.508 | YYA**K**S**KK**YMV**R** |
| 243 | L2-43 | 0.572 | LMD**R**T**RR**DMY**K** |
| 244 | L2-44 | 0.579 | NMG**R**H**KR**PFL**K** |
| 245 | L2-45 | 0.584 | WIY**R**G**RK**DVA**K** |
| 246 | L2-46 | 0.704 | CYW**K**A**KR**YPM**R** |
| 247 | L2-47 | 0.707 | IED**K**G**RR**INP**R** |
| 248 | L2-48 | 0.629 | VST**K**I**KK**EPQ**R** |
| 249 | L2-49 | 0.734 | YPF**K**A**KR**PAE**K** |
| 250 | L2-50 | 0.656 | SAD**R**N**KR**HMT**R** |
| 251 | L2-51 | 0.653 | SLY**R**Q**KR**HDY**K** |
| 252 | L2-52 | 0.624 | LPS**R**P**KK**PVP**K** |
| 253 | L2-53 | 0.614 | PAW**R**C**KR**CQP**K** |
| 254 | L2-54 | 0.609 | HHW**R**F**KR**EMP**R** |
| 255 | L2-55 | 0.524 | PAC**R**S**KR**DWQ**K** |
| 256 | L2-56 | 0.713 | FIC**K**S**RK**FYG**K** |
| 257 | L2-57 | 0.795 | LEH**K**E**RK**DDF**R** |
| 258 | L2-58 | 0.701 | ANP**K**N**RK**DNL**R** |
| 259 | L2-59 | 0.69 | FGV**K**Y**RR**VIC**R** |
| 260 | L2-60 | 0.697 | HAD**K**F**RR**FNM**R** |
| 261 | L2-61 | 0.688 | PTI**R**V**RK**SDD**R** |
| 262 | L2-62 | 0.736 | NLH**K**P**KR**DLP**K** |
| 263 | L2-63 | 0.742 | IDG**K**W**RK**ICT**R** |
| 264 | L2-64 | 0.683 | SPF**R**A**KR**QDV**R** |
| 265 | L2-65 | 0.731 | PLW**K**T**RK**IEP**R** |
| 266 | L2-66 | 0.798 | PNY**K**W**RK**SRR**R** |
| 267 | L2-67 | 0.696 | KTG**R**S**KR**HRW**R** |
| 268 | L2-68 | 0.721 | GPD**K**S**RR**NLH**R** |
| 269 | L2-69 | 0.676 | WCC**K**T**KR**AVM**K** |
| 270 | L2-70 | 0.67 | QLL**K**M**RK**ALS**R** |
| 271 | L2-71 | 0.651 | PYL**R**S**KR**FPP**R** |
| 272 | L2-72 | 0.638 | APH**K**W**RK**QEQ**R** |
| 273 | L2-73 | 0.662 | PPF**K**F**RK**PLG**R** |
| 274 | L2-74 | 0.651 | CIM**R**V**KR**MWW**K** |
| 275 | L2-75 | 0.539 | YIL**R**D**KK**VPL**K** |
| 276 | L2-76 | 0.725 | PWV**R**I**RK**MAS**R** |
| 277 | L2-77 | 0.733 | VAD**R**Q**KK**TAP**K** |
| 278 | L2-78 | 0.718 | HHN**R**M**KK**QYH**R** |
| 279 | L2-79 | 0.689 | EGA**K**Y**RR**DGW**R** |
| 280 | L2-80 | 0.685 | FWD**R**V**KR**NPS**K** |
| 281 | L2-81 | 0.705 | TMW**R**Q**RK**MSC**K** |
| 282 | L2-82 | 0.693 | PDT**R**W**KR**VLF**K** |
| 283 | L2-83 | 0.65 | HQQ**K**C**KK**TTT**K** |
| 284 | L2-84 | 0.574 | PWH**K**G**KR**DFD**R** |
| 285 | L2-85 | 0.768 | VFV**K**W**RR**QMM**R** |
| 286 | L2-86 | 0.701 | SQW**K**I**RK**RLI**R** |
| 287 | L2-87 | 0.716 | EWH**K**V**RR**VYA**K** |
| 288 | L2-88 | 0.744 | NAM**R**T**KR**MSF**K** |
| 289 | L2-89 | 0.749 | PPF**K**F**RK**PLG**R** |
| 290 | L2-90 | 0.765 | TSV**K**K**RK**QRL**R** |
| 291 | L2-91 | 0.517 | PID**K**F**KR**GMV**R** |
| 292 | L2-92 | 0.634 | FVG**R**W**KR**EYA**K** |
| 293 | L2-93 | 0.635 | FNP**K**M**RK**VCL**R** |
| 294 | L2-94 | 0.642 | NPG**K**T**KR**WLQ**R** |
| 295 | L2-95 | 0.522 | IMD**K**R**RK**PGC**R** |
| 296 | L2-96 | 0.708 | ISD**K**A**KR**QHF**K** |
| 297 | L2-97 | 0.531 | DVN**K**Y**RK**HSH**K** |
| 298 | L2-98 | 0.626 | LML**R**G**KR**LTT**K** |
| 299 | L2-99 | 0.52 | CSL**R**A**RK**EEW**R** |
| 300 | L2-100 | 0.551 | MNY**R**C**KR**VQE**R** |
| 301 | L2-101 | 0.592 | NGV**R**E**RK**WQS**K** |
| 302 | L2-102 | 0.608 | YTS**R**C**KK**QPR**K** |
| 303 | L2-103 | 0.571 | QYN**K**G**RK**IHV**R** |
| 304 | L2-104 | 0.563 | PED**R**Q**RK**TWF**K** |
| 305 | L2-105 | 0.488 | IEM**K**P**RK**FGV**K** |
| 306 | L2-106 | 0.926 | QRW**K**W**RK**SLA**R** |
| 307 | L2-107 | 0.681 | VKH**K**E**RK**CQR**R** |
| 308 | L2-108 | 0.716 | QQD**R**P**KR**DIP**K** |
| 309 | L2-109 | 0.596 | YPT**K**G**KR**CMI**R** |
| 310 | L2-110 | 0.831 | RYA**K**H**RK**RQT**R** |
| 311 | L2-111 | 0.594 | GYF**R**P**RK**ETC**K** |
| 312 | L2-112 | 0.646 | CFF**K**M**RR**CNT**K** |
| 313 | L2-113 | 0.64 | QND**K**D**RK**LSH**R** |
| 314 | L2-114 | 0.643 | QVT**K**S**KR**VAF**K** |
| 315 | L2-115 | 0.837 | VRA**K**H**RK**SSL**R** |
| 316 | L2-116 | 0.699 | SHS**R**Q**RK**TPL**R** |
| 317 | L2-117 | 0.607 | DNG**K**I**RR**CLG**K** |
| 318 | L2-118 | 0.65 | FLR**R**L**KK**VHW**K** |
| 319 | L2-119 | 0.56 | FCR**R**S**KK**IGR**R** |
| 320 | L2-120 | 0.607 | PAA**R**T**KR**MYG**R** |
| 321 | L2-121 | 0.492 | HET**K**P**KK**DGL**R** |
| 322 | L2-122 | 0.558 | MGD**R**P**RK**WDS**R** |
| 323 | L2-123 | 0.473 | HDT**K**C**KK**MYA**K** |
| 324 | L2-124 | 0.556 | NVV**R**G**RR**LEC**R** |
| 325 | L2-125 | 0.481 | PAD**K**G**RR**EVM**K** |
| 326 | L2-126 | 0.605 | AMM**K**Y**KK**EFP**K** |
| 327 | L2-127 | 0.569 | YII**R**W**KR**QMT**R** |
| 328 | L2-128 | 0.516 | SPW**R**I**RR**QNI**R** |
| 329 | L2-129 | 0.603 | TCI**K**Y**RR**AHT**K** |
| 330 | L2-130 | 0.64 | GYD**R**A**RK**GTL**R** |
| 331 | L2-131 | 0.462 | MTL**K**H**RR**VYI**K** |
| 332 | L2-132 | 0.515 | LFT**R**I**KR**LVC**K** |
| 333 | L2-133 | 0.526 | DFS**R**D**RR**CLS**K** |
| 334 | L2-134 | 0.44 | VWN**K**V**KR**WLE**R** |
| 335 | L2-135 | 0.557 | FPG**K**N**RK**YCS**R** |
| 336 | L2-136 | 0.454 | YTS**K**N**KR**GCP**R** |
| 337 | L2-137 | 0.452 | CAC**K**Q**RR**ATS**R** |
| 338 | L2-138 | 0.509 | IME**R**Q**RK**SQH**R** |
| 339 | L2-139 | 0.528 | SVL**R**C**RK**CSM**K** |
| 340 | L2-140 | 0.445 | YPQ**K**L**RR**TAL**K** |
| 341 | L2-141 | 0.462 | SSH**K**G**KR**AQS**K** |
| 342 | L2-142 | 0.512 | DNP**R**F**RK**TIL**K** |
| 343 | L2-143 | 0.401 | AIIK**FRK**VQW**K** |
| 344 | L2-144 | 0.521 | PYT**R**C**RK**EIC**K** |
| 345 | L2-145 | 0.468 | MNE**K**P**KK**NDQ**K** |
| 346 | L2-146 | 0.605 | LIG**K**F**KK**PFY**R** |
| 347 | L2-147 | 0.641 | TWC**K**H**KK**LDM**K** |
| 348 | L2-148 | 0.604 | DSN**K**V**RK**CSS**K** |
| 349 | L2-149 | 0.465 | LPM**K**Q**RK**CEF**R** |
| 350 | L2-150 | 0.579 | EDV**R**V**KR**QTC**R** |
| 351 | L2-151 | 0.749 | TQC**K**D**RR**VSD**R** |
| 352 | L2-152 | 0.681 | IAL**K**P**KR**VWL**K** |
| 353 | L2-153 | 0.688 | GHQ**R**G**KR**EGS**R** |
| 354 | L2-154 | 0.736 | NPF**K**Y**KK**ICP**K** |
| 355 | L2-155 | 0.742 | NEA**R**I**KK**CDV**K** |
| 356 | L2-156 | 0.683 | YGL**R**M**RK**WYM**K** |
| 357 | L2-157 | 0.731 | NFY**K**C**RK**LQC**K** |
| 358 | L2-158 | 0.73 | MMT**K**Y**KK**TCC**K** |
| 359 | L2-159 | 0.696 | CNQ**K**T**KK**IAE**K** |
| 360 | L2-160 | 0.721 | ADI**R**M**KK**WYP**K** |
| 361 | L2-161 | 0.579 | AWF**R**V**KR**SNC**R** |
| 362 | L2-162 | 0.584 | NCD**R**T**RK**HWA**R** |
| 63 | L2-163 | 0.725 | PHA**R**T**RK**NIT**K** |
| 364 | L2-164 | 0.733 | VPT**K**M**KK**YET**K** |
| 365 | L2-165 | 0.718 | AYP**K**F**RK**TFN**R** |
| 366 | L2-166 | 0.579 | QQL**R**L**RK**LCG**K** |
| 367 | L2-167 | 0.584 | MFM**R**N**KK**LAW**R** |
| 368 | L2-168 | 0.725 | GGA**K**N**KK**WS**R** |
| 369 | L2-169 | 0.733 | HDP**R**H**KK**TPT**K** |
| 370 | L2-170 | 0.718 | QVH**R**N**KR**YTD**R** |
| 371 | L2-171 | 0.689 | YGT**R**P**KK**YVS**K** |
| 372 | L2-172 | 0.685 | CTW**R**G**RR**PHD**K** |
| 373 | L2-173 | 0.705 | SWA**K**A**RK**LVH**R** |
| 374 | L2-174 | 0.693 | PLF**K**S**RR**AYV**R** |
| 375 | L2-175 | 0.65 | CVM**R**C**RR**SED**K** |
| 376 | L2-176 | 0.574 | WWH**K**H**RR**AQS**K** |
| 377 | L2-177 | 0.768 | LNS**K**P**RR**VEF**K** |
| 378 | L2-178 | 0.718 | VPY**R**H**RR**MQF**K** |
| 379 | L2-179 | 0.716 | GIA**K**S**KR**NAG**R** |
| 380 | L2-180 | 0.744 | DPE**K**W**RK**FYD**R** |
| 381 | L2-181 | 0.749 | PGA**R**C**RK**QDV**K** |
| 382 | L2-182 | 0.681 | HEQ**R**P**KK**GQQ**K** |
| 383 | L2-183 | 0.688 | TCD**R**A**RK**ESF**R** |
| 384 | L2-184 | 0.736 | MHQ**R**E**RR**NFV**K** |
| 385 | L2-185 | 0.742 | FIT**R**F**RK**MGE**K** |
| 386 | L2-186 | 0.683 | PNW**K**V**RR**FGD**K** |
| 387 | L2-187 | 0.731 | TAA**K**W**KK**IIM**K** |
| 388 | L2-188 | 0.73 | CLW**R**L**RK**DNG**R** |
| 389 | L2-189 | 0.696 | AHI**K**S**RK**VWS**R** |
| 390 | L2-190 | 0.721 | NLV**K**S**KK**VEE**K** |
| 391 | L2-191 | 0.689 | PDS**R**L**KK**HEA**K** |
| 392 | L2-192 | 0.685 | EHV**K**L**KR**LDF**R** |
| 393 | L2-193 | 0.705 | PAL**R**M**RR**WCQ**K** |
| 394 | L2-194 | 0.693 | HLE**K**H**RR**CEF**K** |
| 395 | L2-195 | 0.65 | CQA**K**T**RK**AED**R** |
| 396 | L2-196 | 0.574 | QNM**R**M**KR**FIQ**R** |
| 397 | L2-197 | 0.768 | CPY**R**I**RR**GPG**K** |
| 398 | L2-198 | 0.718 | DIY**K**G**KR**TLV**K** |
| 399 | L2-199 | 0.716 | EIC**K**N**RK**PAN**R** |
| 400 | L2-200 | 0.744 | QGM**K**L**KR**IWS**K** |
| | **Spep-2 (con)** | **0.303** | **WTWKGRKSLLR** |

**TABLE 12**

| SEQ ID NO | Peptide (14-mer) | OD | Seq. |
|---|---|---|---|
| 401 | L3-1 | 0.853 | ALG**R**T**KR**LHM**R**VHV |
| 402 | L3-2 | 0.841 | SFY**K**E**RK**CQF**R**SGL |
| 403 | L3-3 | 0.88 | SGV**R**Y**RK**WWI**R**SW |
| 404 | L3-4 | 0.905 | MPQ**K**L**KK**LDI**R**NHN |
| 405 | L3-5 | 0.82 | NED**R**G**KR**PHI**R**VLG |
| 406 | L3-6 | 0.77 | EFV**K**L**RK**ARL**R**GPQ |
| 407 | L3-7 | 0.803 | EGD**K**F**RR**HDI**K**YNF |
| 408 | L3-8 | 0.777 | PIC**R**W**KR**APF**K**WYF |
| 409 | L3-9 | 0.725 | NCN**R**F**RR**TIV**R**HCH |
| 410 | L3-10 | 0.769 | AHG**K**D**RR**YVE**K**LEV |
| 411 | L3-11 | 0.8 | FMQ**K**C**KK**WWD**R**AVF |
| 412 | L3-12 | 0.882 | DPP**K**K**RK**SLL**R**RVS |
| 413 | L3-13 | 0.862 | YCT**R**I**RR**EGM**K**GSE |
| 414 | L3-14 | 0.892 | FPG**K**S**KK**QWH**R**LWP |
| 415 | L3-15 | 0.859 | YEP**R**F**KR**PYG**K**WCH |
| 416 | L3-16 | 0.797 | DYL**R**S**RK**MEE**R**FQE |
| 417 | L3-17 | 0.812 | VGP**K**F**RK**NHR**R**QNR |
| 418 | L3-18 | 0.806 | EYQ**K**C**KK**PSF**R**LTM |
| 419 | L3-19 | 0.796 | YMK**K**K**RK**SLL**R**TSL |
| 420 | L3-20 | 0.755 | CCF**K**L**KK**AYN**K**GPF |
| 421 | L3-21 | 0.843 | NPM**K**L**RK**AET**K**HNV |
| 422 | L3-22 | 0.83 | HSL**K**T**RK**SAF**K**SNT |
| 423 | L3-23 | 0.799 | QYH**K**V**RK**LWF**R**VEP |
| 424 | L3-24 | 0.846 | FVR**K**K**RK**SLL**R**DTR |
| 425 | L3-25 | 0.807 | CFQ**R**K**RK**SLL**R**VLR |
| 426 | L3-26 | 0.802 | VPM**K**Y**RR**CGN**R**QSN |
| 427 | L3-27 | 0.83 | RAR**K**K**RK**SLL**R**RQV |
| 428 | L3-28 | 0.803 | PNG**K**V**RK**RIR**R**RYF |
| 429 | L3-29 | 0.789 | CAS**K**P**RR**TYL**R**AAN |
| 430 | L3-30 | 0.771 | WAH**K**C**KK**PGQ**R**IPP |
| 431 | L3-31 | 0.763 | LSD**K**K**RK**SLL**R**YDY |
| 432 | L3-32 | 0.845 | CAT**R**G**KK**VFS**K**RTM |
| 433 | L3-33 | 0.751 | LRQ**R**S**KR**VLE**K**LRP |
| 434 | L3-34 | 0.74 | LGW**K**K**RK**SLL**R**RHV |
| 435 | L3-35 | 0.747 | LQC**K**Y**RR**GSD**K**QPQ |
| 436 | L3-36 | 0.787 | AFS**R**I**KR**GVL**K**LLS |
| 437 | L3-37 | 0.69 | SNV**K**R**KR**GRC**K**PYR |
| 438 | L3-38 | 0.669 | TVV**K**S**RK**CSV**R**YNW |
| 439 | L3-39 | 0.654 | DDV**K**Q**RK**KHP**R**VQT |
| 440 | L3-40 | 0.733 | ASP**K**G**RR**PTF**R**PQH |
| 441 | L3-41 | 0.81 | GWL**K**A**KR**FPS**R**PPT |
| 442 | L3-42 | 0.71 | ETN**R**T**RK**QCY**K**TTF |
| 443 | L3-43 | 0.93 | FTH**K**N**RR**DSL**R**VWM |
| 444 | L3-44 | 0.719 | PPF**R**L**RK**PLW**R**PQR |
| 445 | L3-45 | 0.735 | PGN**R**M**KK**YQN**R**VHG |
| 446 | L3-46 | 0.618 | SAH**K**K**RK**QTL**R**CSE |
| 447 | L3-47 | 0.558 | LYE**K**Y**KK**HNN**R**EDD |
| 448 | L3-48 | 0.622 | PHC**R**Q**KK**FWI**R**CGT |
| 449 | L3-49 | 0.629 | VAF**K**T**RR**RVQ**R**QSG |
| 450 | L3-50 | 0.634 | VFD**K**F**RK**TEN**R**GVI |
| 451 | L3-51 | 0.754 | NHH**K**T**KR**CSV**R**FNI |
| 452 | L3-52 | 0.757 | GTF**K**W**RK**SGA**R**QYL |
| 453 | L3-53 | 0.679 | HCL**R**T**KK**LIN**K**ICS |
| 454 | L3-54 | 0.784 | FFL**R**C**RR**LLG**K**VQV |
| 455 | L3-55 | 0.706 | HFP**R**A**RR**FEH**R**CML |
| 456 | L3-56 | 0.703 | QIS**K**L**KR**PSY**R**GDD |
| 457 | L3-57 | 0.674 | EEI**K**Q**KK**LHL**R**VWF |
| 458 | L3-58 | 0.664 | SLP**K**W**RK**GGD**R**VFT |
| 459 | L3-59 | 0.659 | HVY**K**N**RR**VWG**K**GWP |
| 460 | L3-60 | 0.574 | EDL**R**C**KK**LEL**R**SVI |
| 461 | L3-61 | 0.768 | THD**K**C**KK**HND**K**QAH |
| 462 | L3-62 | 0.766 | YPE**R**P**RK**LQD**K**SYS |
| 463 | L3-63 | 0.794 | CPW**R**N**RK**AMI**K**GII |
| 464 | L3-64 | 0.799 | HEI**K**Q**KK**YFH**R**GHD |
| 465 | L3-65 | 0.731 | CLE**K**L**RK**AVH**R**QRR |
| 466 | L3-66 | 0.738 | YIS**K**S**KK**TAG**R**WFW |
| 467 | L3-67 | 0.786 | VTW**K**F**RK**AEK**R**WGY |
| 468 | L3-68 | 0.792 | YSS**K**S**RK**LSP**R**TPR |
| 469 | L3-69 | 0.733 | CVN**R**V**KK**SDS**K**GTW |
| 470 | L3-70 | 0.781 | DHE**R**E**RR**LWS**R**FPF |
| 471 | L3-71 | 0.78 | PLI**K**V**KR**GVG**K**LWN |
| 472 | L3-72 | 0.746 | TGC**R**C**KR**SMY**K**NLH |
| 473 | L3-73 | 0.771 | WTC**R**M**RK**YQL**R**TSE |
| 474 | L3-74 | 0.726 | IAI**R**P**RK**MTL**K**IHP |
| 475 | L3-75 | 0.72 | QIP**K**Q**KK**QEQ**R**AIS |
| 476 | L3-76 | 0.701 | QMG**K**F**KK**ISL**K**NTF |
| 477 | L3-77 | 0.688 | VLI**K**Q**RK**WQD**R**SCS |
| 478 | L3-78 | 0.712 | FIT**K**S**RR**QQF**R**NQG |
| 479 | L3-79 | 0.701 | QQF**K**Y**RR**ECV**K**YGS |
| 480 | L3-80 | 0.589 | MEG**R**E**KK**SYN**K**GEN |
| 481 | L3-81 | 0.775 | PTW**R**H**RR**YCA**K**DIG |
| 482 | L3-82 | 0.783 | HMC**R**S**KR**VAW**K**NLI |
| 483 | L3-83 | 0.768 | SYL**K**C**KR**SDY**K**EVP |
| 484 | L3-84 | 0.739 | HTF**K**L**RK**LCQ**K**LFE |
| 485 | L3-85 | 0.735 | QLV**K**L**RK**LAR**R**VSY |
| 486 | L3-86 | 0.755 | SEV**R**P**KK**DHS**R**LFI |
| 487 | L3-87 | 0.743 | FLE**K**C**RK**FII**R**VST |
| 488 | L3-88 | 0.7 | PET**R**H**RK**MFI**R**DFW |
| 489 | L3-89 | 0.624 | EWC**K**N**KR**WHS**R**EYP |
| 490 | L3-90 | 0.818 | LWN**R**Y**KK**LLM**R**IFW |
| 491 | L3-91 | 0.749 | CWC**R**Q**RK**CFH**K**PWI |
| 492 | L3-92 | 0.657 | NLE**R**T**KK**HGL**K**GYM |
| 493 | L3-93 | 0.7 | EGG**R**I**KR**PNY**R**GDG |
| 494 | L3-94 | 0.61 | PVL**K**L**RK**GRV**R**AQP |
| 495 | L3-95 | 0.657 | SIS**R**L**RK**AHQ**K**FIP |
| 496 | L3-96 | 0.567 | EWH**K**H**RR**EMV**R**WGP |
| 497 | L3-97 | 0.684 | WSA**K**G**RR**MGC**K**STM |
| 498 | L3-98 | 0.685 | PNG**K**V**RK**RIR**R**RYF |
| 499 | L3-99 | 0.692 | PCW**K**F**RR**AQM**K**WGI |
| 500 | L3-100 | 0.572 | LEE**R**P**KK**HCA**K**NHC |
| 501 | L3-101 | 0.758 | QPW**R**Q**KK**FWC**R**CWG |
| 502 | L3-102 | 0.581 | IMV**K**I**RR**CPA**R**PLC |
| 503 | L3-103 | 0.676 | NDW**K**L**RK**DFW**R**NHF |
| 504 | L3-104 | 0.57 | ASV**R**D**RK**MGY**K**SDG |
| 505 | L3-105 | 0.601 | YYE**K**I**RR**GEI**K**IAE |
| 506 | L3-106 | 0.642 | DLV**R**K**RK**TML**R**QLV |
| 507 | L3-107 | 0.658 | DLS**K**V**RR**GHG**K**NDI |
| 508 | L3-108 | 0.621 | ESY**R**L**RR**GTD**K**EQW |
| 509 | L3-109 | 0.613 | YIT**K**F**RK**FLM**K**DQF |
| 510 | L3-110 | 0.538 | AWA**K**Y**KK**VQP**K**HHT |
| 511 | L3-111 | 0.976 | TWL**R**G**RR**WPD**K**QPQ |
| 512 | L3-112 | 0.731 | ALP**R**V**RK**DSH**R**EEI |
| 513 | L3-113 | 0.766 | CLF**K**Y**RK**SCV**R**LCG |
| 514 | L3-114 | 0.646 | LQN**R**M**RK**IYW**K**TFD |
| 515 | L3-115 | 0.685 | NDP**R**L**RK**HNH**R**CCT |
| 516 | L3-116 | 0.644 | YGS**K**Q**RR**FEE**K**ICG |
| 517 | L3-117 | 0.696 | CGH**K**N**KK**WHN**R**MDP |
| 518 | L3-118 | 0.69 | YGI**R**D**RR**PMT**K**HTQ |
| 519 | L3-119 | 0.693 | TPL**K**S**RK**YWN**K**HAY |
| 520 | L3-120 | 0.887 | FLY**K**A**KK**ALM**R**DSL |
| 521 | L3-121 | 0.655 | CDD**K**P**KK**TW**K**LTW |
| 522 | L3-122 | 0.691 | MHP**R**P**RK**MSH**K**MSC |
| 523 | L3-123 | 0.654 | PGE**R**L**KK**EDH**R**ASG |
| 524 | L3-124 | 0.515 | GAQ**R**A**RK**PVL**R**AVG |
| 525 | L3-125 | 0.629 | MQE**K**C**RR**CVF**K**GNI |
| 526 | L3-126 | 0.542 | YVD**R**W**RR**MQY**K**LSI |
| 527 | L3-127 | 0.608 | WVD**R**S**RK**FED**R**NCL |
| 528 | L3-128 | 0.523 | IVN**R**A**KR**SQI**R**FDV |
| 529 | L3-129 | 0.606 | IHP**R**Y**KR**HQA**R**SCC |
| 530 | L3-130 | 0.531 | EHS**K**H**RK**DLF**R**MVG |
| 531 | L3-131 | 0.655 | YTP**K**F**RR**IFW**R**IID |
| 532 | L3-132 | 0.619 | TIT**R**V**KR**ASH**K**TPS |
| 533 | L3-133 | 0.566 | VDS**R**E**KK**WRQ**K**CQC |
| 534 | L3-134 | 0.653 | YWF**R**I**KR**AHA**K**GCP |
| 535 | L3-135 | 0.69 | NDH**R**M**RR**SVD**R**GET |
| 536 | L3-136 | 0.512 | PFD**R**A**RR**DLH**R**IMM |
| 537 | L3-137 | 0.565 | QVV**R**L**RR**GKN**R**GTV |
| 538 | L3-138 | 0.576 | TNQ**R**P**RR**ETA**K**TIE |
| 539 | L3-139 | 0.49 | FHH**R**H**KR**IAT**K**HPA |
| 540 | L3-140 | 0.607 | EHC**K**W**KK**MFD**K**EGE |
| 541 | L3-141 | 0.504 | FFY**R**L**RR**CLT**R**WWV |
| 542 | L3-142 | 0.502 | LQC**K**Y**RR**GSD**K**TVV |
| 543 | L3-143 | 0.559 | QTM**K**F**KR**CCN**K**EMV |
| 544 | L3-144 | 0.578 | CSY**K**L**RR**CDM**R**LWG |
| 545 | L3-145 | 0.495 | MHI**K**M**KR**EQV**K**DEE |
| 546 | L3-146 | 0.512 | AVW**R**N**RK**DNV**K**ASE |
| 547 | L3-147 | 0.562 | ADH**R**A**KK**TLS**K**FWT |
| 548 | L3-148 | 0.451 | MMA**R**C**RR**IMC**R**SFN |
| 549 | L3-149 | 0.571 | PFG**K**F**KR**LEN**K**DEC |
| 550 | L3-150 | 0.518 | YLS**R**T**KR**WLA**R**YVE |
| 551 | L3-151 | 0.624 | HTL**K**P**KR**LYP**R**PSE |
| 552 | L3-152 | 0.818 | VVI**K**C**RK**WTH**K**MDH |
| 553 | L3-153 | 0.768 | FHT**KLRK**PIP**K**IDM |
| 554 | L3-154 | 0.766 | ENI**K**S**RK**YFV**K**LTW |
| 555 | L3-155 | 0.794 | AWT**R**F**RR**FQC**K**GMS |
| 556 | L3-156 | 0.799 | IVP**R**D**RK**QAL**R**WTN |
| 557 | L3-157 | 0.731 | CML**R**W**KR**WHM**R**FNP |
| 558 | L3-158 | 0.738 | FDM**R**D**RR**PNA**R**VMP |
| 559 | L3-159 | 0.786 | QIA**K**L**KR**DQM**K**EAW |
| 560 | L3-160 | 0.792 | LTS**R**C**KK**TFQ**K**NSE |
| 561 | L3-161 | 0.733 | MAA**K**W**KK**DGM**K**SHY |
| 562 | L3-162 | 0.781 | ANW**R**T**RR**DLA**K**EHV |
| 563 | L3-163 | 0.78 | PFT**K**M**RK**QFQ**R**MSA |
| 564 | L3-164 | 0.746 | QIN**R**V**KK**SDA**K**FCT |
| 565 | L3-165 | 0.771 | YLV**K**L**RR**FWA**K**IDM |
| 566 | L3-166 | 0.629 | EYV**K**A**KR**TPW**K**YVV |
| 567 | L3-167 | 0.634 | AMC**R**E**KR**FPY**R**AIY |
| 568 | L3-168 | 0.775 | MGL**R**A**RK**YED**K**TLH |
| 569 | L3-169 | 0.783 | FHM**K**S**KK**DGD**K**FVM |
| 570 | L3-170 | 0.768 | CIF**K**F**KK**NMF**K**GYS |
| 571 | L3-171 | 0.629 | LDT**K**N**RR**GAS**K**WDY |
| 572 | L3-172 | 0.634 | VTG**K**G**RR**VTF**R**CMN |
| 573 | L3-173 | 0.775 | QLG**K**Q**KR**EAT**R**EYV |
| 574 | L3-174 | 0.783 | SSG**K**W**KR**PHA**K**YIV |
| 575 | L3-175 | 0.768 | TCH**K**T**KR**SIM**R**ATS |
| 576 | L3-176 | 0.739 | MIG**K**C**RK**CGT**K**CYA |
| 577 | L3-177 | 0.735 | FQP**K**S**RR**AET**K**HSY |
| 578 | L3-178 | 0.755 | PDL**K**F**RK**HQD**R**TAI |
| 579 | L3-179 | 0.743 | YNA**R**V**KR**EGI**K**NIT |
| 580 | L3-180 | 0.7 | QGI**R**P**RK**HLQ**R**MPN |
| 581 | L3-181 | 0.624 | GPC**R**P**RR**HSI**R**LLW |
| 582 | L3-182 | 0.818 | FYL**R**E**RR**HQL**K**TEF |
| 583 | L3-183 | 0.768 | NMA**R**S**RK**NEC**K**YIA |
| 584 | L3-184 | 0.766 | DMD**K**V**KK**WVW**R**SFP |
| 585 | L3-185 | 0.794 | VLP**K**E**RR**WLT**R**FLI |
| 586 | L3-186 | 0.799 | YEH**R**H**KR**FFH**K**DMP |
| 587 | L3-187 | 0.731 | HGH**K**P**RR**WIY**R**MPM |
| 588 | L3-188 | 0.738 | DGI**R**D**KK**CCW**R**DLI |
| 589 | L3-189 | 0.786 | ELV**K**H**RR**LDF**R**DPM |
| 590 | L3-190 | 0.792 | VHH**K**L**RR**VHL**R**LDV |
| 591 | L3-191 | 0.739 | GIN**R**G**RK**VSP**K**DEQ |
| 592 | L3-192 | 0.735 | NGY**R**A**RK**ENL**K**FPE |
| 593 | L3-193 | 0.755 | AAY**R**A**RK**IVE**K**SGW |
| 594 | L3-194 | 0.743 | WAP**R**D**RR**NMG**R**DPA |
| 595 | L3-195 | 0.7 | IED**K**S**KR**FNC**K**ECG |
| 596 | L3-196 | 0.746 | IEM**R**Y**RR**DCS**R**FVN |
| 597 | L3-197 | 0.771 | QQI**K**Y**KR**PTY**R**MVC |
| 598 | L3-198 | 0.78 | LPW**R**E**RR**NGG**R**SSE |
| 599 | L3-199 | 0.781 | PQT**R**Y**RK**YFY**R**PQM |
| 600 | L3-200 | 0.733 | HMC**R**W**RR**QIN**K**GVS |
| | **Spep-2 (con)** | **0.303** | **WTWKGRKSLLR** |

**TABLE 13**

| SEQ ID NO | Peptide | OD | Seq. |
|---|---|---|---|
| **601** | **L2-201** | **0.307** | RRR**R**R**RR**RRR**R** |
| **602** | **L2-202** | **0.314** | GGG**R**K**KR**RQR**R** |
| **603** | **L2-203** | **0.302** | GGR**K**K**RR**QRR**R** |
| **604** | **L3-201** | **0.315** | GGG**R**K**KR**RQR**R**RGG |
| **605** | **L3-202** | **0.301** | GGR**K**K**RR**QRR**R**GGG |
| **605** | **L2-204** | **0.311** | GGG**R**R**RR**RRR**R** |
| | **Spep-2 (con)** | **0.303** | **WTWKGRKSLLR** |

### <Example 14: Investigating acetylcholine receptor binding affinity of optimized peptides>

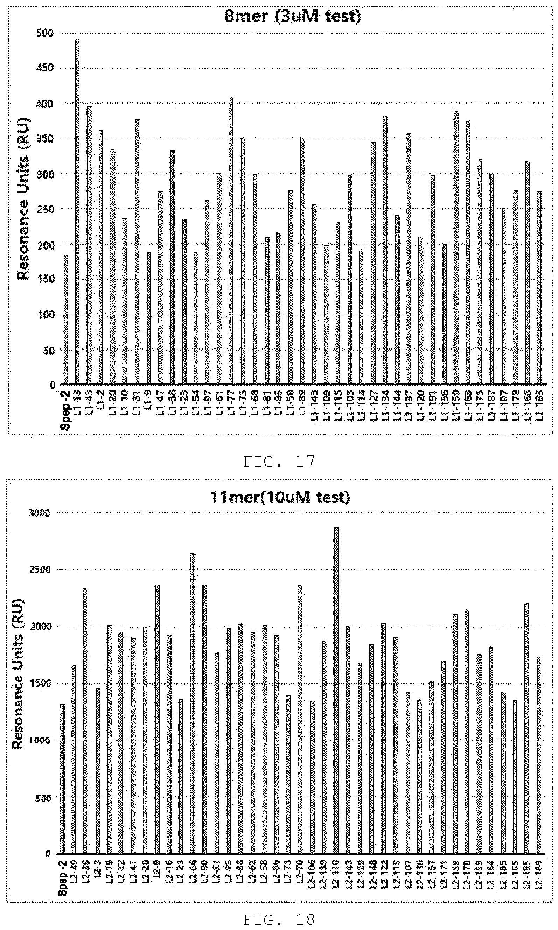

As for each library of the optimized peptides having excellent binding specificity to AchR in Example 13-3, forty peptides were selected and synthesize, and compared for AchR binding affinity (resonance unit: Ru) by using the surface plasmon resonance (SPR) analysis method in Example 2. The optimized peptides were tested at concentration conditions of 3 µM and 10 µM, and the results are shown in FIGS. 17 to 19. Spep-2 was used as a positive control. All of forty 8-mer (L1), 11-mer (L2), and 14-mer (L3) optimized peptides showed high binding affinity to AchR compared with the positive control Spep-2, and 20 peptides with high binding affinity to AchR were deduced for each library.

### <Example 15: Investigating acetylcholine receptor inhibition of respective optimized peptides (L1, L2, L3) >

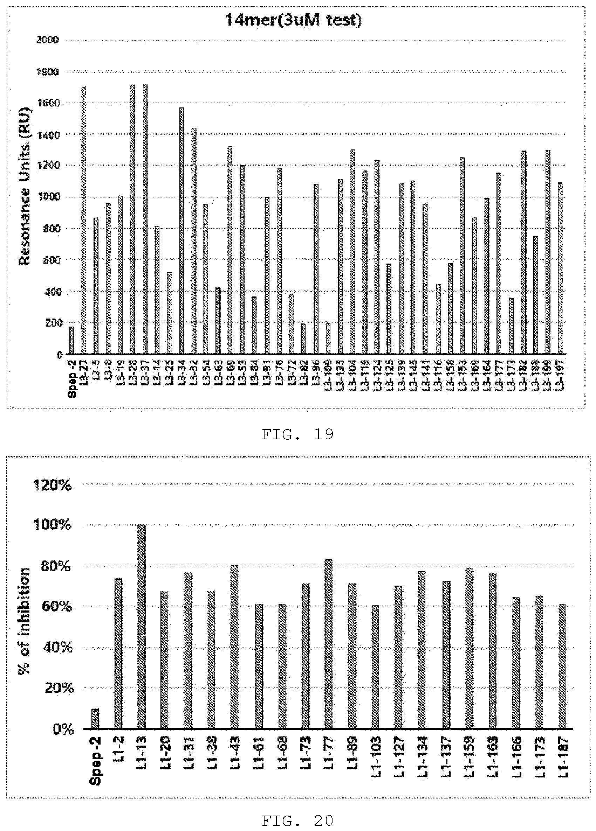

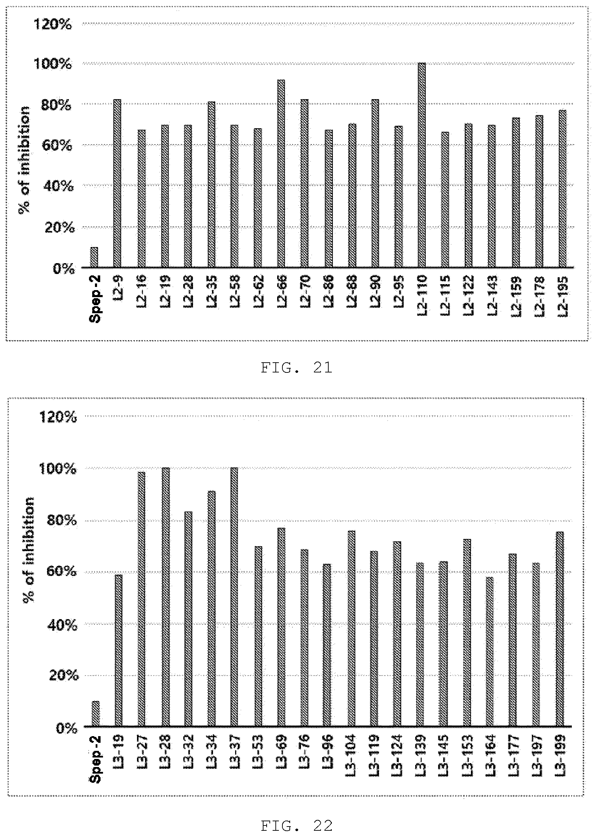

To investigate the AchR inhibitory effect of the 8-mer, 11-mer, and 14-mer peptides with excellent binding affinity to AchR identified in Example 14, the AchR inhibitory effect was investigated by the same method as in Example 3. The treatment with each of the peptides was conducted at 20 µM, and the treatment with Spep-2 as a control was also conducted. To investigate the excellent AchR inhibitory ability of the optimized peptides, a high concentration of nicotine was added at 400 µM or 600 µM, and the results are shown in FIGS. 20 to 22.

As shown in FIGS. 20 to 22, in cases of the addition of nicotine at a high concentration of 400 µM and the treatment with each peptide at 20 µM, the AchR inhibitory rate of Spep-2 as a control was about 10%, indicating little effect, whereas the AchR inhibitory rates of the 8-mer, 11-mer, and 14-mer peptides were all 50% or more, indicating excellent inhibitory effects compared with Spep-2. Out of these, the 8-mer L1-13, 11-mer L2-110, and 14-mer L3-27, 28, and 37 peptides showed an inhibitory effect close to 100%. Furthermore, it was verified that the AchR inhibitory rate of the peptides in cells were almost identical to the AchR binding affinity of each of the peptides in Example 14.

### <Example 16: Investigating acetylcholine receptor binding affinity of representative peptides>

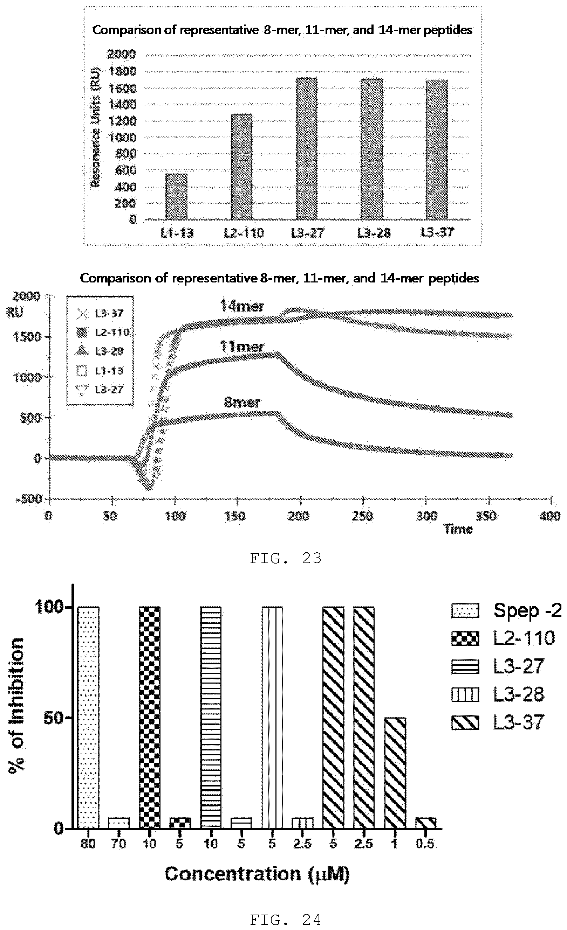

Representative peptides L1-13, L2-110, L3-27, L3-28, and L3-37 identified in Example 15 were compared for AchR binding affinity (resonance units: Ru) by using the surface plasmon resonance (SPR) analysis method in Example 2. The peptides were used at the same concentration condition of 3 µM. The results are shown in FIG. 23.

As shown in FIG. 23, the 11-mer L2-110 and the 14-mer L3-27, 28, and 37 showed higher binding affinity to AchR compared with the 8-mer L1-13, and the 14-mer L3-27, 28, and 37 showed the highest binding affinity. It was therefore verified that when the peptides of the formulas of the present invention were formed to be 8-mer or 11-mer, such peptides also showed high binding affinity to AchR, but the 14-mer peptides had the optimum binding affinity.

### <Example 17: Investigating acetylcholine receptor inhibition of representative peptides L2-110, and L3-27, 28, and 37>

To investigate the AchR inhibitory effect of the L2-110, L3-27, L3-28, and L3-37 peptides identified to have excellent binding ability to AchR in Example 16, the AchR inhibitory effect was investigated by the same method as in Example 3. The treatment with each of the peptides was conducted at different concentrations, and the treatment with Spep-2 as a control was also conducted. The results are shown in FIG. 24.

As shown in FIG. 24, as for nicotine at a high concentration of 400 µM, Spep-2 inhibited AchR by 100% at 80 µM, but could not inhibit at a concentration of 70 µM. The 11-mer L2-110 and the 14-mer L3-27 inhibited AchR by 100% at 10 µM, but could not inhibit at 5 µM. The 14-mer L3-28 inhibited AchR by 100% at 5 µM, but could not inhibit at 2.5 µM. In addition, the 14-mer L3-27 inhibited AchR by 100% at 2.5 µM, and inhibited by 50% even at 1 µM.

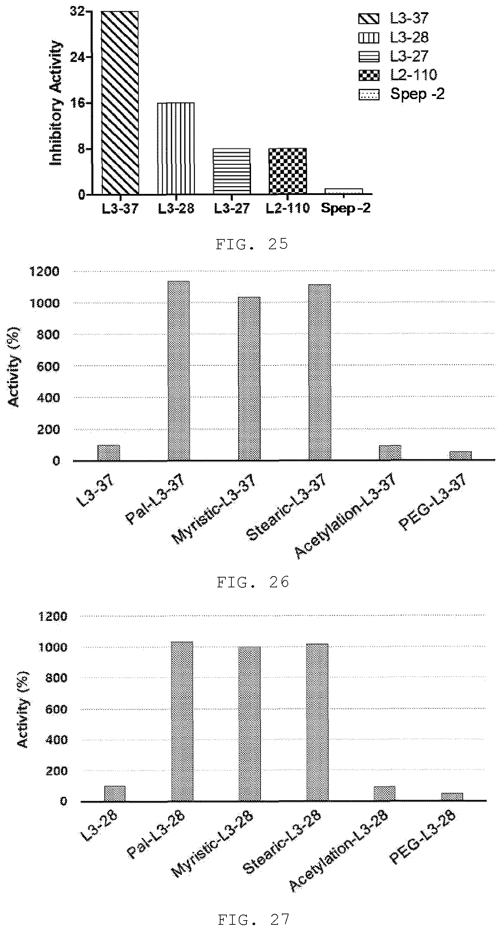

From the above results, the inhibitory ability on nicotinic acetylcholine receptors was expressed in multiples in FIG. 25. Compared with Spep-2, L2-110 and L3-27 showed an inhibitory effect improved by 8 times, L3-28 by 16 times, and L3-37 by 32 times. It was finally verified that the inhibitory effects of these peptides were also excellent in proportion with the binding ability to acetylcholine receptors in Example 15.

### <Example 18: Investigating binding affinity to acetylcholine receptors according to various modifications at each terminus of representative peptides>

Each terminus of the L2-110, L3-27, L3-28, and L3-37 peptides identified in Example 17 was variously modified, and the change in AchR binding affinity was investigated therefor. Specifically, the peptides modified by the attachment of myristic acid or stearic acid in addition to palmitoyl, which are fatty acid derivatives, by the same method as in Example 8, or by acetylation or PEGylation, were compared with the basic peptide without modification. The results are shown in FIGS. 26 to 29.

As shown in FIGS. 26 to 29, when the fatty acid derivatives were attached to the terminus of the peptides according to the present invention, the binding affinity of all the peptides was increased by about 10 times (palmitoyl-, myristyl-, stearic-). The peptides modified by the attachment of palmitoyl showed higher binding affinity than the peptides with the attachment of other fatty acid derivatives.

The AchR binding affinity when the terminus of the peptides of the present invention was acetylated was similar to that when the terminus of the peptides was not modified. The AchR binding affinity when the terminus of the peptides of the present invention was PEGylated was reduced by about 50% compared with that when the termini of the peptides were not modified.

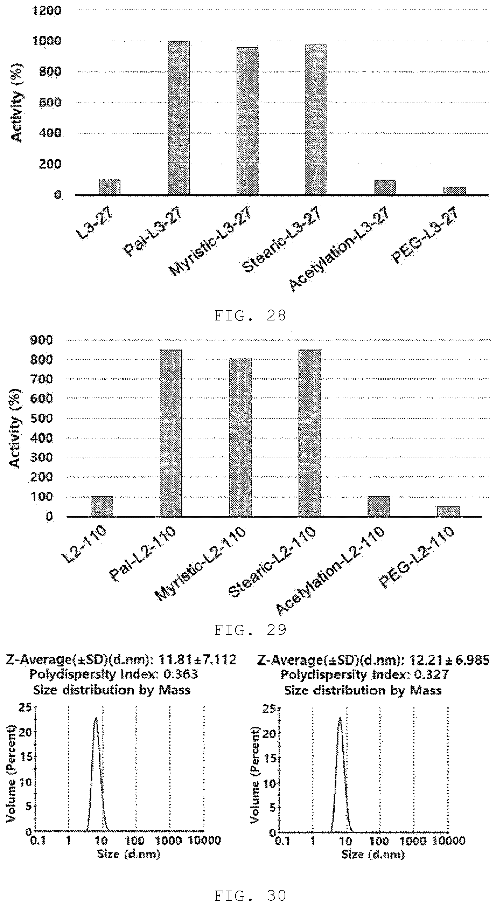

As shown in FIG. 30, the attachment of the fatty acid derivative meristic acid or stearic acid to the terminus of the peptides of the present invention induced the formation of a micelle structure, like the attachment of palmitoyl in Example 8.

### <Example 19: Investigating acetylcholine receptor inhibition of Palmitoyl-L2-110, Palmitoyl-L3-27, Palmitoyl-L3-28, and Palmitoyl-L3-37>

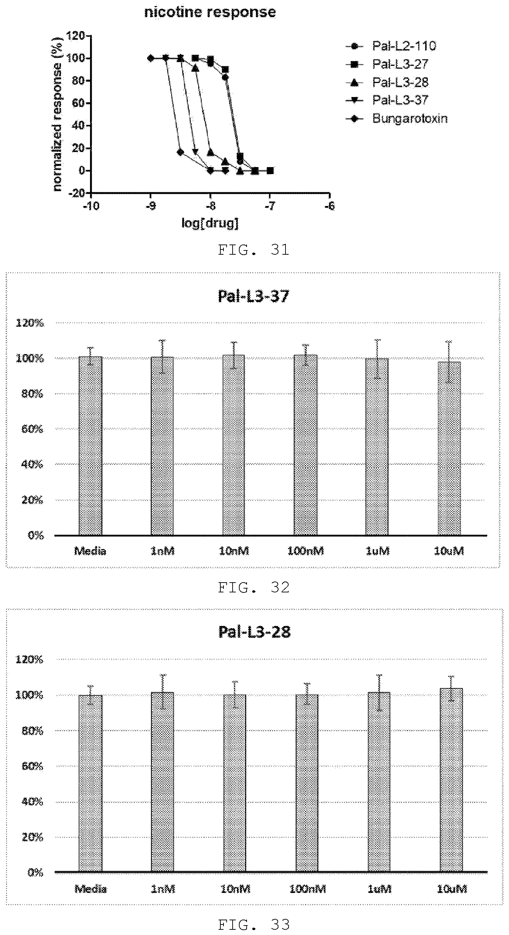

The AchR inhibitory effects of the Palmitoyl-L2-110, Palmitoyl-L3-27, Palmitoyl-L3-28, and Palmitoyl-L3-37 peptides with excellent binding affinity to AchR in Example 18 were investigated by the same method as in Example 3. Bungarotoxin was also used as a control. The results are shown in FIG. 31.

As shown in FIG. 31, the binding affinity to AchR was highest in Palmitoyl-L3-37, followed by Palmitoyl-L3-28, and Palmitoyl-L3-27 and Palmitoyl-L2-110 showed similar levels.

### <Example 20: Investigating cytotoxicity of Palmitoyl-L2-110, Palmitoyl-L3-27, Palmitoyl-L3-28, and Palmitoyl-L3-37 peptides>

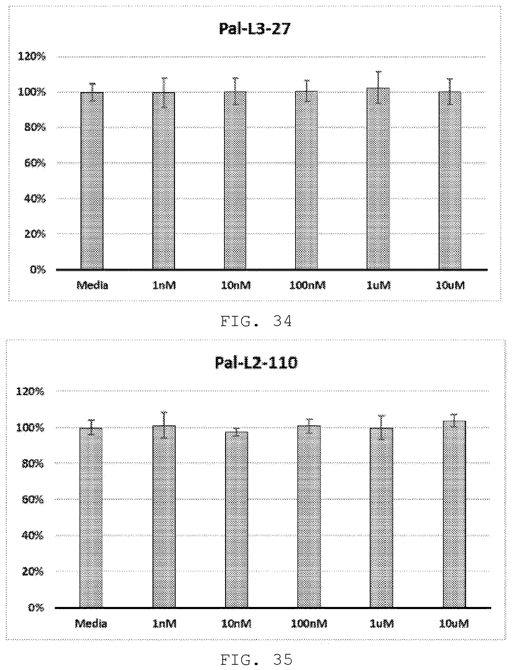

To evaluate cytotoxicity of the Palmitoyl-L2-110, Palmitoyl-L3-27, Palmitoyl-L3-28, and Palmitoyl-L3-37 peptides, WST assay was performed on TE671 cells, and the results are shown in FIGS. 32 to 35.

As shown in FIGS. 32 to 35, all the peptides showed no cytotoxicity even at a treatment concentration of 10 µM.

### <Example 21: Analyzing in vivo efficacy of Palmitoyl-L3-37>

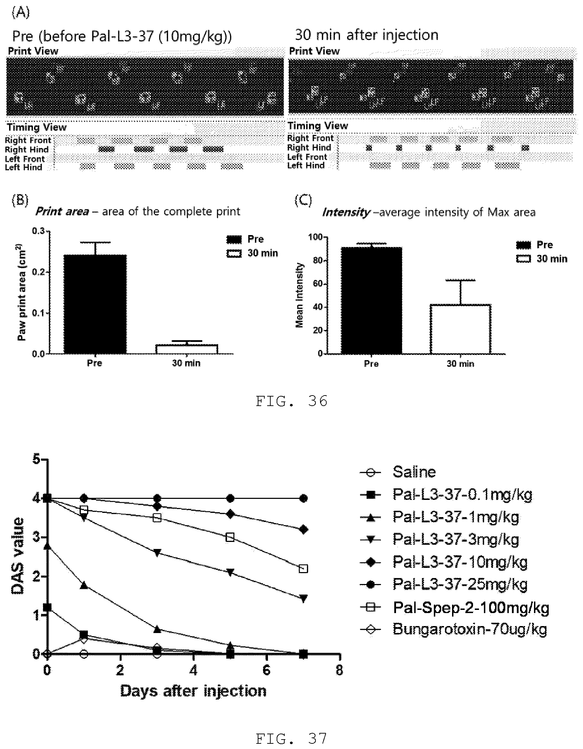

Animal efficacy assay was performed using Palmitoyl-L3-37, which had the highest binding affinity to acetylcholine receptors in Example 18. Female BALB/c mice aged 6 weeks were injected with Palmitoyl-L3-37 at a concentration of 10 mg/kg into the right hind thigh muscle (IM). The CatWalk data of the mice before injection and the CatWalk data of the mice 30 minutes after injection were compared and analyzed, and the results are shown in FIG. 36.

As shown in FIG. 36, the area where the sole of the right hind leg touched the floor was reduced after the injection of Palmitoyl-L3-37 compared with before the injection (A), and the area where the sole of the mouse touched the floor (B) and the average value of the maximum values of area (C) were also reduced after the injection of Palmitoyl-L3-37.

It can be therefore seen that Palmitoyl-L3-37 binds to acetylcholine receptors to thereby affect muscle relaxation in animals through the inhibition of acetylcholine.

### <Example 22: Evaluating animal efficacy (ED50) and animal toxicity (LD50) of Palmitoyl-L3-37>

Animal efficacy (ED50) assay was conducted by digit abduction score (DAS) assay. The DAS assay was developed to measure the local muscle-weakening efficacy of a drug intramuscular (IM) injected to the mouse hind leg skeletal muscle, and Botox was also evaluated by the same method (Aoki, 1999).

Specifically, the DAS values for Palmitoyl-L3-37 in Example 18 were evaluated. Forty-five 6-week-old female BALB/c mice were randomly divided into: 5 groups, which were treated with Palmitoyl-L3-37 at 0.1 mg/kg, 1 mg/kg, 3 mg/kg, 10 mg/kg, 25 mg/kg; and a group treated with a saline solution (sterilized 0.9% NaCl) or 70 µg/kg bungarotoxin as a control group. Then, palmitoyl-L3-37 and the saline solution or bungarotoxin at the concentrations as above were injected each 50 ul into the hind thigh muscles of mice. Thereafter, the mice weight and the sole of the injected portion were observed and recorded every day, and the results are shown in FIGS. 37 to 39.

Referring to FIG. 37, as a result of observation for 7 days after injection, on 10 minutes (Day 0) after the administration of each sample, the Palmitoyl-L3-37 1mg/kg administration group was evaluated as DAS 2; and the Palmitoyl-L3-37 3mg/kg, 10mg/kg, and 25mg/kg administration groups were evaluated as DAS 4; and the Palmitoyl-L3-37 1mg/kg administration group and the saline solution or bungarotoxin administration group as a control were evaluated as DAS 0. It can be therefore seen that the efficacy (ED50) of Palmitoyl-L3-37 was 1 mg/kg in the animal test. In addition, during the 7-day observation, the DAS value was decreased and recovered in the other groups excluding the Palmitoyl-L3-37 25mg/kg group, but DAS4 was maintained in the Palmitoyl-L3-37 25mg/kg group.

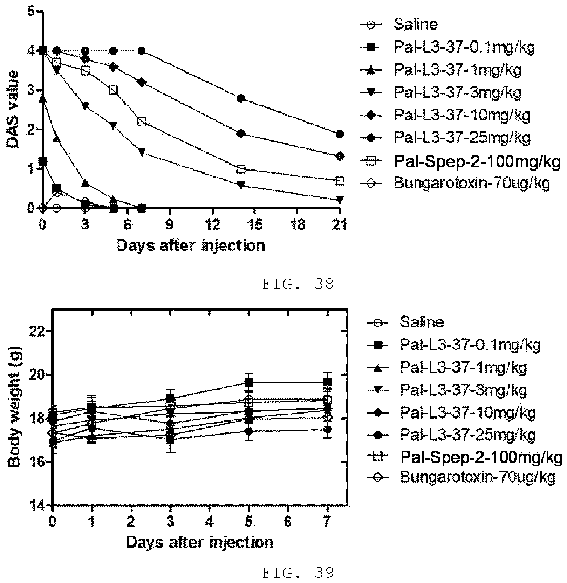

Referring to FIG. 38, as a result of observing the Palmitoyl-L3-37 25mg/kg administration group at weekly intervals until the 21st day after administration, the DAS value was gradually decreased after 7 days in the Palmitoyl-L3-37 25mg/kg administration group, and gradually decreased to 1 or less and recovered after 19 days of administration of Palmitoyl-L3-37. It can be therefore seen that the efficacy of Palmitoyl-L3-37 was reversible.

Last, as a result of evaluating the acute toxicity of Palmitoyl-L3-37 by measuring the weight every day for 7 days after administration (see FIG. 39), the weight of mice gradually increased for 7 days in all groups, and no toxicity was observed in the Palmitoyl-L3-37 administration group, and even at 25 mg/kg, which was the highest treatment concentration.

## Claims

1. An acetylcholine receptor-binding peptide comprising an amino acid sequence set forth in Formula 1 below:
[Formula 1] X_{L}-(K or R)-X-(K or R)-X_{M}-(K or R)-X_{N}
(X_{L}, X_{M}, and X_{N} each independently represent a sequence composed of one to eight arbitrary amino acids and X represents a sequence composed of one arbitrary amino acid).

2. The acetylcholine receptor-binding peptide of claim 1, wherein the peptide comprises the amino acid sequence set forth in Formula 1 above, in which X_{L}, X_{M}, and X_{N} each are independently composed of one to four arbitrary amino acids and X is composed of one arbitrary amino acid.

3. An acetylcholine receptor-binding peptide comprising an amino acid sequence set forth in Formula 2 below:
[Formula 2] (K or R)-X-(K or R)-(K or R)-X_{M-1}-(K or R)
(X_{M―1} represents a sequence composed of one to three arbitrary amino acids and X represents a sequence composed of one arbitrary amino acid).

4. The acetylcholine receptor-binding peptide of claim 3, wherein the peptide comprises the amino acid sequence set forth in Formula 2 above, in which X_{M―1} is composed of three arbitrary amino acids and X is composed of one arbitrary amino acid.

5. The acetylcholine receptor-binding peptide of claim 4, wherein the peptide is a peptide of any one sequence selected from the group consisting of SEQ ID NOs: 1 to 200.

6. An acetylcholine receptor-binding peptide comprising an amino acid sequence set forth in Formula 3 below:
[Formula 3] X_{L}-(K or R)-X-(K or R)-(K or R)-X_{M―1}-(K or R)
(X_{L} and X_{M―1} each independently represent a sequence composed of one to three arbitrary amino acids and X represents a sequence composed of one arbitrary amino acid).

7. The acetylcholine receptor-binding peptide of claim 6, wherein the peptide comprises the amino acid sequence set forth in Formula 3 above, in which X_{L} is composed of one to three arbitrary amino acids, X_{M―1} is composed of three arbitrary amino acids, and X is composed of one arbitrary amino acid.

8. The acetylcholine receptor-binding peptide of claim 6, wherein the peptide comprises the amino acid sequence set forth in Formula 3 above, in which and X_{L} and X_{M―1} each are independently composed of three arbitrary amino acids and X is composed of one arbitrary amino acid.

9. The acetylcholine receptor-binding peptide of claim 8, wherein the peptide is a peptide of any one sequence selected from the group consisting of SEQ ID NOS: 201 to 400.

10. An acetylcholine receptor-binding peptide comprising an amino acid sequence set forth in Formula 4 below:
[Formula 4] X_{L}-(K or R)-X-(K or R)-(K or R)-X_{M―1}-(K or R)-X_{N}
(X_{L}, X_{M―1}, and X_{N} each independently represent a sequence composed of one to three arbitrary amino acids and X represents a sequence composed of one arbitrary amino acid).

11. The acetylcholine receptor-binding peptide of claim 10, wherein the peptide comprises the amino acid sequence set forth in Formula 3, in which X_{L} and X_{N} each are independently composed of one to three arbitrary amino acids, X_{M―1} is composed of three arbitrary amino acids, and X is composed of one arbitrary amino acid.

12. The acetylcholine receptor-binding peptide of claim 11, wherein the peptide comprises an amino acid sequence set forth in Formula 4 above, in which X_{L}, X_{M―1}, and X_{N} each are independently composed of three arbitrary amino acids and X is composed of one arbitrary amino acid.

13. The acetylcholine receptor-binding peptide of claim 12, wherein the peptide is a peptide of any one sequence selected from the group consisting of SEQ ID NOS: 401 to 600.

14. The acetylcholine receptor-binding peptide of any one of claims 1 to 13, wherein the N-terminus or C-terminus of the peptide is modified.

15. The acetylcholine receptor-binding peptide of claim 14, wherein the N-terminus or C-terminus is modified by palmitoylation, acetylation, amidation, formylation or PEGylation, or by a linkage of at least one selected from the group consisting of 2-mercaptoacetic acid, 3-mercaptopropionic acid, 6-mercaptohexanoic acid, pyroglutamic acid, succinimide acid, cystramine, cysteamine, methyl ester, ethyl ester, benzyl ester, myristic acid, stearic acid, palmitic acid, cholesterol, 6-amino hexanoic acid, and 8-amino octanoic acid.

16. A polynucleotide encoding the peptide of any one of claims 1 to 13.

17. A cosmetic composition for wrinkle relief, the cosmetic composition comprising the peptide of any one of claims 1 to 13.

18. A composition for preventing or treating an acetylcholine receptor hyperactivity-associated disease, the composition comprising the peptide of any one of claims 1 to 13.

19. The composition of claim 18, wherein the acetylcholine receptor hyperactivity-associated disease is at least any one selected from the group consisting of cervical dystonia, limb dystonia, truncal dystonia, blepharospasm, spasticity, hemifacial spasm, strabismus, nystagmus, tics, chronic pain, chronic migraine, neurogenic bladder, detrusor-sphincter dyssynergia, achalasia cardia, hyperhidrosis, and sialorrhea.

20. A health functional food composition for alleviating an acetylcholine receptor hyperactivity-associated disease, the composition containing the peptide of any one of claims 1 to 13.

21. A composition comprising an acetylcholine receptor-binding peptide for a medical device, wherein the composition comprises the peptide of any one of claims 1 to 13.


<110>     amicogen

<120>     Peptide for inhibition of acetylcholine receptor and use thereof

<130>     OPC-19-0027

<150>     KR 10-2018-0169425
<151>     2018-12-26

<160>     606

<170>     KoPatentIn 3.0

<210>     1
<211>     8
<212>     PRT
<213>     Artificial Sequence

<220>
<223>     AchR binding peptide


<400>     1
Lys Ala Lys Lys Ile Arg Gln Arg
  1               5


<210>     2
<211>     8
<212>     PRT
<213>     Artificial Sequence

<220>
<223>     AchR binding peptide


<400>     2
Lys Lys Arg Lys Gly Ser Ala Lys
  1               5


<210>     3
<211>     8
<212>     PRT
<213>     Artificial Sequence

<220>
<223>     AchR binding peptide


<400>     3
Arg Gly Lys Arg Gln Leu Gly Arg
  1               5


<210>     4
<211>     8
<212>     PRT
<213>     Artificial Sequence

<220>
<223>     AchR binding peptide

<400>    4
Lys Leu Lys Lys Phe Pro Val Arg
1               5

<210>    5
<211>    8
<212>    PRT
<213>    Artificial Sequence

<220>
<223>    AchR binding peptide

<400>    5
Arg His Lys Lys Ser Pro Trp Lys
1               5

<210>    6
<211>    8
<212>    PRT
<213>    Artificial Sequence

<220>
<223>    AchR binding peptide

<400>    6
Arg Pro Arg Arg Thr His Ile Lys
1               5

<210>    7
<211>    8
<212>    PRT
<213>    Artificial Sequence

<220>
<223>    AchR binding peptide

<400>    7
Lys His Lys Lys Leu Asn Gln Arg
1               5

<210>    8
<211>    8
<212>    PRT
<213>    Artificial Sequence

<220>
<223>    AchR binding peptide

<400>    8
Arg Ala Lys Arg Met Cys Cys Lys
1               5

<210>    9

<211>    8
<212>    PRT
<213>    Artificial Sequence

<220>
<223>    AchR binding peptide

<400>    9
Arg Gln Arg Arg Asn Asp Met Lys
1               5

<210>    10
<211>    8
<212>    PRT
<213>    Artificial Sequence

<220>
<223>    AchR binding peptide

<400>    10
Lys Arg Lys Lys Asp Trp Trp Arg
1               5

<210>    11
<211>    8
<212>    PRT
<213>    Artificial Sequence

<220>
<223>    AchR binding peptide

<400>    11
Arg Ala Arg Lys Asp Ala Phe Lys
1               5

<210>    12
<211>    8
<212>    PRT
<213>    Artificial Sequence

<220>
<223>    AchR binding peptide

<400>    12
Lys Trp Arg Arg Glu Gly Phe Lys
1               5

<210>    13
<211>    8
<212>    PRT
<213>    Artificial Sequence

<220>
<223>    AchR binding peptide

<400>    13
Arg Phe Arg Arg Gly Val Arg Arg
1               5

<210>    14
<211>    8
<212>    PRT
<213>    Artificial Sequence

<220>
<223>    AchR binding peptide

<400>    14
Lys Ala Arg Arg Gln Glu Asp Lys
1               5

<210>    15
<211>    8
<212>    PRT
<213>    Artificial Sequence

<220>
<223>    AchR binding peptide

<400>    15
Arg Phe Lys Arg Leu Cys Met Lys
1               5

<210>    16
<211>    8
<212>    PRT
<213>    Artificial Sequence

<220>
<223>    AchR binding peptide

<400>    16
Lys Leu Lys Arg Arg Ser Arg Arg
1               5

<210>    17
<211>    8
<212>    PRT
<213>    Artificial Sequence

<220>
<223>    AchR binding peptide

<400>    17
Arg Pro Arg Arg Ser Leu Asp Arg
1               5

<210>    18

<211>    8
<212>    PRT
<213>    Artificial Sequence

<220>
<223>    AchR binding peptide


<400>    18
Lys His Lys Lys Gln Asn Val Arg
1               5


<210>    19
<211>    8
<212>    PRT
<213>    Artificial Sequence

<220>
<223>    AchR binding peptide


<400>    19
Lys Phe Lys Arg Thr Glu Gly Arg
1               5


<210>    20
<211>    8
<212>    PRT
<213>    Artificial Sequence

<220>
<223>    AchR binding peptide


<400>    20
Arg Gly Lys Lys Ala Val Arg Lys
1               5


<210>    21
<211>    8
<212>    PRT
<213>    Artificial Sequence

<220>
<223>    AchR binding peptide


<400>    21
Lys Phe Lys Lys His Gly Val Lys
1               5


<210>    22
<211>    8
<212>    PRT
<213>    Artificial Sequence

<220>
<223>    AchR binding peptide

<400>    22
Lys Thr Lys Lys Ile His Ser Lys
1               5

<210>    23
<211>    8
<212>    PRT
<213>    Artificial Sequence

<220>
<223>    AchR binding peptide

<400>    23
Arg Asp Arg Lys Ile His Asp Lys
1               5

<210>    24
<211>    8
<212>    PRT
<213>    Artificial Sequence

<220>
<223>    AchR binding peptide

<400>    24
Arg Pro Arg Arg Gly His Gln Lys
1               5

<210>    25
<211>    8
<212>    PRT
<213>    Artificial Sequence

<220>
<223>    AchR binding peptide

<400>    25
Lys Thr Lys Lys Leu Tyr Glu Lys
1               5

<210>    26
<211>    8
<212>    PRT
<213>    Artificial Sequence

<220>
<223>    AchR binding peptide

<400>    26
Arg Gln Lys Lys Asp Asn Gln Lys
1               5

<210>    27

<211>    8
<212>    PRT
<213>    Artificial Sequence

<220>
<223>    AchR binding peptide


<400>    27
Arg Gln Arg Lys Ser Met Gln Arg
1               5


<210>    28
<211>    8
<212>    PRT
<213>    Artificial Sequence

<220>
<223>    AchR binding peptide


<400>    28
Arg Gly Arg Arg Trp Phe Val Lys
1               5


<210>    29
<211>    8
<212>    PRT
<213>    Artificial Sequence

<220>
<223>    AchR binding peptide


<400>    29
Arg Asn Lys Arg Asp Glu Asn Lys
1               5


<210>    30
<211>    8
<212>    PRT
<213>    Artificial Sequence

<220>
<223>    AchR binding peptide


<400>    30
Arg Leu Arg Arg Trp Cys Val Lys
1               5


<210>    31
<211>    8
<212>    PRT
<213>    Artificial Sequence

<220>
<223>    AchR binding peptide

<400>    31
Arg Gln Lys Lys Pro Trp Val Lys
1               5

<210>    32
<211>    8
<212>    PRT
<213>    Artificial Sequence

<220>
<223>    AchR binding peptide

<400>    32
Arg Trp Lys Arg Ser Glu Gln Arg
1               5

<210>    33
<211>    8
<212>    PRT
<213>    Artificial Sequence

<220>
<223>    AchR binding peptide

<400>    33
Arg Leu Lys Arg Ala Pro Gly Arg
1               5

<210>    34
<211>    8
<212>    PRT
<213>    Artificial Sequence

<220>
<223>    AchR binding peptide

<400>    34
Lys Met Arg Arg Ala Gln Gly Arg
1               5

<210>    35
<211>    8
<212>    PRT
<213>    Artificial Sequence

<220>
<223>    AchR binding peptide

<400>    35
Lys Ile Lys Lys Ala Ala Arg Arg
1               5

<210>    36

<211>    8
<212>    PRT
<213>    Artificial Sequence

<220>
<223>    AchR binding peptide


<400>    36
Lys Asn Arg Lys Asn Leu Tyr Arg
 1               5


<210>    37
<211>    8
<212>    PRT
<213>    Artificial Sequence

<220>
<223>    AchR binding peptide


<400>    37
Lys Cys Lys Lys Met Thr Glu Arg
 1               5


<210>    38
<211>    8
<212>    PRT
<213>    Artificial Sequence

<220>
<223>    AchR binding peptide


<400>    38
Lys Thr Arg Arg Val Asp Leu Arg
 1               5


<210>    39
<211>    8
<212>    PRT
<213>    Artificial Sequence

<220>
<223>    AchR binding peptide


<400>    39
Lys Ser Arg Lys Trp Gly Trp Arg
 1               5


<210>    40
<211>    8
<212>    PRT
<213>    Artificial Sequence

<220>
<223>    AchR binding peptide

<400>    40
Lys Arg Arg Arg Phe Arg Trp Lys
1               5

<210>    41
<211>    8
<212>    PRT
<213>    Artificial Sequence

<220>
<223>    AchR binding peptide

<400>    41
Lys Tyr Lys Lys Gln His Thr Lys
1               5

<210>    42
<211>    8
<212>    PRT
<213>    Artificial Sequence

<220>
<223>    AchR binding peptide

<400>    42
Arg Gly Arg Lys Lys Cys Gly Lys
1               5

<210>    43
<211>    8
<212>    PRT
<213>    Artificial Sequence

<220>
<223>    AchR binding peptide

<400>    43
Lys Leu Arg Lys Gln Lys Leu Arg
1               5

<210>    44
<211>    8
<212>    PRT
<213>    Artificial Sequence

<220>
<223>    AchR binding peptide

<400>    44
Arg Asn Arg Lys Ile Pro Leu Lys
1               5

<210>    45

<211>    8
<212>    PRT
<213>    Artificial Sequence

<220>
<223>    AchR binding peptide


<400>    45
Arg Leu Arg Arg Arg Tyr Arg Arg
1               5


<210>    46
<211>    8
<212>    PRT
<213>    Artificial Sequence

<220>
<223>    AchR binding peptide


<400>    46
Lys Trp Arg Lys Arg Arg Ile Lys
1               5


<210>    47
<211>    8
<212>    PRT
<213>    Artificial Sequence

<220>
<223>    AchR binding peptide


<400>    47
Lys Thr Lys Arg Met Leu Ala Lys
1               5


<210>    48
<211>    8
<212>    PRT
<213>    Artificial Sequence

<220>
<223>    AchR binding peptide


<400>    48
Lys Glu Lys Arg Ala Val His Lys
1               5


<210>    49
<211>    8
<212>    PRT
<213>    Artificial Sequence

<220>
<223>    AchR binding peptide

<400>    49
Arg Val Arg Arg Asp Gly His Arg
1               5

<210>    50
<211>    8
<212>    PRT
<213>    Artificial Sequence

<220>
<223>    AchR binding peptide

<400>    50
Arg Ala Lys Lys Cys Tyr His Lys
1               5

<210>    51
<211>    8
<212>    PRT
<213>    Artificial Sequence

<220>
<223>    AchR binding peptide

<400>    51
Lys Gly Lys Arg Asp Ser Asn Lys
1               5

<210>    52
<211>    8
<212>    PRT
<213>    Artificial Sequence

<220>
<223>    AchR binding peptide

<400>    52
Lys Val Arg Lys Ser Leu Trp Lys
1               5

<210>    53
<211>    8
<212>    PRT
<213>    Artificial Sequence

<220>
<223>    AchR binding peptide

<400>    53
Arg Ser Lys Lys Ser Leu Tyr Arg
1               5

<210>    54

<211>    8
<212>    PRT
<213>    Artificial Sequence

<220>
<223>    AchR binding peptide


<400>    54
Arg Gly Lys Arg Gly Cys Glu Arg
1               5


<210>    55
<211>    8
<212>    PRT
<213>    Artificial Sequence

<220>
<223>    AchR binding peptide


<400>    55
Lys Gln Lys Lys Thr Gly Phe Arg
1               5


<210>    56
<211>    8
<212>    PRT
<213>    Artificial Sequence

<220>
<223>    AchR binding peptide


<400>    56
Lys Tyr Arg Arg Asn Leu Val Arg
1               5


<210>    57
<211>    8
<212>    PRT
<213>    Artificial Sequence

<220>
<223>    AchR binding peptide


<400>    57
Arg Ile Arg Arg Gly Ser Val Arg
1               5


<210>    58
<211>    8
<212>    PRT
<213>    Artificial Sequence

<220>
<223>    AchR binding peptide

<400>    58
Arg Ser Lys Arg Met Asn Gly Lys
1               5

<210>    59
<211>    8
<212>    PRT
<213>    Artificial Sequence

<220>
<223>    AchR binding peptide

<400>    59
Arg His Lys Lys Phe Ile Glu Lys
1               5

<210>    60
<211>    8
<212>    PRT
<213>    Artificial Sequence

<220>
<223>    AchR binding peptide

<400>    60
Arg Ile Arg Arg Val Ser Gln Arg
1               5

<210>    61
<211>    8
<212>    PRT
<213>    Artificial Sequence

<220>
<223>    AchR binding peptide

<400>    61
Arg Pro Lys Lys Trp Met Gln Lys
1               5

<210>    62
<211>    8
<212>    PRT
<213>    Artificial Sequence

<220>
<223>    AchR binding peptide

<400>    62
Lys Glu Arg Lys His Glu Ala Lys
1               5

<210>    63

<211>    8
<212>    PRT
<213>    Artificial Sequence

<220>
<223>    AchR binding peptide


<400>    63
Lys Glu Arg Lys Ala Ser Ala Lys
1               5


<210>    64
<211>    8
<212>    PRT
<213>    Artificial Sequence

<220>
<223>    AchR binding peptide


<400>    64
Lys Met Arg Arg Ile Met Tyr Lys
1               5


<210>    65
<211>    8
<212>    PRT
<213>    Artificial Sequence

<220>
<223>    AchR binding peptide


<400>    65
Lys Trp Lys Arg Asn Cys Thr Lys
1               5


<210>    66
<211>    8
<212>    PRT
<213>    Artificial Sequence

<220>
<223>    AchR binding peptide


<400>    66
Arg Cys Lys Lys Asn Pro Glu Lys
1               5


<210>    67
<211>    8
<212>    PRT
<213>    Artificial Sequence

<220>
<223>    AchR binding peptide

<400>    67
Lys Gln Arg Lys Pro His Gly Arg
1               5

<210>    68
<211>    8
<212>    PRT
<213>    Artificial Sequence

<220>
<223>    AchR binding peptide

<400>    68
Lys Ala Lys Arg Gly His Phe Lys
1               5

<210>    69
<211>    8
<212>    PRT
<213>    Artificial Sequence

<220>
<223>    AchR binding peptide

<400>    69
Lys Ser Lys Arg His His Glu Lys
1               5

<210>    70
<211>    8
<212>    PRT
<213>    Artificial Sequence

<220>
<223>    AchR binding peptide

<400>    70
Lys Ser Arg Lys His Cys Asn Arg
1               5

<210>    71
<211>    8
<212>    PRT
<213>    Artificial Sequence

<220>
<223>    AchR binding peptide

<400>    71
Arg Ser Lys Lys Ile Asn Asn Lys
1               5

<210>    72

<211>    8
<212>    PRT
<213>    Artificial Sequence

<220>
<223>    AchR binding peptide


<400>    72
Arg Val Arg Arg Ser Trp Ile Arg
1               5


<210>    73
<211>    8
<212>    PRT
<213>    Artificial Sequence

<220>
<223>    AchR binding peptide


<400>    73
Lys Ser Arg Arg Ser Ala Gly Lys
1               5


<210>    74
<211>    8
<212>    PRT
<213>    Artificial Sequence

<220>
<223>    AchR binding peptide


<400>    74
Lys Met Arg Lys His Asp Val Arg
1               5


<210>    75
<211>    8
<212>    PRT
<213>    Artificial Sequence

<220>
<223>    AchR binding peptide


<400>    75
Lys Gly Lys Lys Leu Ser Gln Arg
1               5


<210>    76
<211>    8
<212>    PRT
<213>    Artificial Sequence

<220>
<223>    AchR binding peptide

<400>    76
Lys Trp Arg Arg Glu Gln Phe Arg
1               5

<210>    77
<211>    8
<212>    PRT
<213>    Artificial Sequence

<220>
<223>    AchR binding peptide

<400>    77
Lys Asp Lys Arg Trp Ala Asn Arg
1               5

<210>    78
<211>    8
<212>    PRT
<213>    Artificial Sequence

<220>
<223>    AchR binding peptide

<400>    78
Lys His Lys Arg Thr Ala Gln Arg
1               5

<210>    79
<211>    8
<212>    PRT
<213>    Artificial Sequence

<220>
<223>    AchR binding peptide

<400>    79
Lys Pro Lys Arg Glu Met His Lys
1               5

<210>    80
<211>    8
<212>    PRT
<213>    Artificial Sequence

<220>
<223>    AchR binding peptide

<400>    80
Arg Trp Lys Lys Asn Ile Asn Lys
1               5

<210>    81

<211>    8
<212>    PRT
<213>    Artificial Sequence

<220>
<223>    AchR binding peptide

<400>    81
Lys Trp Lys Lys Met Met Glu Arg
1               5

<210>    82
<211>    8
<212>    PRT
<213>    Artificial Sequence

<220>
<223>    AchR binding peptide

<400>    82
Arg Ile Arg Arg Trp Trp Val Arg
1               5

<210>    83
<211>    8
<212>    PRT
<213>    Artificial Sequence

<220>
<223>    AchR binding peptide

<400>    83
Arg Ile Arg Arg His Met Tyr Arg
1               5

<210>    84
<211>    8
<212>    PRT
<213>    Artificial Sequence

<220>
<223>    AchR binding peptide

<400>    84
Lys Pro Lys Lys Pro Pro Ala Arg
1               5

<210>    85
<211>    8
<212>    PRT
<213>    Artificial Sequence

<220>
<223>    AchR binding peptide

<400>    85
Arg Leu Lys Arg Gly Gln Phe Lys
1               5


<210>    86
<211>    8
<212>    PRT
<213>    Artificial Sequence

<220>
<223>    AchR binding peptide


<400>    86
Lys Asn Lys Arg Thr Pro Val Lys
1               5


<210>    87
<211>    8
<212>    PRT
<213>    Artificial Sequence

<220>
<223>    AchR binding peptide


<400>    87
Lys Trp Arg Arg Ala Phe Gly Arg
1               5


<210>    88
<211>    8
<212>    PRT
<213>    Artificial Sequence

<220>
<223>    AchR binding peptide


<400>    88
Lys Asn Lys Lys Cys Val Gly Arg
1               5


<210>    89
<211>    8
<212>    PRT
<213>    Artificial Sequence

<220>
<223>    AchR binding peptide


<400>    89
Arg Thr Arg Lys Cys Val Leu Lys
1               5


<210>    90

<211>    8
<212>    PRT
<213>    Artificial Sequence

<220>
<223>    AchR binding peptide


<400>    90
Lys Ile Lys Arg Asp Leu Tyr Lys
1               5


<210>    91
<211>    8
<212>    PRT
<213>    Artificial Sequence

<220>
<223>    AchR binding peptide


<400>    91
Arg Glu Lys Arg Tyr Met Ala Arg
1               5


<210>    92
<211>    8
<212>    PRT
<213>    Artificial Sequence

<220>
<223>    AchR binding peptide


<400>    92
Arg Val Lys Lys Ala Glu Thr Arg
1               5


<210>    93
<211>    8
<212>    PRT
<213>    Artificial Sequence

<220>
<223>    AchR binding peptide


<400>    93
Arg Gly Arg Arg Gly Thr Met Arg
1               5


<210>    94
<211>    8
<212>    PRT
<213>    Artificial Sequence

<220>
<223>    AchR binding peptide

<400>    94
Arg Thr Lys Arg Trp Gln Leu Lys
  1               5

<210>    95
<211>    8
<212>    PRT
<213>    Artificial Sequence

<220>
<223>    AchR binding peptide

<400>    95
Arg Leu Arg Lys Ser Leu Gly Lys
  1               5

<210>    96
<211>    8
<212>    PRT
<213>    Artificial Sequence

<220>
<223>    AchR binding peptide

<400>    96
Arg Pro Lys Arg Asn Trp Ala Lys
  1               5

<210>    97
<211>    8
<212>    PRT
<213>    Artificial Sequence

<220>
<223>    AchR binding peptide

<400>    97
Lys Asp Arg Lys Val Asn Ser Arg
  1               5

<210>    98
<211>    8
<212>    PRT
<213>    Artificial Sequence

<220>
<223>    AchR binding peptide

<400>    98
Lys Trp Arg Arg Asn Gly Gln Arg
  1               5

<210>    99

<211>    8
<212>    PRT
<213>    Artificial Sequence

<220>
<223>    AchR binding peptide


<400>    99
Arg Phe Lys Arg Met Val Trp Arg
1               5


<210>    100
<211>    8
<212>    PRT
<213>    Artificial Sequence

<220>
<223>    AchR binding peptide


<400>    100
Lys Glu Arg Lys Gly Ala Val Arg
1               5


<210>    101
<211>    8
<212>    PRT
<213>    Artificial Sequence

<220>
<223>    AchR binding peptide


<400>    101
Lys Thr Lys Arg Gly Ser Leu Lys
1               5


<210>    102
<211>    8
<212>    PRT
<213>    Artificial Sequence

<220>
<223>    AchR binding peptide


<400>    102
Lys Thr Lys Arg Val Asp Pro Lys
1               5


<210>    103
<211>    8
<212>    PRT
<213>    Artificial Sequence

<220>
<223>    AchR binding peptide

<400>    103
Arg Ala Arg Lys Leu His Asn Arg
1               5


<210>    104
<211>    8
<212>    PRT
<213>    Artificial Sequence

<220>
<223>    AchR binding peptide


<400>    104
Arg Gly Lys Lys Pro Trp Trp Arg
1               5


<210>    105
<211>    8
<212>    PRT
<213>    Artificial Sequence

<220>
<223>    AchR binding peptide


<400>    105
Lys Gly Lys Lys Tyr Tyr Gln Lys
1               5


<210>    106
<211>    8
<212>    PRT
<213>    Artificial Sequence

<220>
<223>    AchR binding peptide


<400>    106
Arg Trp Arg Arg Ser Val Ile Lys
1               5


<210>    107
<211>    8
<212>    PRT
<213>    Artificial Sequence

<220>
<223>    AchR binding peptide


<400>    107
Arg Gln Lys Lys Asp Phe Leu Lys
1               5


<210>    108

<211>    8
<212>    PRT
<213>    Artificial Sequence

<220>
<223>    AchR binding peptide


<400>    108
Arg Gly Arg Lys Pro Ile Trp Lys
1               5


<210>    109
<211>    8
<212>    PRT
<213>    Artificial Sequence

<220>
<223>    AchR binding peptide


<400>    109
Lys Pro Arg Arg Pro Asp Thr Arg
1               5


<210>    110
<211>    8
<212>    PRT
<213>    Artificial Sequence

<220>
<223>    AchR binding peptide


<400>    110
Lys Val Lys Arg Leu Trp Asn Arg
1               5


<210>    111
<211>    8
<212>    PRT
<213>    Artificial Sequence

<220>
<223>    AchR binding peptide


<400>    111
Lys Asp Arg Lys Gly Val Tyr Lys
1               5


<210>    112
<211>    8
<212>    PRT
<213>    Artificial Sequence

<220>
<223>    AchR binding peptide

<400>    112
Arg Val Lys Arg Phe Val Pro Lys
1               5


<210>    113
<211>    8
<212>    PRT
<213>    Artificial Sequence

<220>
<223>    AchR binding peptide


<400>    113
Lys Pro Lys Arg Asp Gln Ser Arg
1               5


<210>    114
<211>    8
<212>    PRT
<213>    Artificial Sequence

<220>
<223>    AchR binding peptide


<400>    114
Lys Asp Lys Lys Gln Trp Gly Arg
1               5


<210>    115
<211>    8
<212>    PRT
<213>    Artificial Sequence

<220>
<223>    AchR binding peptide


<400>    115
Arg Phe Arg Lys Met Ile Pro Lys
1               5


<210>    116
<211>    8
<212>    PRT
<213>    Artificial Sequence

<220>
<223>    AchR binding peptide


<400>    116
Lys Phe Arg Lys His Val Cys Lys
1               5


<210>    117

<211>    8
<212>    PRT
<213>    Artificial Sequence

<220>
<223>    AchR binding peptide


<400>    117
Arg Pro Lys Arg Ser Pro Ser Lys
1               5


<210>    118
<211>    8
<212>    PRT
<213>    Artificial Sequence

<220>
<223>    AchR binding peptide


<400>    118
Arg Tyr Lys Lys Val Pro Ala Lys
1               5


<210>    119
<211>    8
<212>    PRT
<213>    Artificial Sequence

<220>
<223>    AchR binding peptide


<400>    119
Arg Ser Arg Arg Cys Pro Val Lys
1               5


<210>    120
<211>    8
<212>    PRT
<213>    Artificial Sequence

<220>
<223>    AchR binding peptide


<400>    120
Arg Cys Lys Arg Cys Asp Asn Lys
1               5


<210>    121
<211>    8
<212>    PRT
<213>    Artificial Sequence

<220>
<223>    AchR binding peptide

<400>    121
Arg Gly Lys Lys Gln Leu Ser Lys
1               5

<210>    122
<211>    8
<212>    PRT
<213>    Artificial Sequence

<220>
<223>    AchR binding peptide

<400>    122
Lys Ser Lys Arg Pro Glu Gly Arg
1               5

<210>    123
<211>    8
<212>    PRT
<213>    Artificial Sequence

<220>
<223>    AchR binding peptide

<400>    123
Lys Cys Arg Lys Thr Met Gly Arg
1               5

<210>    124
<211>    8
<212>    PRT
<213>    Artificial Sequence

<220>
<223>    AchR binding peptide

<400>    124
Arg Asp Arg Lys Asn Pro Val Arg
1               5

<210>    125
<211>    8
<212>    PRT
<213>    Artificial Sequence

<220>
<223>    AchR binding peptide

<400>    125
Lys Pro Lys Arg Ser Ala Pro Arg
1               5

<210>    126

<211>    8
<212>    PRT
<213>    Artificial Sequence

<220>
<223>    AchR binding peptide

<400>    126
Lys Gly Lys Arg Thr Gly Cys Lys
1               5

<210>    127
<211>    8
<212>    PRT
<213>    Artificial Sequence

<220>
<223>    AchR binding peptide

<400>    127
Arg Phe Arg Lys Pro Thr Asp Arg
1               5

<210>    128
<211>    8
<212>    PRT
<213>    Artificial Sequence

<220>
<223>    AchR binding peptide

<400>    128
Arg Met Lys Lys Phe His Thr Arg
1               5

<210>    129
<211>    8
<212>    PRT
<213>    Artificial Sequence

<220>
<223>    AchR binding peptide

<400>    129
Lys Ile Lys Lys Tyr Tyr Trp Lys
1               5

<210>    130
<211>    8
<212>    PRT
<213>    Artificial Sequence

<220>
<223>    AchR binding peptide

<400>    130
Arg Ile Lys Arg Asn Asn Cys Lys
1               5


<210>    131
<211>    8
<212>    PRT
<213>    Artificial Sequence

<220>
<223>    AchR binding peptide


<400>    131
Arg His Lys Arg Met Leu Trp Arg
1               5


<210>    132
<211>    8
<212>    PRT
<213>    Artificial Sequence

<220>
<223>    AchR binding peptide


<400>    132
Lys Val Arg Arg Val Phe Leu Lys
1               5


<210>    133
<211>    8
<212>    PRT
<213>    Artificial Sequence

<220>
<223>    AchR binding peptide


<400>    133
Lys Asp Lys Arg Pro Glu Cys Lys
1               5


<210>    134
<211>    8
<212>    PRT
<213>    Artificial Sequence

<220>
<223>    AchR binding peptide


<400>    134
Lys Thr Arg Arg Ser Ala Cys Arg
1               5


<210>    135

<211>    8
<212>    PRT
<213>    Artificial Sequence

<220>
<223>    AchR binding peptide


<400>    135
Lys Phe Arg Lys Gly Pro Tyr Arg
1               5


<210>    136
<211>    8
<212>    PRT
<213>    Artificial Sequence

<220>
<223>    AchR binding peptide


<400>    136
Lys Phe Lys Lys Ser Ala Pro Arg
1               5


<210>    137
<211>    8
<212>    PRT
<213>    Artificial Sequence

<220>
<223>    AchR binding peptide


<400>    137
Lys Pro Arg Lys Ile Gln Gly Arg
1               5


<210>    138
<211>    8
<212>    PRT
<213>    Artificial Sequence

<220>
<223>    AchR binding peptide


<400>    138
Lys Met Arg Lys Gly Trp Asp Lys
1               5


<210>    139
<211>    8
<212>    PRT
<213>    Artificial Sequence

<220>
<223>    AchR binding peptide

<400>    139
Lys Phe Arg Arg Gln Ser Thr Arg
1               5


<210>    140
<211>    8
<212>    PRT
<213>    Artificial Sequence

<220>
<223>    AchR binding peptide


<400>    140
Lys Asn Arg Arg Ala Asp Cys Arg
1               5


<210>    141
<211>    8
<212>    PRT
<213>    Artificial Sequence

<220>
<223>    AchR binding peptide


<400>    141
Lys Asn Lys Arg Trp Val Phe Lys
1               5


<210>    142
<211>    8
<212>    PRT
<213>    Artificial Sequence

<220>
<223>    AchR binding peptide


<400>    142
Arg Cys Arg Lys Asp Thr Ala Arg
1               5


<210>    143
<211>    8
<212>    PRT
<213>    Artificial Sequence

<220>
<223>    AchR binding peptide


<400>    143
Lys Ala Arg Arg Val Gly Thr Lys
1               5


<210>    144

<211>    8
<212>    PRT
<213>    Artificial Sequence

<220>
<223>    AchR binding peptide


<400>    144
Lys Val Lys Lys Ile Tyr Tyr Arg
1               5


<210>    145
<211>    8
<212>    PRT
<213>    Artificial Sequence

<220>
<223>    AchR binding peptide


<400>    145
Lys Ala Lys Arg Asp Tyr Leu Arg
1               5


<210>    146
<211>    8
<212>    PRT
<213>    Artificial Sequence

<220>
<223>    AchR binding peptide


<400>    146
Lys Val Lys Arg Leu Pro Tyr Arg
1               5


<210>    147
<211>    8
<212>    PRT
<213>    Artificial Sequence

<220>
<223>    AchR binding peptide


<400>    147
Arg His Arg Arg Ala Asn Phe Arg
1               5


<210>    148
<211>    8
<212>    PRT
<213>    Artificial Sequence

<220>
<223>    AchR binding peptide

<400>    148
Lys Pro Lys Lys Gly Gly Trp Lys
1               5

<210>    149
<211>    8
<212>    PRT
<213>    Artificial Sequence

<220>
<223>    AchR binding peptide

<400>    149
Lys Glu Lys Arg Leu Tyr Ala Arg
1               5

<210>    150
<211>    8
<212>    PRT
<213>    Artificial Sequence

<220>
<223>    AchR binding peptide

<400>    150
Arg Glu Lys Lys Gln Glu Val Lys
1               5

<210>    151
<211>    8
<212>    PRT
<213>    Artificial Sequence

<220>
<223>    AchR binding peptide

<400>    151
Lys Ser Arg Arg Ala Val Phe Arg
1               5

<210>    152
<211>    8
<212>    PRT
<213>    Artificial Sequence

<220>
<223>    AchR binding peptide

<400>    152
Arg His Arg Lys Tyr Val Asp Lys
1               5

<210>    153

<211>    8
<212>    PRT
<213>    Artificial Sequence

<220>
<223>    AchR binding peptide


<400>    153
Arg His Lys Arg Trp Ser Gly Arg
1               5


<210>    154
<211>    8
<212>    PRT
<213>    Artificial Sequence

<220>
<223>    AchR binding peptide


<400>    154
Arg Asp Arg Lys Phe Val Gln Lys
1               5


<210>    155
<211>    8
<212>    PRT
<213>    Artificial Sequence

<220>
<223>    AchR binding peptide


<400>    155
Arg Tyr Arg Arg Trp Phe Tyr Arg
1               5


<210>    156
<211>    8
<212>    PRT
<213>    Artificial Sequence

<220>
<223>    AchR binding peptide


<400>    156
Arg Gln Arg Arg Met Thr Pro Arg
1               5


<210>    157
<211>    8
<212>    PRT
<213>    Artificial Sequence

<220>
<223>    AchR binding peptide

<400>    157
Arg Asn Lys Arg Met Asp Gly Lys
1               5

<210>    158
<211>    8
<212>    PRT
<213>    Artificial Sequence

<220>
<223>    AchR binding peptide

<400>    158
Arg Pro Lys Lys His Gln Glu Arg
1               5

<210>    159
<211>    8
<212>    PRT
<213>    Artificial Sequence

<220>
<223>    AchR binding peptide

<400>    159
Lys Lys Arg Lys Ser Leu Leu Arg
1               5

<210>    160
<211>    8
<212>    PRT
<213>    Artificial Sequence

<220>
<223>    AchR binding peptide

<400>    160
Lys Ile Lys Arg Asn Val Phe Lys
1               5

<210>    161
<211>    8
<212>    PRT
<213>    Artificial Sequence

<220>
<223>    AchR binding peptide

<400>    161
Lys Tyr Lys Arg Glu Glu Tyr Arg
1               5

<210>    162

<211>    8
<212>    PRT
<213>    Artificial Sequence

<220>
<223>    AchR binding peptide


<400>    162
Lys Ile Arg Arg Val Thr Asn Lys
1               5


<210>    163
<211>    8
<212>    PRT
<213>    Artificial Sequence

<220>
<223>    AchR binding peptide


<400>    163
Lys Val Lys Arg Val Trp Gly Arg
1               5


<210>    164
<211>    8
<212>    PRT
<213>    Artificial Sequence

<220>
<223>    AchR binding peptide


<400>    164
Arg Ile Lys Arg Asp Tyr Cys Lys
1               5


<210>    165
<211>    8
<212>    PRT
<213>    Artificial Sequence

<220>
<223>    AchR binding peptide


<400>    165
Arg Ile Arg Arg Ala His Asp Arg
1               5


<210>    166
<211>    8
<212>    PRT
<213>    Artificial Sequence

<220>
<223>    AchR binding peptide

<400>    166
Arg Ala Arg Lys Glu Ser His Lys
1               5

<210>    167
<211>    8
<212>    PRT
<213>    Artificial Sequence

<220>
<223>    AchR binding peptide

<400>    167
Arg Tyr Lys Lys Gln Gln Tyr Lys
1               5

<210>    168
<211>    8
<212>    PRT
<213>    Artificial Sequence

<220>
<223>    AchR binding peptide

<400>    168
Lys Ile Lys Lys Leu Ser Gln Lys
1               5

<210>    169
<211>    8
<212>    PRT
<213>    Artificial Sequence

<220>
<223>    AchR binding peptide

<400>    169
Lys Leu Lys Arg Ala Met Leu Lys
1               5

<210>    170
<211>    8
<212>    PRT
<213>    Artificial Sequence

<220>
<223>    AchR binding peptide

<400>    170
Lys Ala Arg Lys Asn Ser Ile Arg
1               5

<210>    171

<211>    8
<212>    PRT
<213>    Artificial Sequence

<220>
<223>    AchR binding peptide


<400>    171
Lys Glu Arg Lys Leu Trp Trp Lys
1               5


<210>    172
<211>    8
<212>    PRT
<213>    Artificial Sequence

<220>
<223>    AchR binding peptide


<400>    172
Lys Thr Arg Lys Gln Asp His Arg
1               5


<210>    173
<211>    8
<212>    PRT
<213>    Artificial Sequence

<220>
<223>    AchR binding peptide


<400>    173
Arg Cys Lys Arg Phe Val Gly Lys
1               5


<210>    174
<211>    8
<212>    PRT
<213>    Artificial Sequence

<220>
<223>    AchR binding peptide


<400>    174
Lys Glu Lys Lys Leu Val Trp Lys
1               5


<210>    175
<211>    8
<212>    PRT
<213>    Artificial Sequence

<220>
<223>    AchR binding peptide

<400>    175
Lys Ala Arg Arg Asn Ser Leu Lys
1               5

<210>    176
<211>    8
<212>    PRT
<213>    Artificial Sequence

<220>
<223>    AchR binding peptide

<400>    176
Lys Met Arg Arg Val Ala Pro Arg
1               5

<210>    177
<211>    8
<212>    PRT
<213>    Artificial Sequence

<220>
<223>    AchR binding peptide

<400>    177
Arg Ala Lys Lys Ile Met Phe Arg
1               5

<210>    178
<211>    8
<212>    PRT
<213>    Artificial Sequence

<220>
<223>    AchR binding peptide

<400>    178
Lys Thr Lys Lys Ala Ala Glu Arg
1               5

<210>    179
<211>    8
<212>    PRT
<213>    Artificial Sequence

<220>
<223>    AchR binding peptide

<400>    179
Arg Gly Lys Arg His Trp His Arg
1               5

<210>    180

<211>    8
<212>    PRT
<213>    Artificial Sequence

<220>
<223>    AchR binding peptide


<400>    180
Arg Thr Lys Lys Glu Asn Val Lys
1               5


<210>    181
<211>    8
<212>    PRT
<213>    Artificial Sequence

<220>
<223>    AchR binding peptide


<400>    181
Arg Glu Lys Lys Tyr Ala Tyr Lys
1               5


<210>    182
<211>    8
<212>    PRT
<213>    Artificial Sequence

<220>
<223>    AchR binding peptide


<400>    182
Lys Trp Arg Arg Glu Leu Pro Arg
1               5


<210>    183
<211>    8
<212>    PRT
<213>    Artificial Sequence

<220>
<223>    AchR binding peptide


<400>    183
Lys Ser Arg Arg Met Trp Gly Arg
1               5


<210>    184
<211>    8
<212>    PRT
<213>    Artificial Sequence

<220>
<223>    AchR binding peptide

<400>    184
Lys Cys Lys Lys Asp Asn Asp Lys
1               5

<210>    185
<211>    8
<212>    PRT
<213>    Artificial Sequence

<220>
<223>    AchR binding peptide

<400>    185
Lys Asp Lys Lys Met Pro Gln Arg
1               5

<210>    186
<211>    8
<212>    PRT
<213>    Artificial Sequence

<220>
<223>    AchR binding peptide

<400>    186
Arg His Lys Arg Gln Gln Asp Lys
1               5

<210>    187
<211>    8
<212>    PRT
<213>    Artificial Sequence

<220>
<223>    AchR binding peptide

<400>    187
Arg Leu Lys Lys His Cys Gly Lys
1               5

<210>    188
<211>    8
<212>    PRT
<213>    Artificial Sequence

<220>
<223>    AchR binding peptide

<400>    188
Lys Ala Arg Lys Gln Glu Val Arg
1               5

<210>    189

<211>    8
<212>    PRT
<213>    Artificial Sequence

<220>
<223>    AchR binding peptide


<400>    189
Lys Met Arg Lys Phe Tyr Ser Lys
1               5


<210>    190
<211>    8
<212>    PRT
<213>    Artificial Sequence

<220>
<223>    AchR binding peptide


<400>    190
Lys Met Arg Arg Trp Trp Leu Lys
1               5


<210>    191
<211>    8
<212>    PRT
<213>    Artificial Sequence

<220>
<223>    AchR binding peptide


<400>    191
Lys Gln Lys Arg Gln Trp Ala Arg
1               5


<210>    192
<211>    8
<212>    PRT
<213>    Artificial Sequence

<220>
<223>    AchR binding peptide


<400>    192
Arg Ser Arg Arg Gly Ile Gly Lys
1               5


<210>    193
<211>    8
<212>    PRT
<213>    Artificial Sequence

<220>
<223>    AchR binding peptide

<400>    193
Lys Asp Lys Lys Thr Pro Cys Lys
1               5

<210>    194
<211>    8
<212>    PRT
<213>    Artificial Sequence

<220>
<223>    AchR binding peptide

<400>    194
Arg Ile Arg Lys Ile Thr Trp Arg
1               5

<210>    195
<211>    8
<212>    PRT
<213>    Artificial Sequence

<220>
<223>    AchR binding peptide

<400>    195
Arg His Lys Arg Trp Glu Val Arg
1               5

<210>    196
<211>    8
<212>    PRT
<213>    Artificial Sequence

<220>
<223>    AchR binding peptide

<400>    196
Arg Phe Arg Arg Asn Phe His Lys
1               5

<210>    197
<211>    8
<212>    PRT
<213>    Artificial Sequence

<220>
<223>    AchR binding peptide

<400>    197
Lys Trp Lys Arg Phe Ser Gln Arg
1               5

<210>    198

<211>    8
<212>    PRT
<213>    Artificial Sequence

<220>
<223>    AchR binding peptide


<400>    198
Lys Tyr Lys Lys Ser Phe Thr Lys
1               5


<210>    199
<211>    8
<212>    PRT
<213>    Artificial Sequence

<220>
<223>    AchR binding peptide


<400>    199
Lys Thr Arg Lys Ile Val Met Lys
1               5


<210>    200
<211>    8
<212>    PRT
<213>    Artificial Sequence

<220>
<223>    AchR binding peptide


<400>    200
Arg Thr Lys Lys Ala Tyr Val Lys
1               5


<210>    201
<211>    11
<212>    PRT
<213>    Artificial Sequence

<220>
<223>    AchR binding peptide


<400>    201
Glu Asp Tyr Lys Tyr Arg Arg Gln Asn Tyr Lys
1               5                   10


<210>    202
<211>    11
<212>    PRT
<213>    Artificial Sequence

<220>
<223>    AchR binding peptide

<400>    202
Gly Ile Trp Lys Phe Arg Arg Asn Gln Cys Lys
1               5                   10

<210>    203
<211>    11
<212>    PRT
<213>    Artificial Sequence

<220>
<223>    AchR binding peptide

<400>    203
Ser Pro Val Arg Trp Lys Arg Leu Cys Leu Arg
1               5                   10

<210>    204
<211>    11
<212>    PRT
<213>    Artificial Sequence

<220>
<223>    AchR binding peptide

<400>    204
Glu Cys Tyr Arg Asn Arg Lys Ala Tyr Cys Arg
1               5                   10

<210>    205
<211>    11
<212>    PRT
<213>    Artificial Sequence

<220>
<223>    AchR binding peptide

<400>    205
Thr Gln Gln Arg Leu Lys Arg Val Met Glu Lys
1               5                   10

<210>    206
<211>    11
<212>    PRT
<213>    Artificial Sequence

<220>
<223>    AchR binding peptide

<400>    206
Pro Glu Leu Lys Cys Lys Arg Met Ile Gly Arg
1               5                   10

<210>    207

<211>    11
<212>    PRT
<213>    Artificial Sequence

<220>
<223>    AchR binding peptide


<400>    207
Trp Gln Met Lys Thr Lys Lys Glu Ile Trp Lys
1               5                   10


<210>    208
<211>    11
<212>    PRT
<213>    Artificial Sequence

<220>
<223>    AchR binding peptide


<400>    208
Phe Gly His Arg Gly Lys Lys Glu Trp Ala Arg
1               5                   10


<210>    209
<211>    11
<212>    PRT
<213>    Artificial Sequence

<220>
<223>    AchR binding peptide


<400>    209
Ala Thr Cys Lys Pro Lys Arg Pro Trp Tyr Lys
1               5                   10


<210>    210
<211>    11
<212>    PRT
<213>    Artificial Sequence

<220>
<223>    AchR binding peptide


<400>    210
Thr Ser Gln Lys Val Arg Lys Met Glu Ala Lys
1               5                   10


<210>    211
<211>    11
<212>    PRT
<213>    Artificial Sequence

<220>
<223>    AchR binding peptide

<400>    211
Gly Ile Pro Lys Tyr Arg Arg Gly Cys Ser Arg
1               5                   10

<210>    212
<211>    11
<212>    PRT
<213>    Artificial Sequence

<220>
<223>    AchR binding peptide

<400>    212
Ala Tyr Ala Lys Ala Arg Arg Trp Gly Gln Lys
1               5                   10

<210>    213
<211>    11
<212>    PRT
<213>    Artificial Sequence

<220>
<223>    AchR binding peptide

<400>    213
Gly Gly Leu Lys Ser Lys Arg Leu Thr Thr Arg
1               5                   10

<210>    214
<211>    11
<212>    PRT
<213>    Artificial Sequence

<220>
<223>    AchR binding peptide

<400>    214
Gly Met Trp Lys Val Arg Lys Thr Val Phe Arg
1               5                   10

<210>    215
<211>    11
<212>    PRT
<213>    Artificial Sequence

<220>
<223>    AchR binding peptide

<400>    215
His Thr Thr Arg Gly Lys Arg Cys Asp Pro Arg
1               5                   10

<210>    216

<211>    11
<212>    PRT
<213>    Artificial Sequence

<220>
<223>    AchR binding peptide


<400>    216
Val Asp Val Arg Asn Lys Lys Ser Asn Asn Arg
1               5                   10


<210>    217
<211>    11
<212>    PRT
<213>    Artificial Sequence

<220>
<223>    AchR binding peptide


<400>    217
Gly Leu Asn Arg Trp Arg Arg Cys His His Lys
1               5                   10


<210>    218
<211>    11
<212>    PRT
<213>    Artificial Sequence

<220>
<223>    AchR binding peptide


<400>    218
Leu Gln Ser Arg Ser Lys Lys Tyr Ser Val Lys
1               5                   10


<210>    219
<211>    11
<212>    PRT
<213>    Artificial Sequence

<220>
<223>    AchR binding peptide


<400>    219
Gly Trp Asn Arg Ala Lys Arg Glu Glu Ser Lys
1               5                   10


<210>    220
<211>    11
<212>    PRT
<213>    Artificial Sequence

<220>
<223>    AchR binding peptide

<400>    220
Ser Trp Gln Lys Cys Lys Lys Ala Glu Val Arg
1               5                   10

<210>    221
<211>    11
<212>    PRT
<213>    Artificial Sequence

<220>
<223>    AchR binding peptide

<400>    221
Met Val Met Lys Trp Lys Arg Trp Asn Gln Arg
1               5                   10

<210>    222
<211>    11
<212>    PRT
<213>    Artificial Sequence

<220>
<223>    AchR binding peptide

<400>    222
Met Met Met Arg His Lys Arg Cys Gln Tyr Arg
1               5                   10

<210>    223
<211>    11
<212>    PRT
<213>    Artificial Sequence

<220>
<223>    AchR binding peptide

<400>    223
Gln Thr Tyr Arg Leu Lys Lys Pro Leu Gln Lys
1               5                   10

<210>    224
<211>    11
<212>    PRT
<213>    Artificial Sequence

<220>
<223>    AchR binding peptide

<400>    224
Ser Phe Trp Arg Glu Arg Lys Asn Gly Phe Arg
1               5                   10

<210>    225

<211>     11
<212>     PRT
<213>     Artificial Sequence

<220>
<223>     AchR binding peptide


<400>     225
Arg Leu Glu Arg Pro Arg Arg Glu Leu Thr Lys
1               5                   10


<210>     226
<211>     11
<212>     PRT
<213>     Artificial Sequence

<220>
<223>     AchR binding peptide


<400>     226
Ala Pro Trp Arg Leu Lys Arg Ala Pro Gln Arg
1               5                   10


<210>     227
<211>     11
<212>     PRT
<213>     Artificial Sequence

<220>
<223>     AchR binding peptide


<400>     227
Gln Gly Glu Lys Tyr Arg Arg Thr Glu Ser Lys
1               5                   10


<210>     228
<211>     11
<212>     PRT
<213>     Artificial Sequence

<220>
<223>     AchR binding peptide


<400>     228
Cys Ile Trp Lys Ser Lys Arg Ser Pro Ala Lys
1               5                   10


<210>     229
<211>     11
<212>     PRT
<213>     Artificial Sequence

<220>
<223>     AchR binding peptide

<400>    229
Ala Ile Leu Lys Gly Lys Arg Ile Pro Asn Lys
1               5                   10

<210>    230
<211>    11
<212>    PRT
<213>    Artificial Sequence

<220>
<223>    AchR binding peptide

<400>    230
Pro Thr Leu Lys Trp Arg Lys Pro Val Leu Arg
1               5                   10

<210>    231
<211>    11
<212>    PRT
<213>    Artificial Sequence

<220>
<223>    AchR binding peptide

<400>    231
Asn Asn Ser Arg Ser Lys Arg Phe Met Asn Arg
1               5                   10

<210>    232
<211>    11
<212>    PRT
<213>    Artificial Sequence

<220>
<223>    AchR binding peptide

<400>    232
Leu Tyr Met Arg Cys Lys Lys Gln Ala Pro Arg
1               5                   10

<210>    233
<211>    11
<212>    PRT
<213>    Artificial Sequence

<220>
<223>    AchR binding peptide

<400>    233
Ile Asn Tyr Arg Cys Arg Lys Trp Val Asp Lys
1               5                   10

<210>    234

<211>    11
<212>    PRT
<213>    Artificial Sequence

<220>
<223>    AchR binding peptide


<400>    234
Val Met Asp Arg Asp Arg Lys Trp Trp Trp Arg
1               5                   10


<210>    235
<211>    11
<212>    PRT
<213>    Artificial Sequence

<220>
<223>    AchR binding peptide


<400>    235
Asn Phe Thr Lys Leu Arg Lys Pro Gly Pro Arg
1               5                   10


<210>    236
<211>    11
<212>    PRT
<213>    Artificial Sequence

<220>
<223>    AchR binding peptide


<400>    236
Phe Ile Pro Lys Met Arg Lys Cys Ser Pro Arg
1               5                   10


<210>    237
<211>    11
<212>    PRT
<213>    Artificial Sequence

<220>
<223>    AchR binding peptide


<400>    237
Thr Leu Trp Lys Val Arg Lys Ala Tyr Ser Arg
1               5                   10


<210>    238
<211>    11
<212>    PRT
<213>    Artificial Sequence

<220>
<223>    AchR binding peptide

<400>    238
Glu Ser Glu Lys Ile Lys Arg Leu Ser Thr Arg
1               5                   10

<210>    239
<211>    11
<212>    PRT
<213>    Artificial Sequence

<220>
<223>    AchR binding peptide

<400>    239
Leu Met Thr Lys Asn Arg Arg Asn Ser Phe Arg
1               5                   10

<210>    240
<211>    11
<212>    PRT
<213>    Artificial Sequence

<220>
<223>    AchR binding peptide

<400>    240
Pro Glu Val Lys Val Arg Arg His Ser Met Arg
1               5                   10

<210>    241
<211>    11
<212>    PRT
<213>    Artificial Sequence

<220>
<223>    AchR binding peptide

<400>    241
Ala Glu Val Lys Asp Lys Lys Ala Tyr Tyr Lys
1               5                   10

<210>    242
<211>    11
<212>    PRT
<213>    Artificial Sequence

<220>
<223>    AchR binding peptide

<400>    242
Tyr Tyr Ala Lys Ser Lys Lys Tyr Met Val Arg
1               5                   10

<210>    243

<211>    11
<212>    PRT
<213>    Artificial Sequence

<220>
<223>    AchR binding peptide


<400>    243
Leu Met Asp Arg Thr Arg Arg Asp Met Tyr Lys
1               5                   10


<210>    244
<211>    11
<212>    PRT
<213>    Artificial Sequence

<220>
<223>    AchR binding peptide


<400>    244
Asn Met Gly Arg His Lys Arg Pro Phe Leu Lys
1               5                   10


<210>    245
<211>    11
<212>    PRT
<213>    Artificial Sequence

<220>
<223>    AchR binding peptide


<400>    245
Trp Ile Tyr Arg Gly Arg Lys Asp Val Ala Lys
1               5                   10


<210>    246
<211>    11
<212>    PRT
<213>    Artificial Sequence

<220>
<223>    AchR binding peptide


<400>    246
Cys Tyr Trp Lys Ala Lys Arg Tyr Pro Met Arg
1               5                   10


<210>    247
<211>    11
<212>    PRT
<213>    Artificial Sequence

<220>
<223>    AchR binding peptide

<400>    247
Ile Glu Asp Lys Gly Arg Arg Ile Asn Pro Arg
1               5                   10

<210>    248
<211>    11
<212>    PRT
<213>    Artificial Sequence

<220>
<223>    AchR binding peptide

<400>    248
Val Ser Thr Lys Ile Lys Lys Glu Pro Gln Arg
1               5                   10

<210>    249
<211>    11
<212>    PRT
<213>    Artificial Sequence

<220>
<223>    AchR binding peptide

<400>    249
Tyr Pro Phe Lys Ala Lys Arg Pro Ala Glu Lys
1               5                   10

<210>    250
<211>    11
<212>    PRT
<213>    Artificial Sequence

<220>
<223>    AchR binding peptide

<400>    250
Ser Ala Asp Arg Asn Lys Arg His Met Thr Arg
1               5                   10

<210>    251
<211>    11
<212>    PRT
<213>    Artificial Sequence

<220>
<223>    AchR binding peptide

<400>    251
Ser Leu Tyr Arg Gln Lys Arg His Asp Tyr Lys
1               5                   10

<210>    252

<211>    11
<212>    PRT
<213>    Artificial Sequence

<220>
<223>    AchR binding peptide


<400>    252
Leu Pro Ser Arg Pro Lys Lys Pro Val Pro Lys
1               5                   10


<210>    253
<211>    11
<212>    PRT
<213>    Artificial Sequence

<220>
<223>    AchR binding peptide


<400>    253
Pro Ala Trp Arg Cys Lys Arg Cys Gln Pro Lys
1               5                   10


<210>    254
<211>    11
<212>    PRT
<213>    Artificial Sequence

<220>
<223>    AchR binding peptide


<400>    254
His His Trp Arg Phe Lys Arg Glu Met Pro Arg
1               5                   10


<210>    255
<211>    11
<212>    PRT
<213>    Artificial Sequence

<220>
<223>    AchR binding peptide


<400>    255
Pro Ala Cys Arg Ser Lys Arg Asp Trp Gln Lys
1               5                   10


<210>    256
<211>    11
<212>    PRT
<213>    Artificial Sequence

<220>
<223>    AchR binding peptide

<400>    256
Phe Ile Cys Lys Ser Arg Lys Phe Tyr Gly Lys
1               5                   10

<210>    257
<211>    11
<212>    PRT
<213>    Artificial Sequence

<220>
<223>    AchR binding peptide

<400>    257
Leu Glu His Lys Glu Arg Lys Asp Asp Phe Arg
1               5                   10

<210>    258
<211>    11
<212>    PRT
<213>    Artificial Sequence

<220>
<223>    AchR binding peptide

<400>    258
Ala Asn Pro Lys Asn Arg Lys Asp Asn Leu Arg
1               5                   10

<210>    259
<211>    11
<212>    PRT
<213>    Artificial Sequence

<220>
<223>    AchR binding peptide

<400>    259
Phe Gly Val Lys Tyr Arg Arg Val Ile Cys Arg
1               5                   10

<210>    260
<211>    11
<212>    PRT
<213>    Artificial Sequence

<220>
<223>    AchR binding peptide

<400>    260
His Ala Asp Lys Phe Arg Arg Phe Asn Met Arg
1               5                   10

<210>    261

<211>    11
<212>    PRT
<213>    Artificial Sequence

<220>
<223>    AchR binding peptide


<400>    261
Pro Thr Ile Arg Val Arg Lys Ser Asp Asp Arg
1               5                   10


<210>    262
<211>    11
<212>    PRT
<213>    Artificial Sequence

<220>
<223>    AchR binding peptide


<400>    262
Asn Leu His Lys Pro Lys Arg Asp Leu Pro Lys
1               5                   10


<210>    263
<211>    11
<212>    PRT
<213>    Artificial Sequence

<220>
<223>    AchR binding peptide


<400>    263
Ile Asp Gly Lys Trp Arg Lys Ile Cys Thr Arg
1               5                   10


<210>    264
<211>    11
<212>    PRT
<213>    Artificial Sequence

<220>
<223>    AchR binding peptide


<400>    264
Ser Pro Phe Arg Ala Lys Arg Gln Asp Val Arg
1               5                   10


<210>    265
<211>    11
<212>    PRT
<213>    Artificial Sequence

<220>
<223>    AchR binding peptide

<400>    265
Pro Leu Trp Lys Thr Arg Lys Ile Glu Pro Arg
1               5                   10

<210>    266
<211>    11
<212>    PRT
<213>    Artificial Sequence

<220>
<223>    AchR binding peptide

<400>    266
Pro Asn Tyr Lys Trp Arg Lys Ser Arg Arg Arg
1               5                   10

<210>    267
<211>    11
<212>    PRT
<213>    Artificial Sequence

<220>
<223>    AchR binding peptide

<400>    267
Lys Thr Gly Arg Ser Lys Arg His Arg Trp Arg
1               5                   10

<210>    268
<211>    11
<212>    PRT
<213>    Artificial Sequence

<220>
<223>    AchR binding peptide

<400>    268
Gly Pro Asp Lys Ser Arg Arg Asn Leu His Arg
1               5                   10

<210>    269
<211>    11
<212>    PRT
<213>    Artificial Sequence

<220>
<223>    AchR binding peptide

<400>    269
Trp Cys Cys Lys Thr Lys Arg Ala Val Met Lys
1               5                   10

<210>    270

<211>    11
<212>    PRT
<213>    Artificial Sequence

<220>
<223>    AchR binding peptide


<400>    270
Gln Leu Leu Lys Met Arg Lys Ala Leu Ser Arg
1               5                   10


<210>    271
<211>    11
<212>    PRT
<213>    Artificial Sequence

<220>
<223>    AchR binding peptide


<400>    271
Pro Tyr Leu Arg Ser Lys Arg Phe Pro Pro Arg
1               5                   10


<210>    272
<211>    11
<212>    PRT
<213>    Artificial Sequence

<220>
<223>    AchR binding peptide


<400>    272
Ala Pro His Lys Trp Arg Lys Gln Glu Gln Arg
1               5                   10


<210>    273
<211>    11
<212>    PRT
<213>    Artificial Sequence

<220>
<223>    AchR binding peptide


<400>    273
Pro Pro Phe Lys Phe Arg Lys Pro Leu Gly Arg
1               5                   10


<210>    274
<211>    11
<212>    PRT
<213>    Artificial Sequence

<220>
<223>    AchR binding peptide

<400>    274
Cys Ile Met Arg Val Lys Arg Met Trp Trp Lys
1               5                   10

<210>    275
<211>    11
<212>    PRT
<213>    Artificial Sequence

<220>
<223>    AchR binding peptide

<400>    275
Tyr Ile Leu Arg Asp Lys Lys Val Pro Leu Lys
1               5                   10

<210>    276
<211>    11
<212>    PRT
<213>    Artificial Sequence

<220>
<223>    AchR binding peptide

<400>    276
Pro Trp Val Arg Ile Arg Lys Met Ala Ser Arg
1               5                   10

<210>    277
<211>    11
<212>    PRT
<213>    Artificial Sequence

<220>
<223>    AchR binding peptide

<400>    277
Val Ala Asp Arg Gln Lys Lys Thr Ala Pro Lys
1               5                   10

<210>    278
<211>    11
<212>    PRT
<213>    Artificial Sequence

<220>
<223>    AchR binding peptide

<400>    278
His His Asn Arg Met Lys Lys Gln Tyr His Arg
1               5                   10

<210>    279

<211>    11
<212>    PRT
<213>    Artificial Sequence

<220>
<223>    AchR binding peptide


<400>    279
Glu Gly Ala Lys Tyr Arg Arg Asp Gly Trp Arg
1               5                   10


<210>    280
<211>    11
<212>    PRT
<213>    Artificial Sequence

<220>
<223>    AchR binding peptide


<400>    280
Phe Trp Asp Arg Val Lys Arg Asn Pro Ser Lys
1               5                   10


<210>    281
<211>    11
<212>    PRT
<213>    Artificial Sequence

<220>
<223>    AchR binding peptide


<400>    281
Thr Met Trp Arg Gln Arg Lys Met Ser Cys Lys
1               5                   10


<210>    282
<211>    11
<212>    PRT
<213>    Artificial Sequence

<220>
<223>    AchR binding peptide


<400>    282
Pro Asp Thr Arg Trp Lys Arg Val Leu Phe Lys
1               5                   10


<210>    283
<211>    11
<212>    PRT
<213>    Artificial Sequence

<220>
<223>    AchR binding peptide

<400>    283
His Gln Gln Lys Cys Lys Lys Thr Thr Thr Lys
1               5                   10

<210>    284
<211>    11
<212>    PRT
<213>    Artificial Sequence

<220>
<223>    AchR binding peptide

<400>    284
Pro Trp His Lys Gly Lys Arg Asp Phe Asp Arg
1               5                   10

<210>    285
<211>    11
<212>    PRT
<213>    Artificial Sequence

<220>
<223>    AchR binding peptide

<400>    285
Val Phe Val Lys Trp Arg Arg Gln Met Met Arg
1               5                   10

<210>    286
<211>    11
<212>    PRT
<213>    Artificial Sequence

<220>
<223>    AchR binding peptide

<400>    286
Ser Gln Trp Lys Ile Arg Lys Arg Leu Ile Arg
1               5                   10

<210>    287
<211>    11
<212>    PRT
<213>    Artificial Sequence

<220>
<223>    AchR binding peptide

<400>    287
Glu Trp His Lys Val Arg Arg Val Tyr Ala Lys
1               5                   10

<210>    288

<211>    11
<212>    PRT
<213>    Artificial Sequence

<220>
<223>    AchR binding peptide


<400>    288
Asn Ala Met Arg Thr Lys Arg Met Ser Phe Lys
1               5                   10


<210>    289
<211>    11
<212>    PRT
<213>    Artificial Sequence

<220>
<223>    AchR binding peptide


<400>    289
Pro Pro Phe Lys Phe Arg Lys Pro Leu Gly Arg
1               5                   10


<210>    290
<211>    11
<212>    PRT
<213>    Artificial Sequence

<220>
<223>    AchR binding peptide


<400>    290
Thr Ser Val Lys Lys Arg Lys Gln Arg Leu Arg
1               5                   10


<210>    291
<211>    11
<212>    PRT
<213>    Artificial Sequence

<220>
<223>    AchR binding peptide


<400>    291
Pro Ile Asp Lys Phe Lys Arg Gly Met Val Arg
1               5                   10


<210>    292
<211>    11
<212>    PRT
<213>    Artificial Sequence

<220>
<223>    AchR binding peptide

<400>    292
Phe Val Gly Arg Trp Lys Arg Glu Tyr Ala Lys
1               5                   10

<210>    293
<211>    11
<212>    PRT
<213>    Artificial Sequence

<220>
<223>    AchR binding peptide

<400>    293
Phe Asn Pro Lys Met Arg Lys Val Cys Leu Arg
1               5                   10

<210>    294
<211>    11
<212>    PRT
<213>    Artificial Sequence

<220>
<223>    AchR binding peptide

<400>    294
Asn Pro Gly Lys Thr Lys Arg Trp Leu Gln Arg
1               5                   10

<210>    295
<211>    11
<212>    PRT
<213>    Artificial Sequence

<220>
<223>    AchR binding peptide

<400>    295
Ile Met Asp Lys Arg Arg Lys Pro Gly Cys Arg
1               5                   10

<210>    296
<211>    11
<212>    PRT
<213>    Artificial Sequence

<220>
<223>    AchR binding peptide

<400>    296
Ile Ser Asp Lys Ala Lys Arg Gln His Phe Lys
1               5                   10

<210>    297

<211>    11
<212>    PRT
<213>    Artificial Sequence

<220>
<223>    AchR binding peptide


<400>    297
Asp Val Asn Lys Tyr Arg Lys His Ser His Lys
1               5                   10


<210>    298
<211>    11
<212>    PRT
<213>    Artificial Sequence

<220>
<223>    AchR binding peptide


<400>    298
Leu Met Leu Arg Gly Lys Arg Leu Thr Thr Lys
1               5                   10


<210>    299
<211>    11
<212>    PRT
<213>    Artificial Sequence

<220>
<223>    AchR binding peptide


<400>    299
Cys Ser Leu Arg Ala Arg Lys Glu Glu Trp Arg
1               5                   10


<210>    300
<211>    11
<212>    PRT
<213>    Artificial Sequence

<220>
<223>    AchR binding peptide


<400>    300
Met Asn Tyr Arg Cys Lys Arg Val Gln Glu Arg
1               5                   10


<210>    301
<211>    11
<212>    PRT
<213>    Artificial Sequence

<220>
<223>    AchR binding peptide

<400>    301
Asn Gly Val Arg Glu Arg Lys Trp Gln Ser Lys
1               5                   10

<210>    302
<211>    11
<212>    PRT
<213>    Artificial Sequence

<220>
<223>    AchR binding peptide

<400>    302
Tyr Thr Ser Arg Cys Lys Lys Gln Pro Arg Lys
1               5                   10

<210>    303
<211>    11
<212>    PRT
<213>    Artificial Sequence

<220>
<223>    AchR binding peptide

<400>    303
Gln Tyr Asn Lys Gly Arg Lys Ile His Val Arg
1               5                   10

<210>    304
<211>    11
<212>    PRT
<213>    Artificial Sequence

<220>
<223>    AchR binding peptide

<400>    304
Pro Glu Asp Arg Gln Arg Lys Thr Trp Phe Lys
1               5                   10

<210>    305
<211>    11
<212>    PRT
<213>    Artificial Sequence

<220>
<223>    AchR binding peptide

<400>    305
Ile Glu Met Lys Pro Arg Lys Phe Gly Val Lys
1               5                   10

<210>    306

<211>    11
<212>    PRT
<213>    Artificial Sequence

<220>
<223>    AchR binding peptide


<400>    306
Gln Arg Trp Lys Trp Arg Lys Ser Leu Ala Arg
1               5                   10


<210>    307
<211>    11
<212>    PRT
<213>    Artificial Sequence

<220>
<223>    AchR binding peptide


<400>    307
Val Lys His Lys Glu Arg Lys Cys Gln Arg Arg
1               5                   10


<210>    308
<211>    11
<212>    PRT
<213>    Artificial Sequence

<220>
<223>    AchR binding peptide


<400>    308
Gln Gln Asp Arg Pro Lys Arg Asp Ile Pro Lys
1               5                   10


<210>    309
<211>    11
<212>    PRT
<213>    Artificial Sequence

<220>
<223>    AchR binding peptide


<400>    309
Tyr Pro Thr Lys Gly Lys Arg Cys Met Ile Arg
1               5                   10


<210>    310
<211>    11
<212>    PRT
<213>    Artificial Sequence

<220>
<223>    AchR binding peptide

<400>    310
Arg Tyr Ala Lys His Arg Lys Arg Gln Thr Arg
1               5                   10

<210>    311
<211>    11
<212>    PRT
<213>    Artificial Sequence

<220>
<223>    AchR binding peptide

<400>    311
Gly Tyr Phe Arg Pro Arg Lys Glu Thr Cys Lys
1               5                   10

<210>    312
<211>    11
<212>    PRT
<213>    Artificial Sequence

<220>
<223>    AchR binding peptide

<400>    312
Cys Phe Phe Lys Met Arg Arg Cys Asn Thr Lys
1               5                   10

<210>    313
<211>    11
<212>    PRT
<213>    Artificial Sequence

<220>
<223>    AchR binding peptide

<400>    313
Gln Asn Asp Lys Asp Arg Lys Leu Ser His Arg
1               5                   10

<210>    314
<211>    11
<212>    PRT
<213>    Artificial Sequence

<220>
<223>    AchR binding peptide

<400>    314
Gln Val Thr Lys Ser Lys Arg Val Ala Phe Lys
1               5                   10

<210>    315

<211>    11
<212>    PRT
<213>    Artificial Sequence

<220>
<223>    AchR binding peptide


<400>    315
Val Arg Ala Lys His Arg Lys Ser Ser Leu Arg
1               5                   10


<210>    316
<211>    11
<212>    PRT
<213>    Artificial Sequence

<220>
<223>    AchR binding peptide


<400>    316
Ser His Ser Arg Gln Arg Lys Thr Pro Leu Arg
1               5                   10


<210>    317
<211>    11
<212>    PRT
<213>    Artificial Sequence

<220>
<223>    AchR binding peptide


<400>    317
Asp Asn Gly Lys Ile Arg Arg Cys Leu Gly Lys
1               5                   10


<210>    318
<211>    11
<212>    PRT
<213>    Artificial Sequence

<220>
<223>    AchR binding peptide


<400>    318
Phe Leu Arg Arg Leu Lys Lys Val His Trp Lys
1               5                   10


<210>    319
<211>    11
<212>    PRT
<213>    Artificial Sequence

<220>
<223>    AchR binding peptide

<400>    319
Phe Cys Arg Arg Ser Lys Lys Ile Gly Arg Arg
1               5                   10

<210>    320
<211>    11
<212>    PRT
<213>    Artificial Sequence

<220>
<223>    AchR binding peptide

<400>    320
Pro Ala Ala Arg Thr Lys Arg Met Tyr Gly Arg
1               5                   10

<210>    321
<211>    11
<212>    PRT
<213>    Artificial Sequence

<220>
<223>    AchR binding peptide

<400>    321
His Glu Thr Lys Pro Lys Lys Asp Gly Leu Arg
1               5                   10

<210>    322
<211>    11
<212>    PRT
<213>    Artificial Sequence

<220>
<223>    AchR binding peptide

<400>    322
Met Gly Asp Arg Pro Arg Lys Trp Asp Ser Arg
1               5                   10

<210>    323
<211>    11
<212>    PRT
<213>    Artificial Sequence

<220>
<223>    AchR binding peptide

<400>    323
His Asp Thr Lys Cys Lys Lys Met Tyr Ala Lys
1               5                   10

<210>    324

<211>    11
<212>    PRT
<213>    Artificial Sequence

<220>
<223>    AchR binding peptide


<400>    324
Asn Val Val Arg Gly Arg Arg Leu Glu Cys Arg
1               5                   10


<210>    325
<211>    11
<212>    PRT
<213>    Artificial Sequence

<220>
<223>    AchR binding peptide


<400>    325
Pro Ala Asp Lys Gly Arg Arg Glu Val Met Lys
1               5                   10


<210>    326
<211>    11
<212>    PRT
<213>    Artificial Sequence

<220>
<223>    AchR binding peptide


<400>    326
Ala Met Met Lys Tyr Lys Lys Glu Phe Pro Lys
1               5                   10


<210>    327
<211>    11
<212>    PRT
<213>    Artificial Sequence

<220>
<223>    AchR binding peptide


<400>    327
Tyr Ile Ile Arg Trp Lys Arg Gln Met Thr Arg
1               5                   10


<210>    328
<211>    11
<212>    PRT
<213>    Artificial Sequence

<220>
<223>    AchR binding peptide

<400>    328
Ser Pro Trp Arg Ile Arg Arg Gln Asn Ile Arg
1               5                   10

<210>    329
<211>    11
<212>    PRT
<213>    Artificial Sequence

<220>
<223>    AchR binding peptide

<400>    329
Thr Cys Ile Lys Tyr Arg Arg Ala His Thr Lys
1               5                   10

<210>    330
<211>    11
<212>    PRT
<213>    Artificial Sequence

<220>
<223>    AchR binding peptide

<400>    330
Gly Tyr Asp Arg Ala Arg Lys Gly Thr Leu Arg
1               5                   10

<210>    331
<211>    11
<212>    PRT
<213>    Artificial Sequence

<220>
<223>    AchR binding peptide

<400>    331
Met Thr Leu Lys His Arg Arg Val Tyr Ile Lys
1               5                   10

<210>    332
<211>    11
<212>    PRT
<213>    Artificial Sequence

<220>
<223>    AchR binding peptide

<400>    332
Leu Phe Thr Arg Ile Lys Arg Leu Val Cys Lys
1               5                   10

<210>    333

<211>    11
<212>    PRT
<213>    Artificial Sequence

<220>
<223>    AchR binding peptide


<400>    333
Asp Phe Ser Arg Asp Arg Arg Cys Leu Ser Lys
1               5                   10


<210>    334
<211>    11
<212>    PRT
<213>    Artificial Sequence

<220>
<223>    AchR binding peptide


<400>    334
Val Trp Asn Lys Val Lys Arg Trp Leu Glu Arg
1               5                   10


<210>    335
<211>    11
<212>    PRT
<213>    Artificial Sequence

<220>
<223>    AchR binding peptide


<400>    335
Phe Pro Gly Lys Asn Arg Lys Tyr Cys Ser Arg
1               5                   10


<210>    336
<211>    11
<212>    PRT
<213>    Artificial Sequence

<220>
<223>    AchR binding peptide


<400>    336
Tyr Thr Ser Lys Asn Lys Arg Gly Cys Pro Arg
1               5                   10


<210>    337
<211>    11
<212>    PRT
<213>    Artificial Sequence

<220>
<223>    AchR binding peptide

<400>    337
Cys Ala Cys Lys Gln Arg Arg Ala Thr Ser Arg
1               5                   10

<210>    338
<211>    11
<212>    PRT
<213>    Artificial Sequence

<220>
<223>    AchR binding peptide

<400>    338
Ile Met Glu Arg Gln Arg Lys Ser Gln His Arg
1               5                   10

<210>    339
<211>    11
<212>    PRT
<213>    Artificial Sequence

<220>
<223>    AchR binding peptide

<400>    339
Ser Val Leu Arg Cys Arg Lys Cys Ser Met Lys
1               5                   10

<210>    340
<211>    11
<212>    PRT
<213>    Artificial Sequence

<220>
<223>    AchR binding peptide

<400>    340
Tyr Pro Gln Lys Leu Arg Arg Thr Ala Leu Lys
1               5                   10

<210>    341
<211>    11
<212>    PRT
<213>    Artificial Sequence

<220>
<223>    AchR binding peptide

<400>    341
Ser Ser His Lys Gly Lys Arg Ala Gln Ser Lys
1               5                   10

<210>    342

<211>    11
<212>    PRT
<213>    Artificial Sequence

<220>
<223>    AchR binding peptide


<400>    342
Asp Asn Pro Arg Phe Arg Lys Thr Ile Leu Lys
1               5                   10


<210>    343
<211>    11
<212>    PRT
<213>    Artificial Sequence

<220>
<223>    AchR binding peptide


<400>    343
Ala Ile Ile Lys Phe Arg Lys Val Gln Trp Lys
1               5                   10


<210>    344
<211>    11
<212>    PRT
<213>    Artificial Sequence

<220>
<223>    AchR binding peptide


<400>    344
Pro Tyr Thr Arg Cys Arg Lys Glu Ile Cys Lys
1               5                   10


<210>    345
<211>    11
<212>    PRT
<213>    Artificial Sequence

<220>
<223>    AchR binding peptide


<400>    345
Met Asn Glu Lys Pro Lys Lys Asn Asp Gln Lys
1               5                   10


<210>    346
<211>    11
<212>    PRT
<213>    Artificial Sequence

<220>
<223>    AchR binding peptide

<400>    346
Leu Ile Gly Lys Phe Lys Lys Pro Phe Tyr Arg
1               5                   10

<210>    347
<211>    11
<212>    PRT
<213>    Artificial Sequence

<220>
<223>    AchR binding peptide

<400>    347
Thr Trp Cys Lys His Lys Lys Leu Asp Met Lys
1               5                   10

<210>    348
<211>    11
<212>    PRT
<213>    Artificial Sequence

<220>
<223>    AchR binding peptide

<400>    348
Asp Ser Asn Lys Val Arg Lys Cys Ser Ser Lys
1               5                   10

<210>    349
<211>    11
<212>    PRT
<213>    Artificial Sequence

<220>
<223>    AchR binding peptide

<400>    349
Leu Pro Met Lys Gln Arg Lys Cys Glu Phe Arg
1               5                   10

<210>    350
<211>    11
<212>    PRT
<213>    Artificial Sequence

<220>
<223>    AchR binding peptide

<400>    350
Glu Asp Val Arg Val Lys Arg Gln Thr Cys Arg
1               5                   10

<210>    351

<211>    11
<212>    PRT
<213>    Artificial Sequence

<220>
<223>    AchR binding peptide


<400>    351
Thr Gln Cys Lys Asp Arg Arg Val Ser Asp Arg
1               5                   10


<210>    352
<211>    11
<212>    PRT
<213>    Artificial Sequence

<220>
<223>    AchR binding peptide


<400>    352
Ile Ala Leu Lys Pro Lys Arg Val Trp Leu Lys
1               5                   10


<210>    353
<211>    11
<212>    PRT
<213>    Artificial Sequence

<220>
<223>    AchR binding peptide


<400>    353
Gly His Gln Arg Gly Lys Arg Glu Gly Ser Arg
1               5                   10


<210>    354
<211>    11
<212>    PRT
<213>    Artificial Sequence

<220>
<223>    AchR binding peptide


<400>    354
Asn Pro Phe Lys Tyr Lys Lys Ile Cys Pro Lys
1               5                   10


<210>    355
<211>    11
<212>    PRT
<213>    Artificial Sequence

<220>
<223>    AchR binding peptide

<400>    355
Asn Glu Ala Arg Ile Lys Lys Cys Asp Val Lys
1               5                   10

<210>    356
<211>    11
<212>    PRT
<213>    Artificial Sequence

<220>
<223>    AchR binding peptide

<400>    356
Tyr Gly Leu Arg Met Arg Lys Trp Tyr Met Lys
1               5                   10

<210>    357
<211>    11
<212>    PRT
<213>    Artificial Sequence

<220>
<223>    AchR binding peptide

<400>    357
Asn Phe Tyr Lys Cys Arg Lys Leu Gln Cys Lys
1               5                   10

<210>    358
<211>    11
<212>    PRT
<213>    Artificial Sequence

<220>
<223>    AchR binding peptide

<400>    358
Met Met Thr Lys Tyr Lys Lys Thr Cys Cys Lys
1               5                   10

<210>    359
<211>    11
<212>    PRT
<213>    Artificial Sequence

<220>
<223>    AchR binding peptide

<400>    359
Cys Asn Gln Lys Thr Lys Lys Ile Ala Glu Lys
1               5                   10

<210>    360

<211>    11
<212>    PRT
<213>    Artificial Sequence

<220>
<223>    AchR binding peptide


<400>    360
Ala Asp Ile Arg Met Lys Lys Trp Tyr Pro Lys
1               5                   10


<210>    361
<211>    11
<212>    PRT
<213>    Artificial Sequence

<220>
<223>    AchR binding peptide


<400>    361
Ala Trp Phe Arg Val Lys Arg Ser Asn Cys Arg
1               5                   10


<210>    362
<211>    11
<212>    PRT
<213>    Artificial Sequence

<220>
<223>    AchR binding peptide


<400>    362
Asn Cys Asp Arg Thr Arg Lys His Trp Ala Arg
1               5                   10


<210>    363
<211>    11
<212>    PRT
<213>    Artificial Sequence

<220>
<223>    AchR binding peptide


<400>    363
Pro His Ala Arg Thr Arg Lys Asn Ile Thr Lys
1               5                   10


<210>    364
<211>    11
<212>    PRT
<213>    Artificial Sequence

<220>
<223>    AchR binding peptide

<400>    364
Val Pro Thr Lys Met Lys Lys Tyr Glu Thr Lys
1               5                   10

<210>    365
<211>    11
<212>    PRT
<213>    Artificial Sequence

<220>
<223>    AchR binding peptide

<400>    365
Ala Tyr Pro Lys Phe Arg Lys Thr Phe Asn Arg
1               5                   10

<210>    366
<211>    11
<212>    PRT
<213>    Artificial Sequence

<220>
<223>    AchR binding peptide

<400>    366
Gln Gln Leu Arg Leu Arg Lys Leu Cys Gly Lys
1               5                   10

<210>    367
<211>    11
<212>    PRT
<213>    Artificial Sequence

<220>
<223>    AchR binding peptide

<400>    367
Met Phe Met Arg Asn Lys Lys Leu Ala Trp Arg
1               5                   10

<210>    368
<211>    11
<212>    PRT
<213>    Artificial Sequence

<220>
<223>    AchR binding peptide

<400>    368
Gly Gly Ala Lys Asn Lys Lys Val Val Ser Arg
1               5                   10

<210>    369

<211>    11
<212>    PRT
<213>    Artificial Sequence

<220>
<223>    AchR binding peptide


<400>    369
His Asp Pro Arg His Lys Lys Thr Pro Thr Lys
1               5                   10


<210>    370
<211>    11
<212>    PRT
<213>    Artificial Sequence

<220>
<223>    AchR binding peptide


<400>    370
Gln Val His Arg Asn Lys Arg Tyr Thr Asp Arg
1               5                   10


<210>    371
<211>    11
<212>    PRT
<213>    Artificial Sequence

<220>
<223>    AchR binding peptide


<400>    371
Tyr Gly Thr Arg Pro Lys Lys Tyr Val Ser Lys
1               5                   10


<210>    372
<211>    11
<212>    PRT
<213>    Artificial Sequence

<220>
<223>    AchR binding peptide


<400>    372
Cys Thr Trp Arg Gly Arg Arg Pro His Asp Lys
1               5                   10


<210>    373
<211>    11
<212>    PRT
<213>    Artificial Sequence

<220>
<223>    AchR binding peptide

<400>    373
Ser Trp Ala Lys Ala Arg Lys Leu Val His Arg
1               5                   10

<210>    374
<211>    11
<212>    PRT
<213>    Artificial Sequence

<220>
<223>    AchR binding peptide

<400>    374
Pro Leu Phe Lys Ser Arg Arg Ala Tyr Val Arg
1               5                   10

<210>    375
<211>    11
<212>    PRT
<213>    Artificial Sequence

<220>
<223>    AchR binding peptide

<400>    375
Cys Val Met Arg Cys Arg Arg Ser Glu Asp Lys
1               5                   10

<210>    376
<211>    11
<212>    PRT
<213>    Artificial Sequence

<220>
<223>    AchR binding peptide

<400>    376
Trp Trp His Lys His Arg Arg Ala Gln Ser Lys
1               5                   10

<210>    377
<211>    11
<212>    PRT
<213>    Artificial Sequence

<220>
<223>    AchR binding peptide

<400>    377
Leu Asn Ser Lys Pro Arg Arg Val Glu Phe Lys
1               5                   10

<210>    378

<211>    11
<212>    PRT
<213>    Artificial Sequence

<220>
<223>    AchR binding peptide


<400>    378
Val Pro Tyr Arg His Arg Arg Met Gln Phe Lys
1               5                   10


<210>    379
<211>    11
<212>    PRT
<213>    Artificial Sequence

<220>
<223>    AchR binding peptide


<400>    379
Gly Ile Ala Lys Ser Lys Arg Asn Ala Gly Arg
1               5                   10


<210>    380
<211>    11
<212>    PRT
<213>    Artificial Sequence

<220>
<223>    AchR binding peptide


<400>    380
Asp Pro Glu Lys Trp Arg Lys Phe Tyr Asp Arg
1               5                   10


<210>    381
<211>    11
<212>    PRT
<213>    Artificial Sequence

<220>
<223>    AchR binding peptide


<400>    381
Pro Gly Ala Arg Cys Arg Lys Gln Asp Val Lys
1               5                   10


<210>    382
<211>    11
<212>    PRT
<213>    Artificial Sequence

<220>
<223>    AchR binding peptide

<400>    382
His Glu Gln Arg Pro Lys Lys Gly Gln Gln Lys
1               5                   10

<210>    383
<211>    11
<212>    PRT
<213>    Artificial Sequence

<220>
<223>    AchR binding peptide

<400>    383
Thr Cys Asp Arg Ala Arg Lys Glu Ser Phe Arg
1               5                   10

<210>    384
<211>    11
<212>    PRT
<213>    Artificial Sequence

<220>
<223>    AchR binding peptide

<400>    384
Met His Gln Arg Glu Arg Arg Asn Phe Val Lys
1               5                   10

<210>    385
<211>    11
<212>    PRT
<213>    Artificial Sequence

<220>
<223>    AchR binding peptide

<400>    385
Phe Ile Thr Arg Phe Arg Lys Met Gly Glu Lys
1               5                   10

<210>    386
<211>    11
<212>    PRT
<213>    Artificial Sequence

<220>
<223>    AchR binding peptide

<400>    386
Pro Asn Trp Lys Val Arg Arg Phe Gly Asp Lys
1               5                   10

<210>    387

<211>    11
<212>    PRT
<213>    Artificial Sequence

<220>
<223>    AchR binding peptide


<400>    387
Thr Ala Ala Lys Trp Lys Lys Ile Ile Met Lys
1               5                   10


<210>    388
<211>    11
<212>    PRT
<213>    Artificial Sequence

<220>
<223>    AchR binding peptide


<400>    388
Cys Leu Trp Arg Leu Arg Lys Asp Asn Gly Arg
1               5                   10


<210>    389
<211>    11
<212>    PRT
<213>    Artificial Sequence

<220>
<223>    AchR binding peptide


<400>    389
Ala His Ile Lys Ser Arg Lys Val Trp Ser Arg
1               5                   10


<210>    390
<211>    11
<212>    PRT
<213>    Artificial Sequence

<220>
<223>    AchR binding peptide


<400>    390
Asn Leu Val Lys Ser Lys Lys Val Glu Glu Lys
1               5                   10


<210>    391
<211>    11
<212>    PRT
<213>    Artificial Sequence

<220>
<223>    AchR binding peptide

<400>    391
Pro Asp Ser Arg Leu Lys Lys His Glu Ala Lys
1               5                   10

<210>    392
<211>    11
<212>    PRT
<213>    Artificial Sequence

<220>
<223>    AchR binding peptide

<400>    392
Glu His Val Lys Leu Lys Arg Leu Asp Phe Arg
1               5                   10

<210>    393
<211>    11
<212>    PRT
<213>    Artificial Sequence

<220>
<223>    AchR binding peptide

<400>    393
Pro Ala Leu Arg Met Arg Arg Trp Cys Gln Lys
1               5                   10

<210>    394
<211>    11
<212>    PRT
<213>    Artificial Sequence

<220>
<223>    AchR binding peptide

<400>    394
His Leu Glu Lys His Arg Arg Cys Glu Phe Lys
1               5                   10

<210>    395
<211>    11
<212>    PRT
<213>    Artificial Sequence

<220>
<223>    AchR binding peptide

<400>    395
Cys Gln Ala Lys Thr Arg Lys Ala Glu Asp Arg
1               5                   10

<210>    396

<211>    11
<212>    PRT
<213>    Artificial Sequence

<220>
<223>    AchR binding peptide


<400>    396
Gln Asn Met Arg Met Lys Arg Phe Ile Gln Arg
1               5                   10


<210>    397
<211>    11
<212>    PRT
<213>    Artificial Sequence

<220>
<223>    AchR binding peptide


<400>    397
Cys Pro Tyr Arg Ile Arg Arg Gly Pro Gly Lys
1               5                   10


<210>    398
<211>    11
<212>    PRT
<213>    Artificial Sequence

<220>
<223>    AchR binding peptide


<400>    398
Asp Ile Tyr Lys Gly Lys Arg Thr Leu Val Lys
1               5                   10


<210>    399
<211>    11
<212>    PRT
<213>    Artificial Sequence

<220>
<223>    AchR binding peptide


<400>    399
Glu Ile Cys Lys Asn Arg Lys Pro Ala Asn Arg
1               5                   10


<210>    400
<211>    11
<212>    PRT
<213>    Artificial Sequence

<220>
<223>    AchR binding peptide

<400>    400
Gln Gly Met Lys Leu Lys Arg Ile Trp Ser Lys
1               5                   10


<210>    401
<211>    14
<212>    PRT
<213>    Artificial Sequence

<220>
<223>    AchR binding peptide


<400>    401
Ala Leu Gly Arg Thr Lys Arg Leu His Met Arg Val His Val
1               5                   10


<210>    402
<211>    14
<212>    PRT
<213>    Artificial Sequence

<220>
<223>    AchR binding peptide


<400>    402
Ser Phe Tyr Lys Glu Arg Lys Cys Gln Phe Arg Ser Gly Leu
1               5                   10


<210>    403
<211>    14
<212>    PRT
<213>    Artificial Sequence

<220>
<223>    AchR binding peptide


<400>    403
Ser Gly Val Arg Tyr Arg Lys Trp Trp Ile Arg Ser Val Val
1               5                   10


<210>    404
<211>    14
<212>    PRT
<213>    Artificial Sequence

<220>
<223>    AchR binding peptide


<400>    404
Met Pro Gln Lys Leu Lys Lys Leu Asp Ile Arg Asn His Asn
1               5                   10


<210>    405

<211>    14
<212>    PRT
<213>    Artificial Sequence

<220>
<223>    AchR binding peptide


<400>    405
Asn Glu Asp Arg Gly Lys Arg Pro His Ile Arg Val Leu Gly
1               5                   10


<210>    406
<211>    14
<212>    PRT
<213>    Artificial Sequence

<220>
<223>    AchR binding peptide


<400>    406
Glu Phe Val Lys Leu Arg Lys Ala Arg Leu Arg Gly Pro Gln
1               5                   10


<210>    407
<211>    14
<212>    PRT
<213>    Artificial Sequence

<220>
<223>    AchR binding peptide


<400>    407
Glu Gly Asp Lys Phe Arg Arg His Asp Ile Lys Tyr Asn Phe
1               5                   10


<210>    408
<211>    14
<212>    PRT
<213>    Artificial Sequence

<220>
<223>    AchR binding peptide


<400>    408
Pro Ile Cys Arg Trp Lys Arg Ala Pro Phe Lys Trp Tyr Phe
1               5                   10


<210>    409
<211>    14
<212>    PRT
<213>    Artificial Sequence

<220>
<223>    AchR binding peptide

<400>    409
Asn Cys Asn Arg Phe Arg Arg Thr Ile Val Arg His Cys His
1               5                   10

<210>    410
<211>    14
<212>    PRT
<213>    Artificial Sequence

<220>
<223>    AchR binding peptide

<400>    410
Ala His Gly Lys Asp Arg Arg Tyr Val Glu Lys Leu Glu Val
1               5                   10

<210>    411
<211>    14
<212>    PRT
<213>    Artificial Sequence

<220>
<223>    AchR binding peptide

<400>    411
Phe Met Gln Lys Cys Lys Lys Trp Trp Asp Arg Ala Val Phe
1               5                   10

<210>    412
<211>    14
<212>    PRT
<213>    Artificial Sequence

<220>
<223>    AchR binding peptide

<400>    412
Asp Pro Pro Lys Lys Arg Lys Ser Leu Leu Arg Arg Val Ser
1               5                   10

<210>    413
<211>    14
<212>    PRT
<213>    Artificial Sequence

<220>
<223>    AchR binding peptide

<400>    413
Tyr Cys Thr Arg Ile Arg Arg Glu Gly Met Lys Gly Ser Glu
1               5                   10

<210>    414

<211>    14
<212>    PRT
<213>    Artificial Sequence

<220>
<223>    AchR binding peptide


<400>    414
Phe Pro Gly Lys Ser Lys Lys Gln Trp His Arg Leu Trp Pro
1               5                   10


<210>    415
<211>    14
<212>    PRT
<213>    Artificial Sequence

<220>
<223>    AchR binding peptide


<400>    415
Tyr Glu Pro Arg Phe Lys Arg Pro Tyr Gly Lys Trp Cys His
1               5                   10


<210>    416
<211>    14
<212>    PRT
<213>    Artificial Sequence

<220>
<223>    AchR binding peptide


<400>    416
Asp Tyr Leu Arg Ser Arg Lys Met Glu Glu Arg Phe Gln Glu
1               5                   10


<210>    417
<211>    14
<212>    PRT
<213>    Artificial Sequence

<220>
<223>    AchR binding peptide


<400>    417
Val Gly Pro Lys Phe Arg Lys Asn His Arg Arg Gln Asn Arg
1               5                   10


<210>    418
<211>    14
<212>    PRT
<213>    Artificial Sequence

<220>
<223>    AchR binding peptide

<400>    418
Glu Tyr Gln Lys Cys Lys Lys Pro Ser Phe Arg Leu Thr Met
1               5                   10

<210>    419
<211>    14
<212>    PRT
<213>    Artificial Sequence

<220>
<223>    AchR binding peptide

<400>    419
Tyr Met Lys Lys Lys Arg Lys Ser Leu Leu Arg Thr Ser Leu
1               5                   10

<210>    420
<211>    14
<212>    PRT
<213>    Artificial Sequence

<220>
<223>    AchR binding peptide

<400>    420
Cys Cys Phe Lys Leu Lys Lys Ala Tyr Asn Lys Gly Pro Phe
1               5                   10

<210>    421
<211>    14
<212>    PRT
<213>    Artificial Sequence

<220>
<223>    AchR binding peptide

<400>    421
Asn Pro Met Lys Leu Arg Lys Ala Glu Thr Lys His Asn Val
1               5                   10

<210>    422
<211>    14
<212>    PRT
<213>    Artificial Sequence

<220>
<223>    AchR binding peptide

<400>    422
His Ser Leu Lys Thr Arg Lys Ser Ala Phe Lys Ser Asn Thr
1               5                   10

<210>    423

<211>    14
<212>    PRT
<213>    Artificial Sequence

<220>
<223>    AchR binding peptide


<400>    423
Gln Tyr His Lys Val Arg Lys Leu Trp Phe Arg Val Glu Pro
1               5                   10


<210>    424
<211>    14
<212>    PRT
<213>    Artificial Sequence

<220>
<223>    AchR binding peptide


<400>    424
Phe Val Arg Lys Lys Arg Lys Ser Leu Leu Arg Asp Thr Arg
1               5                   10


<210>    425
<211>    14
<212>    PRT
<213>    Artificial Sequence

<220>
<223>    AchR binding peptide


<400>    425
Cys Phe Gln Arg Lys Arg Lys Ser Leu Leu Arg Val Leu Arg
1               5                   10


<210>    426
<211>    14
<212>    PRT
<213>    Artificial Sequence

<220>
<223>    AchR binding peptide


<400>    426
Val Pro Met Lys Tyr Arg Arg Cys Gly Asn Arg Gln Ser Asn
1               5                   10


<210>    427
<211>    14
<212>    PRT
<213>    Artificial Sequence

<220>
<223>    AchR binding peptide

<400>    427
Arg Ala Arg Lys Lys Arg Lys Ser Leu Leu Arg Arg Gln Val
1               5                   10

<210>    428
<211>    14
<212>    PRT
<213>    Artificial Sequence

<220>
<223>    AchR binding peptide

<400>    428
Pro Asn Gly Lys Val Arg Lys Arg Ile Arg Arg Arg Tyr Phe
1               5                   10

<210>    429
<211>    14
<212>    PRT
<213>    Artificial Sequence

<220>
<223>    AchR binding peptide

<400>    429
Cys Ala Ser Lys Pro Arg Arg Thr Tyr Leu Arg Ala Ala Asn
1               5                   10

<210>    430
<211>    14
<212>    PRT
<213>    Artificial Sequence

<220>
<223>    AchR binding peptide

<400>    430
Trp Ala His Lys Cys Lys Lys Pro Gly Gln Arg Ile Pro Pro
1               5                   10

<210>    431
<211>    14
<212>    PRT
<213>    Artificial Sequence

<220>
<223>    AchR binding peptide

<400>    431
Leu Ser Asp Lys Lys Arg Lys Ser Leu Leu Arg Tyr Asp Tyr
1               5                   10

<210>    432

<211>    14
<212>    PRT
<213>    Artificial Sequence

<220>
<223>    AchR binding peptide


<400>    432
Cys Ala Thr Arg Gly Lys Lys Val Phe Ser Lys Arg Thr Met
1               5                   10


<210>    433
<211>    14
<212>    PRT
<213>    Artificial Sequence

<220>
<223>    AchR binding peptide


<400>    433
Leu Arg Gln Arg Ser Lys Arg Val Leu Glu Lys Leu Arg Pro
1               5                   10


<210>    434
<211>    14
<212>    PRT
<213>    Artificial Sequence

<220>
<223>    AchR binding peptide


<400>    434
Leu Gly Trp Lys Lys Arg Lys Ser Leu Leu Arg Arg His Val
1               5                   10


<210>    435
<211>    14
<212>    PRT
<213>    Artificial Sequence

<220>
<223>    AchR binding peptide


<400>    435
Leu Gln Cys Lys Tyr Arg Arg Gly Ser Asp Lys Gln Pro Gln
1               5                   10


<210>    436
<211>    14
<212>    PRT
<213>    Artificial Sequence

<220>
<223>    AchR binding peptide

<400>    436
Ala Phe Ser Arg Ile Lys Arg Gly Val Leu Lys Leu Leu Ser
1               5                   10

<210>    437
<211>    14
<212>    PRT
<213>    Artificial Sequence

<220>
<223>    AchR binding peptide

<400>    437
Ser Asn Val Lys Arg Lys Arg Gly Arg Cys Lys Pro Tyr Arg
1               5                   10

<210>    438
<211>    14
<212>    PRT
<213>    Artificial Sequence

<220>
<223>    AchR binding peptide

<400>    438
Thr Val Val Lys Ser Arg Lys Cys Ser Val Arg Tyr Asn Trp
1               5                   10

<210>    439
<211>    14
<212>    PRT
<213>    Artificial Sequence

<220>
<223>    AchR binding peptide

<400>    439
Asp Asp Val Lys Gln Arg Lys Lys His Pro Arg Val Gln Thr
1               5                   10

<210>    440
<211>    14
<212>    PRT
<213>    Artificial Sequence

<220>
<223>    AchR binding peptide

<400>    440
Ala Ser Pro Lys Gly Arg Arg Pro Thr Phe Arg Pro Gln His
1               5                   10

<210>    441

<211>    14
<212>    PRT
<213>    Artificial Sequence

<220>
<223>    AchR binding peptide


<400>    441
Gly Trp Leu Lys Ala Lys Arg Phe Pro Ser Arg Pro Pro Thr
1               5                   10


<210>    442
<211>    14
<212>    PRT
<213>    Artificial Sequence

<220>
<223>    AchR binding peptide


<400>    442
Glu Thr Asn Arg Thr Arg Lys Gln Cys Tyr Lys Thr Thr Phe
1               5                   10


<210>    443
<211>    14
<212>    PRT
<213>    Artificial Sequence

<220>
<223>    AchR binding peptide


<400>    443
Phe Thr His Lys Asn Arg Arg Asp Ser Leu Arg Val Trp Met
1               5                   10


<210>    444
<211>    14
<212>    PRT
<213>    Artificial Sequence

<220>
<223>    AchR binding peptide


<400>    444
Pro Pro Phe Arg Leu Arg Lys Pro Leu Trp Arg Pro Gln Arg
1               5                   10


<210>    445
<211>    14
<212>    PRT
<213>    Artificial Sequence

<220>
<223>    AchR binding peptide

<400>    445
Pro Gly Asn Arg Met Lys Lys Tyr Gln Asn Arg Val His Gly
1               5                   10

<210>    446
<211>    14
<212>    PRT
<213>    Artificial Sequence

<220>
<223>    AchR binding peptide

<400>    446
Ser Ala His Lys Lys Arg Lys Gln Thr Leu Arg Cys Ser Glu
1               5                   10

<210>    447
<211>    14
<212>    PRT
<213>    Artificial Sequence

<220>
<223>    AchR binding peptide

<400>    447
Leu Tyr Glu Lys Tyr Lys Lys His Asn Asn Arg Glu Asp Asp
1               5                   10

<210>    448
<211>    14
<212>    PRT
<213>    Artificial Sequence

<220>
<223>    AchR binding peptide

<400>    448
Pro His Cys Arg Gln Lys Lys Phe Trp Ile Arg Cys Gly Thr
1               5                   10

<210>    449
<211>    14
<212>    PRT
<213>    Artificial Sequence

<220>
<223>    AchR binding peptide

<400>    449
Val Ala Phe Lys Thr Arg Arg Arg Val Gln Arg Gln Ser Gly
1               5                   10

<210>    450

<211>    14
<212>    PRT
<213>    Artificial Sequence

<220>
<223>    AchR binding peptide


<400>    450
Val Phe Asp Lys Phe Arg Lys Thr Glu Asn Arg Gly Val Ile
1               5                   10


<210>    451
<211>    14
<212>    PRT
<213>    Artificial Sequence

<220>
<223>    AchR binding peptide


<400>    451
Asn His His Lys Thr Lys Arg Cys Ser Val Arg Phe Asn Ile
1               5                   10


<210>    452
<211>    14
<212>    PRT
<213>    Artificial Sequence

<220>
<223>    AchR binding peptide


<400>    452
Gly Thr Phe Lys Trp Arg Lys Ser Gly Ala Arg Gln Tyr Leu
1               5                   10


<210>    453
<211>    14
<212>    PRT
<213>    Artificial Sequence

<220>
<223>    AchR binding peptide


<400>    453
His Cys Leu Arg Thr Lys Lys Leu Ile Asn Lys Ile Cys Ser
1               5                   10


<210>    454
<211>    14
<212>    PRT
<213>    Artificial Sequence

<220>
<223>    AchR binding peptide

<400>     454
Phe Phe Leu Arg Cys Arg Arg Leu Leu Gly Lys Val Gln Val
1               5                   10

<210>     455
<211>     14
<212>     PRT
<213>     Artificial Sequence

<220>
<223>     AchR binding peptide

<400>     455
His Phe Pro Arg Ala Arg Arg Phe Glu His Arg Cys Met Leu
1               5                   10

<210>     456
<211>     14
<212>     PRT
<213>     Artificial Sequence

<220>
<223>     AchR binding peptide

<400>     456
Gln Ile Ser Lys Leu Lys Arg Pro Ser Tyr Arg Gly Asp Asp
1               5                   10

<210>     457
<211>     14
<212>     PRT
<213>     Artificial Sequence

<220>
<223>     AchR binding peptide

<400>     457
Glu Glu Ile Lys Gln Lys Lys Leu His Leu Arg Val Trp Phe
1               5                   10

<210>     458
<211>     14
<212>     PRT
<213>     Artificial Sequence

<220>
<223>     AchR binding peptide

<400>     458
Ser Leu Pro Lys Trp Arg Lys Gly Gly Asp Arg Val Phe Thr
1               5                   10

<210>     459

<211>    14
<212>    PRT
<213>    Artificial Sequence

<220>
<223>    AchR binding peptide


<400>    459
His Val Tyr Lys Asn Arg Arg Val Trp Gly Lys Gly Trp Pro
1               5                   10


<210>    460
<211>    14
<212>    PRT
<213>    Artificial Sequence

<220>
<223>    AchR binding peptide


<400>    460
Glu Asp Leu Arg Cys Lys Lys Leu Glu Leu Arg Ser Val Ile
1               5                   10


<210>    461
<211>    14
<212>    PRT
<213>    Artificial Sequence

<220>
<223>    AchR binding peptide


<400>    461
Thr His Asp Lys Cys Lys Lys His Asn Asp Lys Gln Ala His
1               5                   10


<210>    462
<211>    14
<212>    PRT
<213>    Artificial Sequence

<220>
<223>    AchR binding peptide


<400>    462
Tyr Pro Glu Arg Pro Arg Lys Leu Gln Asp Lys Ser Tyr Ser
1               5                   10


<210>    463
<211>    14
<212>    PRT
<213>    Artificial Sequence

<220>
<223>    AchR binding peptide

<400>    463
Cys Pro Trp Arg Asn Arg Lys Ala Met Ile Lys Gly Ile Ile
1               5                   10

<210>    464
<211>    14
<212>    PRT
<213>    Artificial Sequence

<220>
<223>    AchR binding peptide

<400>    464
His Glu Ile Lys Gln Lys Lys Tyr Phe His Arg Gly His Asp
1               5                   10

<210>    465
<211>    14
<212>    PRT
<213>    Artificial Sequence

<220>
<223>    AchR binding peptide

<400>    465
Cys Leu Glu Lys Leu Arg Lys Ala Val His Arg Gln Arg Arg
1               5                   10

<210>    466
<211>    14
<212>    PRT
<213>    Artificial Sequence

<220>
<223>    AchR binding peptide

<400>    466
Tyr Ile Ser Lys Ser Lys Lys Thr Ala Gly Arg Trp Phe Trp
1               5                   10

<210>    467
<211>    14
<212>    PRT
<213>    Artificial Sequence

<220>
<223>    AchR binding peptide

<400>    467
Val Thr Trp Lys Phe Arg Lys Ala Glu Lys Arg Trp Gly Tyr
1               5                   10

<210>    468

<211>     14
<212>     PRT
<213>     Artificial Sequence

<220>
<223>     AchR binding peptide


<400>     468
Tyr Ser Ser Lys Ser Arg Lys Leu Ser Pro Arg Thr Pro Arg
1               5                   10


<210>     469
<211>     14
<212>     PRT
<213>     Artificial Sequence

<220>
<223>     AchR binding peptide


<400>     469
Cys Val Asn Arg Val Lys Lys Ser Asp Ser Lys Gly Thr Trp
1               5                   10


<210>     470
<211>     14
<212>     PRT
<213>     Artificial Sequence

<220>
<223>     AchR binding peptide


<400>     470
Asp His Glu Arg Glu Arg Arg Leu Trp Ser Arg Phe Pro Phe
1               5                   10


<210>     471
<211>     14
<212>     PRT
<213>     Artificial Sequence

<220>
<223>     AchR binding peptide


<400>     471
Pro Leu Ile Lys Val Lys Arg Gly Val Gly Lys Leu Trp Asn
1               5                   10


<210>     472
<211>     14
<212>     PRT
<213>     Artificial Sequence

<220>
<223>     AchR binding peptide

<400>    472
Thr Gly Cys Arg Cys Lys Arg Ser Met Tyr Lys Asn Leu His
1               5                   10

<210>    473
<211>    14
<212>    PRT
<213>    Artificial Sequence

<220>
<223>    AchR binding peptide

<400>    473
Trp Thr Cys Arg Met Arg Lys Tyr Gln Leu Arg Thr Ser Glu
1               5                   10

<210>    474
<211>    14
<212>    PRT
<213>    Artificial Sequence

<220>
<223>    AchR binding peptide

<400>    474
Ile Ala Ile Arg Pro Arg Lys Met Thr Leu Lys Ile His Pro
1               5                   10

<210>    475
<211>    14
<212>    PRT
<213>    Artificial Sequence

<220>
<223>    AchR binding peptide

<400>    475
Gln Ile Pro Lys Gln Lys Lys Gln Glu Gln Arg Ala Ile Ser
1               5                   10

<210>    476
<211>    14
<212>    PRT
<213>    Artificial Sequence

<220>
<223>    AchR binding peptide

<400>    476
Gln Met Gly Lys Phe Lys Lys Ile Ser Leu Lys Asn Thr Phe
1               5                   10

<210>    477

<211>    14
<212>    PRT
<213>    Artificial Sequence

<220>
<223>    AchR binding peptide


<400>    477
Val Leu Ile Lys Gln Arg Lys Trp Gln Asp Arg Ser Cys Ser
1               5                   10


<210>    478
<211>    14
<212>    PRT
<213>    Artificial Sequence

<220>
<223>    AchR binding peptide


<400>    478
Phe Ile Thr Lys Ser Arg Arg Gln Gln Phe Arg Asn Gln Gly
1               5                   10


<210>    479
<211>    14
<212>    PRT
<213>    Artificial Sequence

<220>
<223>    AchR binding peptide


<400>    479
Gln Gln Phe Lys Tyr Arg Arg Glu Cys Val Lys Tyr Gly Ser
1               5                   10


<210>    480
<211>    14
<212>    PRT
<213>    Artificial Sequence

<220>
<223>    AchR binding peptide


<400>    480
Met Glu Gly Arg Glu Lys Lys Ser Tyr Asn Lys Gly Glu Asn
1               5                   10


<210>    481
<211>    14
<212>    PRT
<213>    Artificial Sequence

<220>
<223>    AchR binding peptide

<400>    481
Pro Thr Trp Arg His Arg Arg Tyr Cys Ala Lys Asp Ile Gly
1               5                   10

<210>    482
<211>    14
<212>    PRT
<213>    Artificial Sequence

<220>
<223>    AchR binding peptide

<400>    482
His Met Cys Arg Ser Lys Arg Val Ala Trp Lys Asn Leu Ile
1               5                   10

<210>    483
<211>    14
<212>    PRT
<213>    Artificial Sequence

<220>
<223>    AchR binding peptide

<400>    483
Ser Tyr Leu Lys Cys Lys Arg Ser Asp Tyr Lys Glu Val Pro
1               5                   10

<210>    484
<211>    14
<212>    PRT
<213>    Artificial Sequence

<220>
<223>    AchR binding peptide

<400>    484
His Thr Phe Lys Leu Arg Lys Leu Cys Gln Lys Leu Phe Glu
1               5                   10

<210>    485
<211>    14
<212>    PRT
<213>    Artificial Sequence

<220>
<223>    AchR binding peptide

<400>    485
Gln Leu Val Lys Leu Arg Lys Leu Ala Arg Arg Val Ser Tyr
1               5                   10

<210>    486

<211>    14
<212>    PRT
<213>    Artificial Sequence

<220>
<223>    AchR binding peptide


<400>    486
Ser Glu Val Arg Pro Lys Lys Asp His Ser Arg Leu Phe Ile
1               5                   10


<210>    487
<211>    14
<212>    PRT
<213>    Artificial Sequence

<220>
<223>    AchR binding peptide


<400>    487
Phe Leu Glu Lys Cys Arg Lys Phe Ile Ile Arg Val Ser Thr
1               5                   10


<210>    488
<211>    14
<212>    PRT
<213>    Artificial Sequence

<220>
<223>    AchR binding peptide


<400>    488
Pro Glu Thr Arg His Arg Lys Met Phe Ile Arg Asp Phe Trp
1               5                   10


<210>    489
<211>    14
<212>    PRT
<213>    Artificial Sequence

<220>
<223>    AchR binding peptide


<400>    489
Glu Trp Cys Lys Asn Lys Arg Trp His Ser Arg Glu Tyr Pro
1               5                   10


<210>    490
<211>    14
<212>    PRT
<213>    Artificial Sequence

<220>
<223>    AchR binding peptide

<400>    490
Leu Trp Asn Arg Tyr Lys Lys Leu Leu Met Arg Ile Phe Trp
1               5                   10

<210>    491
<211>    14
<212>    PRT
<213>    Artificial Sequence

<220>
<223>    AchR binding peptide

<400>    491
Cys Trp Cys Arg Gln Arg Lys Cys Phe His Lys Pro Trp Ile
1               5                   10

<210>    492
<211>    14
<212>    PRT
<213>    Artificial Sequence

<220>
<223>    AchR binding peptide

<400>    492
Asn Leu Glu Arg Thr Lys Lys His Gly Leu Lys Gly Tyr Met
1               5                   10

<210>    493
<211>    14
<212>    PRT
<213>    Artificial Sequence

<220>
<223>    AchR binding peptide

<400>    493
Glu Gly Gly Arg Ile Lys Arg Pro Asn Tyr Arg Gly Asp Gly
1               5                   10

<210>    494
<211>    14
<212>    PRT
<213>    Artificial Sequence

<220>
<223>    AchR binding peptide

<400>    494
Pro Val Leu Lys Leu Arg Lys Gly Arg Val Arg Ala Gln Pro
1               5                   10

<210>    495

<211>    14
<212>    PRT
<213>    Artificial Sequence

<220>
<223>    AchR binding peptide


<400>    495
Ser Ile Ser Arg Leu Arg Lys Ala His Gln Lys Phe Ile Pro
1               5                   10


<210>    496
<211>    14
<212>    PRT
<213>    Artificial Sequence

<220>
<223>    AchR binding peptide


<400>    496
Glu Trp His Lys His Arg Arg Glu Met Val Arg Trp Gly Pro
1               5                   10


<210>    497
<211>    14
<212>    PRT
<213>    Artificial Sequence

<220>
<223>    AchR binding peptide


<400>    497
Trp Ser Ala Lys Gly Arg Arg Met Gly Cys Lys Ser Thr Met
1               5                   10


<210>    498
<211>    14
<212>    PRT
<213>    Artificial Sequence

<220>
<223>    AchR binding peptide


<400>    498
Pro Asn Gly Lys Val Arg Lys Arg Ile Arg Arg Arg Tyr Phe
1               5                   10


<210>    499
<211>    14
<212>    PRT
<213>    Artificial Sequence

<220>
<223>    AchR binding peptide

<400>    499
Pro Cys Trp Lys Phe Arg Arg Ala Gln Met Lys Trp Gly Ile
1               5                   10

<210>    500
<211>    14
<212>    PRT
<213>    Artificial Sequence

<220>
<223>    AchR binding peptide

<400>    500
Leu Glu Glu Arg Pro Lys Lys His Cys Ala Lys Asn His Cys
1               5                   10

<210>    501
<211>    14
<212>    PRT
<213>    Artificial Sequence

<220>
<223>    AchR binding peptide

<400>    501
Gln Pro Trp Arg Gln Lys Lys Phe Trp Cys Arg Cys Trp Gly
1               5                   10

<210>    502
<211>    14
<212>    PRT
<213>    Artificial Sequence

<220>
<223>    AchR binding peptide

<400>    502
Ile Met Val Lys Ile Arg Arg Cys Pro Ala Arg Pro Leu Cys
1               5                   10

<210>    503
<211>    14
<212>    PRT
<213>    Artificial Sequence

<220>
<223>    AchR binding peptide

<400>    503
Asn Asp Trp Lys Leu Arg Lys Asp Phe Trp Arg Asn His Phe
1               5                   10

<210>    504

<211>    14
<212>    PRT
<213>    Artificial Sequence

<220>
<223>    AchR binding peptide


<400>    504
Ala Ser Val Arg Asp Arg Lys Met Gly Tyr Lys Ser Asp Gly
1               5                   10


<210>    505
<211>    14
<212>    PRT
<213>    Artificial Sequence

<220>
<223>    AchR binding peptide


<400>    505
Tyr Tyr Glu Lys Ile Arg Arg Gly Glu Ile Lys Ile Ala Glu
1               5                   10


<210>    506
<211>    14
<212>    PRT
<213>    Artificial Sequence

<220>
<223>    AchR binding peptide


<400>    506
Asp Leu Val Arg Lys Arg Lys Thr Met Leu Arg Gln Leu Val
1               5                   10


<210>    507
<211>    14
<212>    PRT
<213>    Artificial Sequence

<220>
<223>    AchR binding peptide


<400>    507
Asp Leu Ser Lys Val Arg Arg Gly His Gly Lys Asn Asp Ile
1               5                   10


<210>    508
<211>    14
<212>    PRT
<213>    Artificial Sequence

<220>
<223>    AchR binding peptide

<400>     508
Glu Ser Tyr Arg Leu Arg Arg Gly Thr Asp Lys Glu Gln Trp
1               5                   10

<210>     509
<211>     14
<212>     PRT
<213>     Artificial Sequence

<220>
<223>     AchR binding peptide

<400>     509
Tyr Ile Thr Lys Phe Arg Lys Phe Leu Met Lys Asp Gln Phe
1               5                   10

<210>     510
<211>     14
<212>     PRT
<213>     Artificial Sequence

<220>
<223>     AchR binding peptide

<400>     510
Ala Trp Ala Lys Tyr Lys Lys Val Gln Pro Lys His His Thr
1               5                   10

<210>     511
<211>     14
<212>     PRT
<213>     Artificial Sequence

<220>
<223>     AchR binding peptide

<400>     511
Thr Trp Leu Arg Gly Arg Arg Trp Pro Asp Lys Gln Pro Gln
1               5                   10

<210>     512
<211>     14
<212>     PRT
<213>     Artificial Sequence

<220>
<223>     AchR binding peptide

<400>     512
Ala Leu Pro Arg Val Arg Lys Asp Ser His Arg Glu Glu Ile
1               5                   10

<210>     513

<211>    14
<212>    PRT
<213>    Artificial Sequence

<220>
<223>    AchR binding peptide


<400>    513
Cys Leu Phe Lys Tyr Arg Lys Ser Cys Val Arg Leu Cys Gly
1               5                   10


<210>    514
<211>    14
<212>    PRT
<213>    Artificial Sequence

<220>
<223>    AchR binding peptide


<400>    514
Leu Gln Asn Arg Met Arg Lys Ile Tyr Trp Lys Thr Phe Asp
1               5                   10


<210>    515
<211>    14
<212>    PRT
<213>    Artificial Sequence

<220>
<223>    AchR binding peptide


<400>    515
Asn Asp Pro Arg Leu Arg Lys His Asn His Arg Cys Cys Thr
1               5                   10


<210>    516
<211>    14
<212>    PRT
<213>    Artificial Sequence

<220>
<223>    AchR binding peptide


<400>    516
Tyr Gly Ser Lys Gln Arg Arg Phe Glu Glu Lys Ile Cys Gly
1               5                   10


<210>    517
<211>    14
<212>    PRT
<213>    Artificial Sequence

<220>
<223>    AchR binding peptide

<400>    517
Cys Gly His Lys Asn Lys Lys Trp His Asn Arg Met Asp Pro
1               5                   10

<210>    518
<211>    14
<212>    PRT
<213>    Artificial Sequence

<220>
<223>    AchR binding peptide

<400>    518
Tyr Gly Ile Arg Asp Arg Arg Pro Met Thr Lys His Thr Gln
1               5                   10

<210>    519
<211>    14
<212>    PRT
<213>    Artificial Sequence

<220>
<223>    AchR binding peptide

<400>    519
Thr Pro Leu Lys Ser Arg Lys Tyr Trp Asn Lys His Ala Tyr
1               5                   10

<210>    520
<211>    14
<212>    PRT
<213>    Artificial Sequence

<220>
<223>    AchR binding peptide

<400>    520
Phe Leu Tyr Lys Ala Lys Lys Ala Leu Met Arg Asp Ser Leu
1               5                   10

<210>    521
<211>    14
<212>    PRT
<213>    Artificial Sequence

<220>
<223>    AchR binding peptide

<400>    521
Cys Asp Asp Lys Pro Lys Lys Thr Val Val Lys Leu Thr Trp
1               5                   10

<210>    522

<211>    14
<212>    PRT
<213>    Artificial Sequence

<220>
<223>    AchR binding peptide


<400>    522
Met His Pro Arg Pro Arg Lys Met Ser His Lys Met Ser Cys
1               5                   10


<210>    523
<211>    14
<212>    PRT
<213>    Artificial Sequence

<220>
<223>    AchR binding peptide


<400>    523
Pro Gly Glu Arg Leu Lys Lys Glu Asp His Arg Ala Ser Gly
1               5                   10


<210>    524
<211>    14
<212>    PRT
<213>    Artificial Sequence

<220>
<223>    AchR binding peptide


<400>    524
Gly Ala Gln Arg Ala Arg Lys Pro Val Leu Arg Ala Val Gly
1               5                   10


<210>    525
<211>    14
<212>    PRT
<213>    Artificial Sequence

<220>
<223>    AchR binding peptide


<400>    525
Met Gln Glu Lys Cys Arg Arg Cys Val Phe Lys Gly Asn Ile
1               5                   10


<210>    526
<211>    14
<212>    PRT
<213>    Artificial Sequence

<220>
<223>    AchR binding peptide

<400>    526
Tyr Val Asp Arg Trp Arg Arg Met Gln Tyr Lys Leu Ser Ile
1               5                   10

<210>    527
<211>    14
<212>    PRT
<213>    Artificial Sequence

<220>
<223>    AchR binding peptide

<400>    527
Trp Val Asp Arg Ser Arg Lys Phe Glu Asp Arg Asn Cys Leu
1               5                   10

<210>    528
<211>    14
<212>    PRT
<213>    Artificial Sequence

<220>
<223>    AchR binding peptide

<400>    528
Ile Val Asn Arg Ala Lys Arg Ser Gln Ile Arg Phe Asp Val
1               5                   10

<210>    529
<211>    14
<212>    PRT
<213>    Artificial Sequence

<220>
<223>    AchR binding peptide

<400>    529
Ile His Pro Arg Tyr Lys Arg His Gln Ala Arg Ser Cys Cys
1               5                   10

<210>    530
<211>    14
<212>    PRT
<213>    Artificial Sequence

<220>
<223>    AchR binding peptide

<400>    530
Glu His Ser Lys His Arg Lys Asp Leu Phe Arg Met Val Gly
1               5                   10

<210>    531

<211>    14
<212>    PRT
<213>    Artificial Sequence

<220>
<223>    AchR binding peptide


<400>    531
Tyr Thr Pro Lys Phe Arg Arg Ile Phe Trp Arg Ile Ile Asp
1               5                   10


<210>    532
<211>    14
<212>    PRT
<213>    Artificial Sequence

<220>
<223>    AchR binding peptide


<400>    532
Thr Ile Thr Arg Val Lys Arg Ala Ser His Lys Thr Pro Ser
1               5                   10


<210>    533
<211>    14
<212>    PRT
<213>    Artificial Sequence

<220>
<223>    AchR binding peptide


<400>    533
Val Asp Ser Arg Glu Lys Lys Trp Arg Gln Lys Cys Gln Cys
1               5                   10


<210>    534
<211>    14
<212>    PRT
<213>    Artificial Sequence

<220>
<223>    AchR binding peptide


<400>    534
Tyr Trp Phe Arg Ile Lys Arg Ala His Ala Lys Gly Cys Pro
1               5                   10


<210>    535
<211>    14
<212>    PRT
<213>    Artificial Sequence

<220>
<223>    AchR binding peptide

<400>    535
Asn Asp His Arg Met Arg Arg Ser Val Asp Arg Gly Glu Thr
1               5                   10

<210>    536
<211>    14
<212>    PRT
<213>    Artificial Sequence

<220>
<223>    AchR binding peptide

<400>    536
Pro Phe Asp Arg Ala Arg Arg Asp Leu His Arg Ile Met Met
1               5                   10

<210>    537
<211>    14
<212>    PRT
<213>    Artificial Sequence

<220>
<223>    AchR binding peptide

<400>    537
Gln Val Val Arg Leu Arg Arg Gly Lys Asn Arg Gly Thr Val
1               5                   10

<210>    538
<211>    14
<212>    PRT
<213>    Artificial Sequence

<220>
<223>    AchR binding peptide

<400>    538
Thr Asn Gln Arg Pro Arg Arg Glu Thr Ala Lys Thr Ile Glu
1               5                   10

<210>    539
<211>    14
<212>    PRT
<213>    Artificial Sequence

<220>
<223>    AchR binding peptide

<400>    539
Phe His His Arg His Lys Arg Ile Ala Thr Lys His Pro Ala
1               5                   10

<210>    540

<211>    14
<212>    PRT
<213>    Artificial Sequence

<220>
<223>    AchR binding peptide


<400>    540
Glu His Cys Lys Trp Lys Lys Met Phe Asp Lys Glu Gly Glu
1               5                   10


<210>    541
<211>    14
<212>    PRT
<213>    Artificial Sequence

<220>
<223>    AchR binding peptide


<400>    541
Phe Phe Tyr Arg Leu Arg Arg Cys Leu Thr Arg Trp Trp Val
1               5                   10


<210>    542
<211>    14
<212>    PRT
<213>    Artificial Sequence

<220>
<223>    AchR binding peptide


<400>    542
Leu Gln Cys Lys Tyr Arg Arg Gly Ser Asp Lys Thr Val Val
1               5                   10


<210>    543
<211>    14
<212>    PRT
<213>    Artificial Sequence

<220>
<223>    AchR binding peptide


<400>    543
Gln Thr Met Lys Phe Lys Arg Cys Cys Asn Lys Glu Met Val
1               5                   10


<210>    544
<211>    14
<212>    PRT
<213>    Artificial Sequence

<220>
<223>    AchR binding peptide

<400>    544
Cys Ser Tyr Lys Leu Arg Arg Cys Asp Met Arg Leu Trp Gly
1               5                   10

<210>    545
<211>    14
<212>    PRT
<213>    Artificial Sequence

<220>
<223>    AchR binding peptide

<400>    545
Met His Ile Lys Met Lys Arg Glu Gln Val Lys Asp Glu Glu
1               5                   10

<210>    546
<211>    14
<212>    PRT
<213>    Artificial Sequence

<220>
<223>    AchR binding peptide

<400>    546
Ala Val Trp Arg Asn Arg Lys Asp Asn Val Lys Ala Ser Glu
1               5                   10

<210>    547
<211>    14
<212>    PRT
<213>    Artificial Sequence

<220>
<223>    AchR binding peptide

<400>    547
Ala Asp His Arg Ala Lys Lys Thr Leu Ser Lys Phe Trp Thr
1               5                   10

<210>    548
<211>    14
<212>    PRT
<213>    Artificial Sequence

<220>
<223>    AchR binding peptide

<400>    548
Met Met Ala Arg Cys Arg Arg Ile Met Cys Arg Ser Phe Asn
1               5                   10

<210>    549

<211>    14
<212>    PRT
<213>    Artificial Sequence

<220>
<223>    AchR binding peptide


<400>    549
Pro Phe Gly Lys Phe Lys Arg Leu Glu Asn Lys Asp Glu Cys
1               5                   10


<210>    550
<211>    14
<212>    PRT
<213>    Artificial Sequence

<220>
<223>    AchR binding peptide


<400>    550
Tyr Leu Ser Arg Thr Lys Arg Trp Leu Ala Arg Tyr Val Glu
1               5                   10


<210>    551
<211>    14
<212>    PRT
<213>    Artificial Sequence

<220>
<223>    AchR binding peptide


<400>    551
His Thr Leu Lys Pro Lys Arg Leu Tyr Pro Arg Pro Ser Glu
1               5                   10


<210>    552
<211>    14
<212>    PRT
<213>    Artificial Sequence

<220>
<223>    AchR binding peptide


<400>    552
Val Val Ile Lys Cys Arg Lys Trp Thr His Lys Met Asp His
1               5                   10


<210>    553
<211>    14
<212>    PRT
<213>    Artificial Sequence

<220>
<223>    AchR binding peptide

<400>    553
Phe His Thr Lys Leu Arg Lys Pro Ile Pro Lys Ile Asp Met
1               5                   10

<210>    554
<211>    14
<212>    PRT
<213>    Artificial Sequence

<220>
<223>    AchR binding peptide

<400>    554
Glu Asn Ile Lys Ser Arg Lys Tyr Phe Val Lys Leu Thr Trp
1               5                   10

<210>    555
<211>    14
<212>    PRT
<213>    Artificial Sequence

<220>
<223>    AchR binding peptide

<400>    555
Ala Trp Thr Arg Phe Arg Arg Phe Gln Cys Lys Gly Met Ser
1               5                   10

<210>    556
<211>    14
<212>    PRT
<213>    Artificial Sequence

<220>
<223>    AchR binding peptide

<400>    556
Ile Val Pro Arg Asp Arg Lys Gln Ala Leu Arg Trp Thr Asn
1               5                   10

<210>    557
<211>    14
<212>    PRT
<213>    Artificial Sequence

<220>
<223>    AchR binding peptide

<400>    557
Cys Met Leu Arg Trp Lys Arg Trp His Met Arg Phe Asn Pro
1               5                   10

<210>    558

<211>     14
<212>     PRT
<213>     Artificial Sequence

<220>
<223>     AchR binding peptide


<400>     558
Phe Asp Met Arg Asp Arg Arg Pro Asn Ala Arg Val Met Pro
1               5                   10


<210>     559
<211>     14
<212>     PRT
<213>     Artificial Sequence

<220>
<223>     AchR binding peptide


<400>     559
Gln Ile Ala Lys Leu Lys Arg Asp Gln Met Lys Glu Ala Trp
1               5                   10


<210>     560
<211>     14
<212>     PRT
<213>     Artificial Sequence

<220>
<223>     AchR binding peptide


<400>     560
Leu Thr Ser Arg Cys Lys Lys Thr Phe Gln Lys Asn Ser Glu
1               5                   10


<210>     561
<211>     14
<212>     PRT
<213>     Artificial Sequence

<220>
<223>     AchR binding peptide


<400>     561
Met Ala Ala Lys Trp Lys Lys Asp Gly Met Lys Ser His Tyr
1               5                   10


<210>     562
<211>     14
<212>     PRT
<213>     Artificial Sequence

<220>
<223>     AchR binding peptide

<400>    562
Ala Asn Trp Arg Thr Arg Arg Asp Leu Ala Lys Glu His Val
1               5                   10

<210>    563
<211>    14
<212>    PRT
<213>    Artificial Sequence

<220>
<223>    AchR binding peptide

<400>    563
Pro Phe Thr Lys Met Arg Lys Gln Phe Gln Arg Met Ser Ala
1               5                   10

<210>    564
<211>    14
<212>    PRT
<213>    Artificial Sequence

<220>
<223>    AchR binding peptide

<400>    564
Gln Ile Asn Arg Val Lys Lys Ser Asp Ala Lys Phe Cys Thr
1               5                   10

<210>    565
<211>    14
<212>    PRT
<213>    Artificial Sequence

<220>
<223>    AchR binding peptide

<400>    565
Tyr Leu Val Lys Leu Arg Arg Phe Trp Ala Lys Ile Asp Met
1               5                   10

<210>    566
<211>    14
<212>    PRT
<213>    Artificial Sequence

<220>
<223>    AchR binding peptide

<400>    566
Glu Tyr Val Lys Ala Lys Arg Thr Pro Trp Lys Tyr Val Val
1               5                   10

<210>    567

<211>    14
<212>    PRT
<213>    Artificial Sequence

<220>
<223>    AchR binding peptide


<400>    567
Ala Met Cys Arg Glu Lys Arg Phe Pro Tyr Arg Ala Ile Tyr
1               5                   10


<210>    568
<211>    14
<212>    PRT
<213>    Artificial Sequence

<220>
<223>    AchR binding peptide


<400>    568
Met Gly Leu Arg Ala Arg Lys Tyr Glu Asp Lys Thr Leu His
1               5                   10


<210>    569
<211>    14
<212>    PRT
<213>    Artificial Sequence

<220>
<223>    AchR binding peptide


<400>    569
Phe His Met Lys Ser Lys Lys Asp Gly Asp Lys Phe Val Met
1               5                   10


<210>    570
<211>    14
<212>    PRT
<213>    Artificial Sequence

<220>
<223>    AchR binding peptide


<400>    570
Cys Ile Phe Lys Phe Lys Lys Asn Met Phe Lys Gly Tyr Ser
1               5                   10


<210>    571
<211>    14
<212>    PRT
<213>    Artificial Sequence

<220>
<223>    AchR binding peptide

<400>    571
Leu Asp Thr Lys Asn Arg Arg Gly Ala Ser Lys Trp Asp Tyr
1               5                   10

<210>    572
<211>    14
<212>    PRT
<213>    Artificial Sequence

<220>
<223>    AchR binding peptide

<400>    572
Val Thr Gly Lys Gly Arg Arg Val Thr Phe Arg Cys Met Asn
1               5                   10

<210>    573
<211>    14
<212>    PRT
<213>    Artificial Sequence

<220>
<223>    AchR binding peptide

<400>    573
Gln Leu Gly Lys Gln Lys Arg Glu Ala Thr Arg Glu Tyr Val
1               5                   10

<210>    574
<211>    14
<212>    PRT
<213>    Artificial Sequence

<220>
<223>    AchR binding peptide

<400>    574
Ser Ser Gly Lys Trp Lys Arg Pro His Ala Lys Tyr Ile Val
1               5                   10

<210>    575
<211>    14
<212>    PRT
<213>    Artificial Sequence

<220>
<223>    AchR binding peptide

<400>    575
Thr Cys His Lys Thr Lys Arg Ser Ile Met Arg Ala Thr Ser
1               5                   10

<210>    576

<211>     14
<212>     PRT
<213>     Artificial Sequence

<220>
<223>     AchR binding peptide


<400>     576
Met Ile Gly Lys Cys Arg Lys Cys Gly Thr Lys Cys Tyr Ala
1               5                   10


<210>     577
<211>     14
<212>     PRT
<213>     Artificial Sequence

<220>
<223>     AchR binding peptide


<400>     577
Phe Gln Pro Lys Ser Arg Arg Ala Glu Thr Lys His Ser Tyr
1               5                   10


<210>     578
<211>     14
<212>     PRT
<213>     Artificial Sequence

<220>
<223>     AchR binding peptide


<400>     578
Pro Asp Leu Lys Phe Arg Lys His Gln Asp Arg Thr Ala Ile
1               5                   10


<210>     579
<211>     14
<212>     PRT
<213>     Artificial Sequence

<220>
<223>     AchR binding peptide


<400>     579
Tyr Asn Ala Arg Val Lys Arg Glu Gly Ile Lys Asn Ile Thr
1               5                   10


<210>     580
<211>     14
<212>     PRT
<213>     Artificial Sequence

<220>
<223>     AchR binding peptide

<400>    580
Gln Gly Ile Arg Pro Arg Lys His Leu Gln Arg Met Pro Asn
1               5                   10

<210>    581
<211>    14
<212>    PRT
<213>    Artificial Sequence

<220>
<223>    AchR binding peptide

<400>    581
Gly Pro Cys Arg Pro Arg Arg His Ser Ile Arg Leu Leu Trp
1               5                   10

<210>    582
<211>    14
<212>    PRT
<213>    Artificial Sequence

<220>
<223>    AchR binding peptide

<400>    582
Phe Tyr Leu Arg Glu Arg Arg His Gln Leu Lys Thr Glu Phe
1               5                   10

<210>    583
<211>    14
<212>    PRT
<213>    Artificial Sequence

<220>
<223>    AchR binding peptide

<400>    583
Asn Met Ala Arg Ser Arg Lys Asn Glu Cys Lys Tyr Ile Ala
1               5                   10

<210>    584
<211>    14
<212>    PRT
<213>    Artificial Sequence

<220>
<223>    AchR binding peptide

<400>    584
Asp Met Asp Lys Val Lys Lys Trp Val Trp Arg Ser Phe Pro
1               5                   10

<210>    585

<211>     14
<212>     PRT
<213>     Artificial Sequence

<220>
<223>     AchR binding peptide


<400>     585
Val Leu Pro Lys Glu Arg Arg Trp Leu Thr Arg Phe Leu Ile
1               5                   10


<210>     586
<211>     14
<212>     PRT
<213>     Artificial Sequence

<220>
<223>     AchR binding peptide


<400>     586
Tyr Glu His Arg His Lys Arg Phe Phe His Lys Asp Met Pro
1               5                   10


<210>     587
<211>     14
<212>     PRT
<213>     Artificial Sequence

<220>
<223>     AchR binding peptide


<400>     587
His Gly His Lys Pro Arg Arg Trp Ile Tyr Arg Met Pro Met
1               5                   10


<210>     588
<211>     14
<212>     PRT
<213>     Artificial Sequence

<220>
<223>     AchR binding peptide


<400>     588
Asp Gly Ile Arg Asp Lys Lys Cys Cys Trp Arg Asp Leu Ile
1               5                   10


<210>     589
<211>     14
<212>     PRT
<213>     Artificial Sequence

<220>
<223>     AchR binding peptide

<400>    589
Glu Leu Val Lys His Arg Arg Leu Asp Phe Arg Asp Pro Met
1               5                   10

<210>    590
<211>    14
<212>    PRT
<213>    Artificial Sequence

<220>
<223>    AchR binding peptide

<400>    590
Val His His Lys Leu Arg Arg Val His Leu Arg Leu Asp Val
1               5                   10

<210>    591
<211>    14
<212>    PRT
<213>    Artificial Sequence

<220>
<223>    AchR binding peptide

<400>    591
Gly Ile Asn Arg Gly Arg Lys Val Ser Pro Lys Asp Glu Gln
1               5                   10

<210>    592
<211>    14
<212>    PRT
<213>    Artificial Sequence

<220>
<223>    AchR binding peptide

<400>    592
Asn Gly Tyr Arg Ala Arg Lys Glu Asn Leu Lys Phe Pro Glu
1               5                   10

<210>    593
<211>    14
<212>    PRT
<213>    Artificial Sequence

<220>
<223>    AchR binding peptide

<400>    593
Ala Ala Tyr Arg Ala Arg Lys Ile Val Glu Lys Ser Gly Trp
1               5                   10

<210>    594

<211>    14
<212>    PRT
<213>    Artificial Sequence

<220>
<223>    AchR binding peptide


<400>    594
Trp Ala Pro Arg Asp Arg Arg Asn Met Gly Arg Asp Pro Ala
1               5                   10


<210>    595
<211>    14
<212>    PRT
<213>    Artificial Sequence

<220>
<223>    AchR binding peptide


<400>    595
Ile Glu Asp Lys Ser Lys Arg Phe Asn Cys Lys Glu Cys Gly
1               5                   10


<210>    596
<211>    14
<212>    PRT
<213>    Artificial Sequence

<220>
<223>    AchR binding peptide


<400>    596
Ile Glu Met Arg Tyr Arg Arg Asp Cys Ser Arg Phe Val Asn
1               5                   10


<210>    597
<211>    14
<212>    PRT
<213>    Artificial Sequence

<220>
<223>    AchR binding peptide


<400>    597
Gln Gln Ile Lys Tyr Lys Arg Pro Thr Tyr Arg Met Val Cys
1               5                   10


<210>    598
<211>    14
<212>    PRT
<213>    Artificial Sequence

<220>
<223>    AchR binding peptide

<400>    598
Leu Pro Trp Arg Glu Arg Arg Asn Gly Gly Arg Ser Ser Glu
1               5                   10

<210>    599
<211>    14
<212>    PRT
<213>    Artificial Sequence

<220>
<223>    AchR binding peptide

<400>    599
Pro Gln Thr Arg Tyr Arg Lys Tyr Phe Tyr Arg Pro Gln Met
1               5                   10

<210>    600
<211>    14
<212>    PRT
<213>    Artificial Sequence

<220>
<223>    AchR binding peptide

<400>    600
His Met Cys Arg Trp Arg Arg Gln Ile Asn Lys Gly Val Ser
1               5                   10

<210>    601
<211>    11
<212>    PRT
<213>    Artificial Sequence

<220>
<223>    AchR binding peptide

<400>    601
Arg Arg Arg Arg Arg Arg Arg Arg Arg Arg Arg
1               5                   10

<210>    602
<211>    11
<212>    PRT
<213>    Artificial Sequence

<220>
<223>    AchR binding peptide

<400>    602
Gly Gly Gly Arg Lys Lys Arg Arg Gln Arg Arg
1               5                   10

<210>    603

<211>    11
<212>    PRT
<213>    Artificial Sequence

<220>
<223>    AchR binding peptide


<400>    603
Gly Gly Arg Lys Lys Arg Arg Gln Arg Arg Arg
1               5                   10


<210>    604
<211>    14
<212>    PRT
<213>    Artificial Sequence

<220>
<223>    AchR binding peptide


<400>    604
Gly Gly Gly Arg Lys Lys Arg Arg Gln Arg Arg Arg Gly Gly
1               5                   10


<210>    605
<211>    14
<212>    PRT
<213>    Artificial Sequence

<220>
<223>    AchR binding peptide


<400>    605
Gly Gly Arg Lys Lys Arg Arg Gln Arg Arg Arg Gly Gly Gly
1               5                   10


<210>    606
<211>    11
<212>    PRT
<213>    Artificial Sequence

<220>
<223>    AchR binding peptide


<400>    606
Gly Gly Gly Arg Arg Arg Arg Arg Arg Arg Arg
1               5                   10